# EUROPEAN PATENT APPLICATION

(11) **EP 4 840 491 A1**
(43) Date of publication of application: **18.11.2026**
(21) Application number: 24916745.3
(22) Date of filing: 26.12.2024
(51) Int. Cl.: A61M 15/00

(54) **POWDER INHALER, AIR INFLOW BAFFLE, DOSE PROTECTION PLATE, INHALATION TRIGGER MECHANISM, AIRBAG PRESSING MEMBER, DRIVING CAM, AND GAS COMPRESSING MECHANISM**

(30) Priority: 12.01.2024 CN 202410051869; 12.01.2024 CN 202410055013; 12.01.2024 CN 202410051884; 12.01.2024 CN 202410051892
(71) Applicant: Transpire Bio Inc, Sunrise, FL 33323 (US)
(72) Inventor: LIU, Yongwei, Shenzhen, Guangdong 518102 (CN); ZHANG, Xieen, Shenzhen, Guangdong 518102 (CN); CHENG, Shiyi, Shenzhen, Guangdong 518102 (CN)
(74) Representative: Latscha Schöllhorn Partner AG
(86) International application number: PCT/CN2024/142672
(87) International publication number: WO 2025/148698

(57) **Abstract**

A powder inhaler, an air inlet baffle, a dose protection plate, an inhalation trigger mechanism, an airbag pressing member, a drive cam, and an air compressing mechanism are provided. The powder inhaler includes a functional mechanism, a suction nozzle (101), and an outer cap (4). The functional mechanism includes an inhalation channel (706) in communication with the suction nozzle (101). The outer cap (4) is linked to and cooperates with the functional mechanism, and is limited to rotate back and forth between a first position and a second position, where the outer cap blocks the suction nozzle (101) when the outer cap is configured to be at the first position, and the outer cap unblocks the suction nozzle (101) when the outer cap is configured to be at the second position. A stroke of the outer cap (4) rotating from the first position to the second position includes a cap-opening idle stroke and a cap-opening load stroke. The outer cap (4) does not trigger an action of the functional mechanism in the cap-opening idle stroke; and the outer cap (4) triggers, in the cap-opening load stroke, the functional mechanism to deliver powder to the inhalation channel (706). A stroke of resetting from the second position to the first position includes a cap-closing idle stroke and a cap-closing load stroke. The outer cap (4) does not trigger an action of the functional mechanism in the cap-closing idle stroke; and the outer cap (4) triggers, in the cap-closing load stroke, the functional mechanism to reset. Through the foregoing setting, linkage between mechanisms is implemented.

## Description

### CROSS-REFERENCE TO RELATED APPLICATIONS

This application claims priorities to Chinese Patent Application No. 202410051869.2, No. 202410055013.2, No. 202410051884.7, and No. 202410051892.1 filed on January 12, 2024, which are incorporated herein by reference in their entireties.

### TECHNICAL FIELD

This application relates to the field of inhalation device technologies, and in particular, to a powder inhaler, an air inlet baffle, a dose protection plate, an inhalation trigger mechanism, an airbag pressing member, a drive cam, and an air compressing mechanism.

### BACKGROUND

A powder inhaler generally includes a housing assembly and various functional mechanisms, and dispenses a powdered pharmaceutical formulation via airflow inhalation, to draw the powder from a powder metering component into a suction nozzle for inhalation by a user.

However, due to structural limitations, existing powder inhalers fail to implement effective linkage among various systems of a device during cap opening and closing, and suffer from problems such as complex powder delivery manners, uncontrollable delivery doses, and susceptibility to powder waste. In addition, the existing powder inhalers cannot ensure protection of powder such as drug powder during low-speed airflow inhalation. During user inhalation, an air flow exhibits a poor deagglomeration effect on the drug powder, resulting in low utilization of the drug powder and a high risk of drug powder waste.

### SUMMARY

This application mainly provides a powder inhaler, an air inlet baffle, a dose protection plate, an inhalation trigger mechanism, an airbag pressing member, a drive cam, and an air compressing mechanism, to resolve a problem in the related art that various mechanisms of a device fail to implement effective linkage during cap opening and closing of the powder inhaler.

To resolve the foregoing technical problem, a technical solution adopted by this application is to provide a powder inhaler, including:
a functional mechanism, including an inhalation channel;
a suction nozzle, in communication with the inhalation channel; and
an outer cap, being linked to and cooperating with the functional mechanism, and being limited to rotate back and forth between a first position and a second position, where the outer cap blocks the suction nozzle when the outer cap is configured to be at the first position; and the outer cap unblocks the suction nozzle when the outer cap is configured to be at the second position, where
a stroke of the outer cap rotating from the first position to the second position includes a cap-opening idle stroke and a cap-opening load stroke following the cap-opening idle stroke; the outer cap does not trigger an action of the functional mechanism in the cap-opening idle stroke; and the outer cap triggers, in the cap-opening load stroke, the functional mechanism to deliver powder to the inhalation channel; and/or
a stroke of the outer cap rotating and resetting from the second position to the first position includes a cap-closing idle stroke and a cap-closing load stroke following the cap-closing idle stroke; the outer cap does not trigger an action of the functional mechanism in the cap-closing idle stroke; and the outer cap triggers, in the cap-closing load stroke, the functional mechanism to reset.

The cap-opening idle stroke includes the first cap-opening sub-idle stroke and the second cap-opening sub-idle stroke following the first cap-opening sub-idle stroke, where a torque in the second cap-opening sub-idle stroke is greater than a torque in the first cap-opening sub-idle stroke; and
preferably, a torque gradually increases or abruptly increases in a process of switching from the first cap-opening sub-idle stroke to the second cap-opening sub-idle stroke.

The cap-opening load stroke includes the first cap-opening sub-load stroke and the second cap-opening sub-load stroke following the first cap-opening sub-load stroke, where a torque in the first cap-opening sub-load stroke is less than a torque in the second cap-opening sub-load stroke and/or the cap-opening idle stroke.

The cap-closing idle stroke includes the first cap-closing sub-idle stroke and the second cap-closing sub-idle stroke following the first cap-closing sub-idle stroke, where
a torque in the first cap-closing sub-idle stroke is greater than a torque in the second cap-closing sub-idle stroke; and
preferably, the torque in the first cap-closing sub-idle stroke is constant or gradually increases.

The cap-closing load stroke includes a first cap-closing sub-load stroke, a second cap-closing sub-load stroke, and a third cap-closing sub-load stroke that are set in chronological order;
a torque in the second cap-closing sub-load stroke is greater than a torque in the first cap-closing sub-load stroke and/or the third cap-closing sub-load stroke;
preferably, the torque in the third cap-closing sub-load stroke is greater than or equal to the torque in the first cap-closing sub-load stroke; and
preferably, the torque in the second cap-closing sub-load stroke and/or the first cap-closing sub-load stroke gradually increases.

The functional mechanism includes: an air compressing mechanism; and
a powder delivery mechanism, including a storage cavity and a dose cup, where the storage cavity is configured to store the powder, and the storage cavity has a powder outlet; the cap-opening load stroke includes a first cap-opening sub-load stroke and a second cap-opening sub-load stroke following the first cap-opening sub-load stroke; the outer cap triggers, in the first cap-opening sub-load stroke, the air compressing mechanism to press the powder from the storage cavity into the dose cup; and the outer cap drives, in the second cap-opening sub-load stroke, the dose cup to deliver the powder to the inhalation channel.

The delivery mechanism includes a powder container and a powder metering wheel; the powder container has the inhalation channel and the storage cavity; the powder metering wheel is rotatably connected to the powder container; the powder metering wheel includes the dose cup; and
the outer cap triggers, in the first cap-opening sub-load stroke, the air compressing mechanism to first press air into the powder container, and then relieve pressure in the powder container, where the powder metering wheel is capable of rotating back and forth between a third position and a fourth position; when the powder metering wheel is configured to be at the third position, the dose cup is disposed corresponding to the powder outlet of the storage cavity and is configured to receive the powder in the powder container; when the powder metering wheel is configured to be at the fourth position, the dose cup is disposed corresponding to an inlet of the inhalation channel; and the outer cap drives, in the second cap-opening sub-load stroke, the dose cup to rotate from the third position to the fourth position.

The powder inhaler further includes: an inhalation trigger mechanism, including a dose protection plate and an air inlet baffle that are linked to and cooperate with each other, a resetting torsion spring, and a drive torsion spring, where the resetting torsion spring limits the air inlet baffle to an airflow channel; and the dose protection plate is limited by the air inlet baffle to the inlet of the inhalation channel and blocks the dose cup, where
the outer cap presses the resetting torsion spring in the second cap-opening sub-load stroke, and releases limitation of the resetting torsion spring on the air inlet baffle; and
when negative pressure of the airflow channel exceeds a threshold, the air inlet baffle rotates under an action of an air flow to release limitation on the dose protection plate, and the dose protection plate rotates to deviate under an action of the drive torsion spring and unblocks the dose cup of the powder metering wheel.

The functional mechanism includes: an air compressing mechanism; and
a powder delivery mechanism, including a powder container and a powder metering wheel, where the powder container has a storage cavity, and is configured to store the powder; and the powder metering wheel is rotatably connected to the powder container, where
the cap-closing load stroke includes a first cap-closing sub-load stroke, a second cap-closing sub-load stroke, and a third cap-closing sub-load stroke that are set in chronological order; the outer cap triggers, in the first cap-closing sub-load stroke, the powder metering wheel to reset and rotate; the outer cap continues to trigger, in the second cap-closing sub-load stroke, the powder metering wheel to reset and rotate and triggers the air compressing mechanism to complete resetting; and the outer cap triggers, in the third cap-closing sub-load stroke, only the powder metering wheel to reset and rotate, and triggers the powder metering wheel to reset and rotate to the first position.

The powder inhaler further includes: an inhalation trigger mechanism, including a dose protection plate and an air inlet baffle that are linked to and cooperate with each other, a resetting torsion spring, and a drive torsion spring, where the resetting torsion spring limits the air inlet baffle to an airflow channel; and the dose protection plate is limited by the air inlet baffle to an inlet of the inhalation channel and blocks the dose cup, where
the outer cap presses the resetting torsion spring in the cap-opening load stroke, and releases limitation of the resetting torsion spring on the air inlet baffle;
when negative pressure of the airflow channel exceeds a threshold, the air inlet baffle rotates under an action of an air flow to open the airflow channel and release limitation on the dose protection plate, and the dose protection plate rotates to deviate under an action of the drive torsion spring and unblocks the dose cup of the powder metering wheel; and
further, the outer cap further triggers, in the cap-closing load stroke, the dose protection plate and the air inlet baffle to reset.

The outer cap drives, in the first cap-closing sub-load stroke and the second cap-closing sub-load stroke via the powder metering wheel, the dose protection plate to reset to a position beyond the inlet of the inhalation channel, and compresses the drive torsion spring; the resetting torsion spring drives the air inlet baffle to reset and rotate and close the airflow channel; and
the outer cap is in the third cap-closing sub-load stroke, the powder metering wheel disengages from the dose protection plate, and the drive torsion spring drives the dose protection plate to rotate to the inlet of the inhalation channel, and is limited by the air inlet baffle at the inlet of the inhalation channel.

An angle of the outer cap at the first position is defined as 0 degrees, and an angle of the outer cap at the second position is greater than or equal to 120 degrees and less than or equal to 180 degrees;
preferably, the angle of the outer cap at the second position is 150 degrees; and
a critical angle between the cap-opening idle stroke and the cap-opening load stroke is greater than or equal to 10 degrees and less than or equal to 15 degrees; and/or a torque of the outer cap in the cap-opening idle stroke is greater than or equal to 0.05 N•m and less than or equal to 0.3 N•m, and a torque of the outer cap in the cap-opening load stroke is greater than or equal to 0 N•m and less than or equal to 0.15 N•m; and/or
the cap-opening idle stroke includes the first cap-opening sub-idle stroke and the second cap-opening sub-idle stroke following the first cap-opening sub-idle stroke, where a critical angle between the first cap-opening sub-idle stroke and the second cap-opening sub-idle stroke is greater than or equal to 6 degrees and less than or equal to 10 degrees; and/or a torque of the outer cap in the first cap-opening sub-idle stroke is greater than or equal to 0.02 N•m and less than or equal to 0.08 N•m, and a torque of the outer cap in the second cap-opening sub-idle stroke is greater than or equal to 0.1 N•m and less than or equal to 0.2 N•m; and/or
the cap-opening load stroke includes the first cap-opening sub-load stroke and the second cap-opening sub-load stroke following the first cap-opening sub-load stroke, where a critical angle between the first cap-opening sub-load stroke and the second cap-opening sub-load stroke is greater than or equal to 60 degrees and less than or equal to 65 degrees; and/or a torque of the outer cap in the first cap-opening sub-load stroke is constant, and is greater than or equal to 0 N•m and less than or equal to 0.05 N•m, and a torque of the outer cap in the second cap-opening sub-load stroke is constant, and is greater than or equal to 0.05 N•m and less than or equal to 0.15 N•m; and/or
a critical angle between the cap-closing idle stroke and the cap-closing load stroke is greater than or equal to 80 degrees and less than or equal to 95 degrees; and/or a maximum torque of the outer cap in the cap-closing idle stroke is greater than or equal to 0.03 N•m and less than or equal to 0.07 N•m, and a maximum torque of the outer cap in the cap-closing load stroke is greater than 0.05 N•m and less than or equal to 0.3 N•m; and/or
the cap-closing idle stroke includes the first cap-closing sub-idle stroke and the second cap-closing sub-idle stroke following the first cap-closing sub-idle stroke, where a critical angle between the first cap-closing sub-idle stroke and the second cap-closing sub-idle stroke is greater than or equal to 135 degrees and less than or equal to 145 degrees; and/or a torque of the outer cap in the first cap-closing sub-idle stroke is constant, and is greater than or equal to 0.03 N•m and less than or equal to 0.07 N•m, and a torque of the outer cap in the second cap-closing sub-idle stroke is constant, and is less than or equal to 0.02 N•m; and/or
the cap-closing load stroke includes the first cap-closing sub-load stroke, the second cap-closing sub-load stroke, and the third cap-closing sub-load stroke that are set in the chronological order, where a critical angle between the first cap-closing sub-load stroke and the second cap-closing sub-load stroke is greater than or equal to 60 degrees and less than or equal to 65 degrees, and a critical angle between the second cap-closing sub-load stroke and the third cap-closing sub-load stroke is greater than or equal to 6 degrees and less than or equal to 10 degrees; and/or a torque of the outer cap in the first cap-closing sub-load stroke is constant, and is greater than or equal to 0.05 N•m and less than or equal to 0.15 N•m, a torque of the outer cap in the second cap-closing sub-load stroke gradually increases, and a maximum value of the torque is greater than or equal to 0.15 N•m and less than or equal to 0.3 N•m, and a torque of the outer cap in the third cap-closing sub-load stroke is constant, and is greater than or equal to 0.05 N•m and less than or equal to 0.15 N•m.

To resolve the foregoing technical problem, a technical solution adopted by this application is to provide an air inlet baffle, configured for a powder inhaler, where the air inlet baffle includes:
a baffle body, where
a first surface of the baffle body has a boss, and a peripheral side surface of the boss is spaced apart from a peripheral side surface of the baffle body.

The boss covers a central region of the first surface of the baffle body.

The outer peripheral side surface of the boss is evenly spaced apart from the outer peripheral side surface of the baffle body along a circumferential direction of the boss; and a part of the first surface of the baffle body that is not covered by the boss forms an annular surface.

The air inlet baffle further includes a rotating shaft, disposed at a first end of the baffle body; and
a height of the boss gradually decreases along a direction from the first end of the baffle body to an opposite second end of the baffle body, to enable a top surface of the boss to form an inclined surface.

The baffle body is recessed to form the boss.

The air inlet baffle further includes a rotating shaft and a rotating member; the rotating shaft is disposed at one end of the baffle body; and the rotating member is connected to a free end of the rotating shaft and is spaced apart from the baffle body, where
a first end of the rotating member has a curved surface.

A surface of a second end of the rotating member that is away from the baffle body has a protruding cylinder.

A quantity of rotating shafts is two, and the rotating shafts are respectively disposed on two opposite sides of the baffle body and are defined as a first rotating shaft and a second rotating shaft; a quantity of rotating members is two, and the rotating members are defined as a first rotating member and a second rotating member;
the first rotating member is connected to a free end of the first rotating shaft, a first end of the first rotating member has a first curved surface, and a surface of a second end of the first rotating member that is away from the baffle body has the protruding cylinder; and
the second rotating member is connected to a free end of the second rotating shaft, a first end of the second rotating member has a second curved surface, and a second end of the second rotating member has a circular-arc-shaped groove surface.

A quantity of rotating shafts is two, and the rotating shafts are respectively disposed on two opposite sides of the baffle body; one end of each rotating shaft is connected to a side surface of the baffle body; and
the side surface of the baffle body further has a shoulder surrounding the rotating shaft, and the shoulder is spaced apart from the rotating member.

To resolve the foregoing technical problem, another technical solution adopted by this application is to provide a dose protection plate, configured for a powder inhaler, where the dose protection plate includes:
an annular body; and
a blocking portion, connected to one end of the annular body and configured to block or unblock a dose cup of the powder inhaler.

The dose protection plate further includes a pressing member, and the pressing member is disposed on an outer side surface of the annular body; and a surface of the pressing member that is away from the annular body includes a pressing arc surface.

The pressing member includes a cylindrical convex surface, and the cylindrical convex surface is disposed on one side of the pressing arc surface.

The dose protection plate further includes an elastic arm claw, and one end of the elastic arm claw is connected to the annular body.

The dose protection plate further includes a wedge-shaped post, and one end of the wedge-shaped post is connected to the annular body.

To resolve the foregoing technical problem, another technical solution adopted by this application is to provide an inhalation trigger mechanism, including:
any air inlet baffle as described above; and/or
any dose protection plate as described above.

To resolve the foregoing technical problem, another technical solution adopted by this application is to provide a powder inhaler, including:
a powder delivery mechanism, including a powder container and a powder metering wheel, where the powder container has a storage cavity, an inhalation channel, and an airflow channel; the inhalation channel is in communication with the airflow channel; the storage cavity is configured to store powder, and the storage cavity has a powder outlet; the powder metering wheel is rotatably connected to the powder container; the powder metering wheel includes a dose cup; the dose cup is configured to receive the powder from the powder outlet and deliver the powder to an inlet of the inhalation channel; and
the inhalation trigger mechanism as described above, where the air inlet baffle is rotatably connected to a side wall of the airflow channel, and the first surface of the baffle body faces an outer side of a port of the airflow channel; the dose protection plate is rotatably connected to the powder container; and the air inlet baffle is linked to and cooperates with the dose protection plate, where
when the inhalation trigger mechanism is in an initial state, the air inlet baffle seals the airflow channel, and the inhalation channel is not in communication with external air; the blocking portion blocks the powder outlet of the dose cup that is located at the inlet of the inhalation channel; and
when negative pressure in the inhalation channel exceeds a threshold, the air inlet baffle rotates to enable the airflow channel to be opened and triggers the dose protection plate to rotate, to enable the blocking portion to deviate and unblock the powder outlet of the dose cup, and the airflow channel is in communication with the external air and the inhalation channel.

The powder inhaler further includes:
a housing assembly, including a front housing, where the front housing has a suction nozzle, and the suction nozzle is disposed corresponding to the inhalation channel and is in communication with the inhalation channel; a side wall of the front housing is provided with an air inlet and a grille, and the grille protrudes out of an outer wall surface of the front housing; an inner wall surface of the front housing is provided with an annular flange surrounding the air inlet, one end of the annular flange is disposed in the airflow channel, and the side wall of the front housing seals the port of the airflow channel;
the air inlet baffle has the annular surface; when the inhalation trigger mechanism is in an initial state, the boss is embedded in the annular flange, an inner peripheral surface of the annular flange is spaced apart from and cooperates with an outer peripheral surface of the boss to form a first flow channel segment, an end surface at one end of the annular flange that is away from the front housing abuts against and cooperates with the annular surface to form a second flow channel segment, and the first flow channel segment and the second flow channel segment form an L-shaped air inlet flow channel; the pressing arc surface cooperates with a circular-arc-shaped groove surface of the air inlet baffle to implement concentric arc surface pressing; and
when the negative pressure in the inhalation channel exceeds the threshold, the air inlet baffle rotates to enable the airflow channel to be opened, and the airflow channel is in communication with the external air through the air inlet.

A ratio of a distance between the top surface of the boss and the first surface of the baffle body to a thickness of the annular flange is 1:2 to 7:1.

The side wall of the front housing is provided with at least two air inlets and at least one grille, and the grille and the air inlets are alternately provided; the annular flange is disposed around the air inlets and the grille;
the side wall of the airflow channel is connected to a side wall of the storage cavity, and one end of the side wall of the airflow channel that is connected to the side wall of the storage cavity is provided with a first flow guiding hole and a second flow guiding hole that are spaced apart from each other; one end of the inhalation channel that is close to the suction nozzle has a first airflow inlet and a second airflow inlet that are spaced apart from each other, the first flow guiding hole is in communication with the airflow channel and the first airflow inlet, and the second flow guiding hole is in communication with the airflow channel and the second airflow inlet; and
the inner wall surface of the front housing is spaced apart from an outer wall surface of the inhalation channel to form a flow guiding channel, and the flow guiding channel is in communication with the airflow channel, the first airflow inlet, and the second airflow inlet.

The powder inhaler further includes:
an outer cap, rotatably connected to the housing assembly and capable of rotating back and forth between a first position and a second position, where the outer cap blocks the suction nozzle and the air inlet when the outer cap is configured to be at the first position; and the suction nozzle and the air inlet are exposed when the outer cap is configured to be at the second position; and
an air compressing mechanism, disposed on the powder container, and configured to press the powder in the powder container into the dose cup, where
the powder metering wheel is capable of rotating back and forth between a third position and a fourth position; when the powder metering wheel is configured to be at the third position, the dose cup is disposed corresponding to the powder outlet of the storage cavity; when the powder metering wheel is configured to be at the fourth position, the dose cup is disposed corresponding to the inlet of the inhalation channel;
the outer cap is linked to and cooperates with the powder delivery mechanism and the air compressing mechanism respectively; in a process in which the outer cap rotates from the first position to the second position, the outer cap first drives the air compressing mechanism to press the powder in the storage cavity into the dose cup, and then drives the powder metering wheel to rotate from the third position to the fourth position; and
in a process in which the outer cap reversely rotates and resets from the second position to the first position, the outer cap drives the powder metering wheel, the dose protection plate, and the air compressing mechanism respectively to reset, and triggers the air inlet baffle to reversely rotate and reset.

To resolve the foregoing technical problem, a technical solution adopted by this application is to provide an airbag pressing member, configured for a powder inhaler. A side wall of the airbag pressing member has a pressure relief hole; and the airbag pressing member is configured to press an air compressing airbag.

One end of the side wall of the airbag pressing member that is away from a top wall of the airbag pressing member has a protruding rod, and one end of the protruding rod that is away from the top wall has an arc surface.

One end of the protruding rod that is away from the top wall has a tip, and an end surface of the tip is the arc surface.

The arc surface is a circular arc surface.

The top wall of the airbag pressing member has a fixing hole, and the fixing hole is configured to connect to a top portion of the air compressing airbag, so as to drive the air compressing airbag to extend and contract.

To resolve the foregoing technical problem, another technical solution adopted by this application is to provide a drive cam, configured for a powder inhaler, where the drive cam includes:
a body portion; and
a gear, coaxially connected to the body portion, and configured to drive the body portion to rotate, where
a surface of the body portion has a guide groove, and a side surface of the guide groove is a cam curved surface.

An outer peripheral side surface of the body portion has an arc-shaped groove located at one end of the cam curved surface.

The gear is disposed on a surface of the body portion, and the guide groove is provided on a surface of the body portion that faces the gear and is spaced apart from the gear;
the cam curved surface includes a first curved surface segment and a second curved surface segment that are connected to each other, and the second curved surface segment is located at one end of the first curved surface segment that is away from the arc-shaped groove; and the first curved surface segment is a non-circular arc surface, and the second curved surface segment is a circular arc surface and is disposed concentric to the peripheral side surface of the body portion.

A stop groove is provided at one end of the second curved surface segment that is away from the first curved surface segment.

The arc-shaped groove and/or the stop groove are/is a circular-arc-shaped groove.

A surface of the body portion that faces away from the gear has a rib; and one end of the rib is disposed corresponding to an end portion of the second curved surface segment that is close to the first curved surface segment.

A surface of the body portion that faces away from the gear further has an annular boss; and the annular boss is disposed coaxially with the gear.

To resolve the foregoing technical problem, another technical solution adopted by this application is to provide an air compressing mechanism, including:
an air compressing airbag;
an elastic member;
any airbag pressing member as described above; and/or
any drive cam as described above.

The arc surface of the protruding rod cooperates with the cam curved surface of the drive cam to enable the airbag pressing member to move back and forth between a fifth position and a sixth position; and
when the airbag pressing member is configured to be at an initial position, the tip of the protruding rod is embedded in the arc-shaped groove of the cam curved surface, to initially position the airbag pressing member.

To resolve the foregoing technical problem, another technical solution adopted by this application is to provide a powder inhaler, including:
a powder delivery mechanism, including a powder container, where the powder container has a storage cavity, a first end of the storage cavity has a powder outlet, and a second end of the storage cavity has an air compressing port; and a side wall of the storage cavity has a vent hole; and
the air compressing mechanism as described above, where
the air compressing airbag is disposed at the second end of the storage cavity and is in communication with the air compressing port; the airbag pressing member is movably sleeved on an outer side of the air compressing airbag and an outer side of the storage cavity;
the drive cam and the elastic member are configured to drive the airbag pressing member to move back and forth between the fifth position and the sixth position, so as to drive the air compressing airbag to extend and contract; when the airbag pressing member is configured to be at the fifth position, the side wall of the airbag pressing member seals the vent hole, and the pressure relief hole is not in communication with the vent hole; and when the airbag pressing member is configured to be at the sixth position, the pressure relief hole is in communication with the vent hole, to relieve pressure in the storage cavity.

The powder inhaler further includes:
a filter membrane, disposed at the second end of the storage cavity, where the filter membrane is located at the air compressing port and is spaced apart from a port of the air compressing port; and one end of the vent hole is in communication with space between the filter membrane and the air compressing airbag;
a housing assembly, having a suction nozzle; and
an outer cap, rotatably connected to the housing assembly and capable of rotating back and forth between a first position and a second position, where the outer cap blocks the suction nozzle when the outer cap is configured to be at the first position; the suction nozzle is exposed when the outer cap is configured to be at the second position;
the powder delivery mechanism further includes a powder metering wheel; the powder container further has an inhalation channel, and the suction nozzle is in communication with the inhalation channel; the powder metering wheel is rotatably connected to the powder container; the powder metering wheel includes a dose cup; the powder metering wheel is capable of rotating back and forth between a third position and a fourth position; when the powder metering wheel is configured to be at the third position, the dose cup is disposed corresponding to the powder outlet of the storage cavity and is configured to receive the powder in the powder container; and when the powder metering wheel is configured to be at the fourth position, the dose cup is disposed corresponding to an inlet of the inhalation channel, where
the outer cap is linked to and cooperates with the powder metering wheel and the air compressing mechanism respectively; when the outer cap is configured to be at the first position, the airbag pressing member is limited at the fifth position; in a process in which the outer cap rotates from the first position to the second position, limitation on the airbag pressing member is first released, to enable the elastic member to drive the airbag pressing member to move from the fifth position to the sixth position, and then drive the powder metering wheel to rotate from the third position to the fourth position; and
in a process in which the outer cap reversely rotates and resets from the second position to the first position, the outer cap drives the powder metering wheel to reversely rotate and reset, and drives the airbag pressing member to move in a reverse direction and reset to the fifth position.

Beneficial effects of this application are as follows: Different from the related art, this application discloses a powder inhaler, an air inlet baffle, a dose protection plate, an inhalation trigger mechanism, an airbag pressing member, a drive cam, and an air compressing mechanism. The powder inhaler includes a functional mechanism, a suction nozzle, and an outer cap. The functional mechanism includes an inhalation channel; the suction nozzle is in communication with the inhalation channel; the outer cap is linked to and cooperates with the functional mechanism, and is limited to rotate back and forth between a first position and a second position, where the outer cap blocks the suction nozzle when the outer cap is configured to be at the first position, and the outer cap unblocks the suction nozzle when the outer cap is configured to be at the second position. A stroke of the outer cap rotating from the first position to the second position includes a cap-opening idle stroke and a cap-opening load stroke following the cap-opening idle stroke, where the outer cap does not trigger an action of the functional mechanism in the cap-opening idle stroke; and the outer cap triggers, in the cap-opening load stroke, the functional mechanism to deliver powder to the inhalation channel; and/or a stroke of the outer cap rotating and resetting from the second position to the first position includes a cap-closing idle stroke and a cap-closing load stroke following the cap-closing idle stroke; and the outer cap does not trigger an action of the functional mechanism in the cap-closing idle stroke; and the outer cap triggers, in the cap-closing load stroke, the functional mechanism to reset. Through the foregoing setting, the powder inhaler implements effective linkage between various functional mechanisms during cap opening and closing, thereby enhancing performance of the powder inhaler.

### BRIEF DESCRIPTION OF THE DRAWINGS

To describe the technical solutions in embodiments of this application or the related art more clearly, the following briefly describes the accompanying drawings required for describing the embodiments or the related art. Apparently, the accompanying drawings in the following description show merely some embodiments of this application, and a person of ordinary skill in the art may still derive other drawings from the accompanying drawings without creative efforts.
FIG. 1 is a schematic diagram of a structure of a powder inhaler in a cap-closing state according to an embodiment of this application;
FIG. 2 is a schematic diagram of a structure of the powder inhaler provided in FIG. 1 in a cap-opening state;
FIG. 3 is a schematic diagram of a structure of the powder inhaler provided in FIG. 1 in another cap-opening state;
FIG. 4 is a schematic diagram of a structure of the powder inhaler that is provided in FIG. 3 and that is viewed from another angle;
FIG. 5A is a schematic diagram of a structure of an outer cap of the powder inhaler that is provided in FIG. 1 and that is viewed from an angle;
FIG. 5B is a schematic diagram of a structure of the outer cap provided in FIG. 5A that is viewed from another angle;
FIG. 6A is a schematic diagram of a structure of the powder inhaler that is provided in FIG. 1 with an outer cap removed and that is viewed from an angle;
FIG. 6B is a schematic diagram of a structure of the powder inhaler that is provided in FIG. 1 with an outer cap removed and that is viewed from another angle;
FIG. 7A is a schematic diagram of a structure of the powder inhaler that is provided in FIG. 2 and that is viewed from another angle;
FIG. 7B is a schematic partially enlarged view of the powder inhaler provided in FIG. 7A;
FIG. 8A is a schematic diagram of a structure of the powder inhaler provided in FIG. 1 with an outer cap removed;
FIG. 8B is a schematic partially enlarged view of the powder inhaler provided in FIG. 8A;
FIG. 9A is a schematic cross-sectional view of the powder inhaler that is provided in FIG. 1 with an outer cap removed and that is in a state;
FIG. 9B is a schematic cross-sectional view of the powder inhaler that is provided in FIG. 1 with an outer cap removed and that is in another state;
FIG. 9C is a schematic cross-sectional view of the powder inhaler that is provided in FIG. 1 with an outer cap removed and that is viewed from another angle in a state;
FIG. 9D is a schematic cross-sectional view of the powder inhaler that is provided in FIG. 1 with an outer cap removed and that is viewed from another angle in another state;
FIG. 9E is a schematic partially enlarged view of a region A in FIG. 9C;
FIG. 9F is a schematic partially enlarged view of a region A in FIG. 9D;
FIG. 9G is a schematic partially enlarged view of a region B in FIG. 9C;
FIG. 9H is a schematic diagram of an L-shaped air inlet flow channel of the powder inhaler provided in FIG. 9C;
FIG. 10A is a schematic diagram of a structure of a drive cam that is of the powder inhaler provided in FIG. 1 and that is viewed from an angle;
FIG. 10B is a schematic diagram of a structure of the drive cam provided in FIG. 10A that is viewed from another angle;
FIG. 10C is a schematic diagram of a structure of the drive cam that is provided in FIG. 10A and that is viewed from still another angle;
FIG. 11 is a schematic diagram of a structure of an airbag pressing member of the powder inhaler provided in FIG. 1;
FIG. 12A is a schematic diagram of a structure of a powder metering wheel of a powder delivery mechanism of the powder inhaler provided in FIG. 1 being at a third position in a powder container;
FIG. 12B is a schematic diagram of a structure of a powder metering wheel of a powder delivery mechanism of the powder inhaler provided in FIG. 1 being at a fourth position in a powder container;
FIG. 13A is a schematic diagram of a structure of a powder container that is of the powder inhaler provided in FIG. 1 and that is viewed from an angle;
FIG. 13B is a schematic diagram of a structure of the powder container that is provided in FIG. 13A and that is viewed from another angle;
FIG. 14A is a schematic diagram of a structure of a powder metering wheel that is of the powder inhaler provided in FIG. 1 and that is viewed from an angle;
FIG. 14B is a schematic diagram of a structure of the powder metering wheel that is provided in FIG. 14A and that is viewed from another angle;
FIG. 15A is a schematic diagram of a structure of an inhalation trigger device that is of the powder inhaler provided in FIG. 1 and that is in a state in a powder container;
FIG. 15B is a schematic diagram of a structure of the inhalation trigger device that is provided in FIG. 15A and that is in another state in a powder container;
FIG. 16A is a schematic diagram of a structure of the inhalation trigger device provided in FIG. 15A with a powder container removed;
FIG. 16B is a schematic diagram of a structure of the inhalation trigger device provided in FIG. 15B with a powder container removed;
FIG. 17A is a schematic diagram of a structure of the inhalation trigger device that is provided in FIG. 16A and that is viewed from another angle;
FIG. 17B is a schematic diagram of a structure of the inhalation trigger device that is provided in FIG. 16B and that is viewed from another angle;
FIG. 18A is a schematic diagram of an exploded structure of a counting mechanism of the powder inhaler provided in FIG. 1;
FIG. 18B is a schematic diagram of an assembly structure of the counting mechanism provided in FIG. 18A;
FIG. 19A is a schematic diagram of a structure of a counter base that is of the counting mechanism provided in FIG. 18A and that is viewed from an angle;
FIG. 19B is a schematic diagram of a structure of the counter base that is provided in FIG. 19A and that is viewed from another angle;
FIG. 20 is a schematic diagram of a structure of a dose protection plate of an inhalation trigger device of the powder inhaler provided in FIG. 1;
FIG. 21A is a schematic cross-sectional view of assembly of a powder metering wheel and a dose protection plate that are of the powder inhaler provided in FIG. 1 and that are in a state;
FIG. 21B is a schematic cross-sectional view of assembly of a powder metering wheel and a dose protection plate that are of the powder inhaler provided in FIG. 1 and that are in another state;
FIG. 21C is a partially enlarged schematic diagram of FIG. 21A;
FIG. 21D is a partially enlarged schematic diagram of FIG. 21B;
FIG. 21E is a schematic cross-sectional view of assembly of a powder metering wheel and a dose protection plate that are of the powder inhaler provided in FIG. 1 and that are in still another state;
FIG. 21F is a partially enlarged schematic diagram of FIG. 21E;
FIG. 22A is a schematic diagram of a structure of an air inlet baffle that is of an inhalation trigger device of the powder inhaler provided in FIG. 1 and that is viewed from an angle;
FIG. 22B is a schematic diagram of a structure of the air inlet baffle that is provided in FIG. 21A and that is viewed from another angle;
FIG. 22C is a schematic diagram of a structure of the air inlet baffle that is provided in FIG. 22A and that is viewed from still another angle;
FIG. 23A is a schematic diagram of a structure of a front housing that is of the powder inhaler provided in FIG. 1 and that is viewed from an angle;
FIG. 23B is a schematic diagram of a structure of a front housing that is of the powder inhaler provided in FIG. 1 and that is viewed from another angle;
FIG. 24A is a schematic diagram of a structure of a units digit wheel that is of the counting mechanism provided in FIG. 18A and that is viewed from an angle;
FIG. 24B is a schematic diagram of a structure of the units digit wheel that is provided in FIG. 24A and that is viewed from another angle;
FIG. 25 is a schematic diagram of a structure of a tens digit wheel of the counting mechanism provided in FIG. 18A;
FIG. 26 is a schematic cross-sectional view of another embodiment of a powder inhaler according to this application;
FIG. 27 is a schematic cross-sectional view of still another embodiment of a powder inhaler according to this application;
FIG. 28 is a schematic diagram of a cycle of a cap opening and closing process of the powder inhaler provided in FIG. 1;
FIG. 29A is a schematic diagram of a curve of a cap-opening angle and a torque in an implementation of a cap-opening process of the powder inhaler provided in FIG. 1;
FIG. 29B is a schematic diagram of a curve of a cap-closing angle and a torque in an implementation of a cap-closing process of the powder inhaler provided in FIG. 1;
FIG. 30A is a schematic diagram of a curve of a cap-opening angle and a torque in another implementation of a cap-opening process of the powder inhaler provided in FIG. 1;
FIG. 30B is a schematic diagram of a curve of a cap-closing angle and a torque in another implementation of a cap-closing process of the powder inhaler provided in FIG. 1;
FIG. 31A is a schematic diagram of a bottom view structure of the powder inhaler that is provided in FIG. 1 and that is viewed from an angle;
FIG. 31B is a schematic diagram of the powder inhaler that is provided in FIG. 31A and that is in a state of being placed on a horizontal surface;
FIG. 31C is a schematic diagram of the powder inhaler that is provided in FIG. 31A and that is in a handheld state;
FIG. 31D is a schematic diagram of the powder inhaler that is provided in FIG. 31A with a cap opened and that is in a handheld state; and
FIG. 31E is a schematic diagram of the powder inhaler that is provided in FIG. 31A and that is in an oral inhalation state.

### DETAILED DESCRIPTION

The technical solutions in embodiments of this application are clearly and completely described in the following with reference to the accompanying drawings in the embodiments of this application. Apparently, the described embodiments are merely some rather than all of the embodiments of this application. All other embodiments obtained by a person skilled in the art based on the embodiments of this application without creative efforts shall fall within the protection scope of this application.

In this embodiment of this application, the terms "first", "second" and "third" are used merely for the purpose of description, and shall not be construed as indicating or implying relative importance or implying a quantity of indicated technical features. Therefore, features defining "first" "second" and "third" can explicitly or implicitly include at least one of the features. In descriptions of this application, "more" means at least two, such as two and three unless it is specifically defined otherwise. In addition, the terms "include", "have", and any variant thereof are intended to cover a non-exclusive inclusion. For example, a process, method, system, product, or device that includes a series of steps or units is not limited to the listed steps or units; and instead, further optionally includes a step or unit that is not listed, or further optionally includes another step or unit that is intrinsic to the process, method, product, or device.

"Embodiment" mentioned in this specification means that particular features, structures, or characteristics described with reference to the embodiment may be included in at least an implementation of this application. The term appearing at different positions of this specification may not refer to the same embodiment or an independent or alternative embodiment that is mutually exclusive with another embodiment. A person skilled in the art explicitly or implicitly understands that the embodiments described in this specification may be combined with other embodiments.

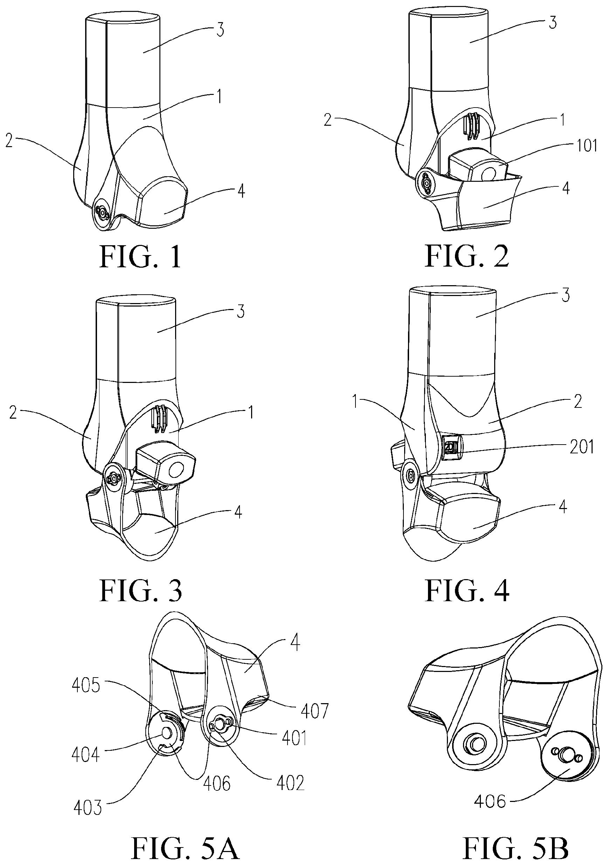

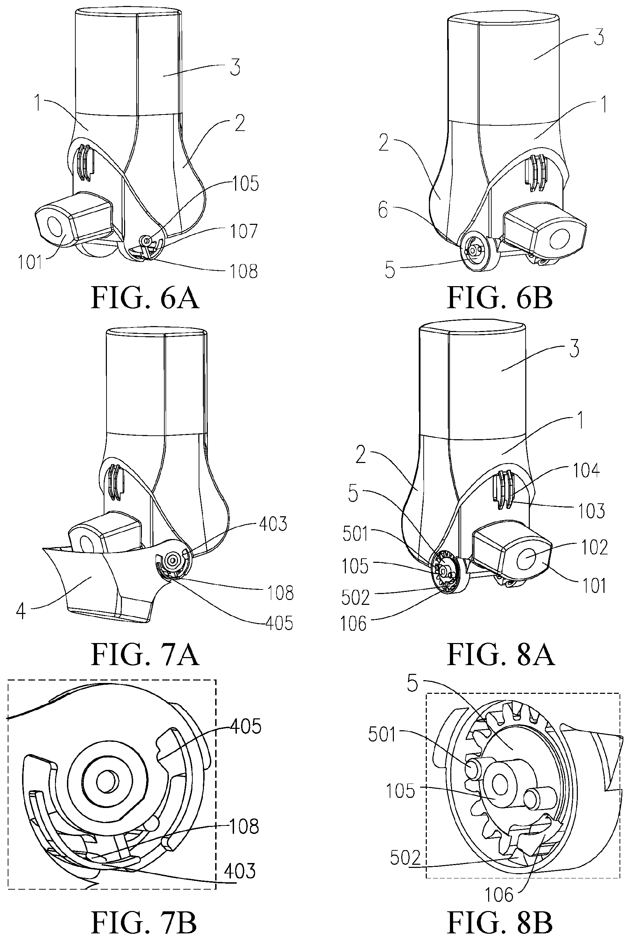

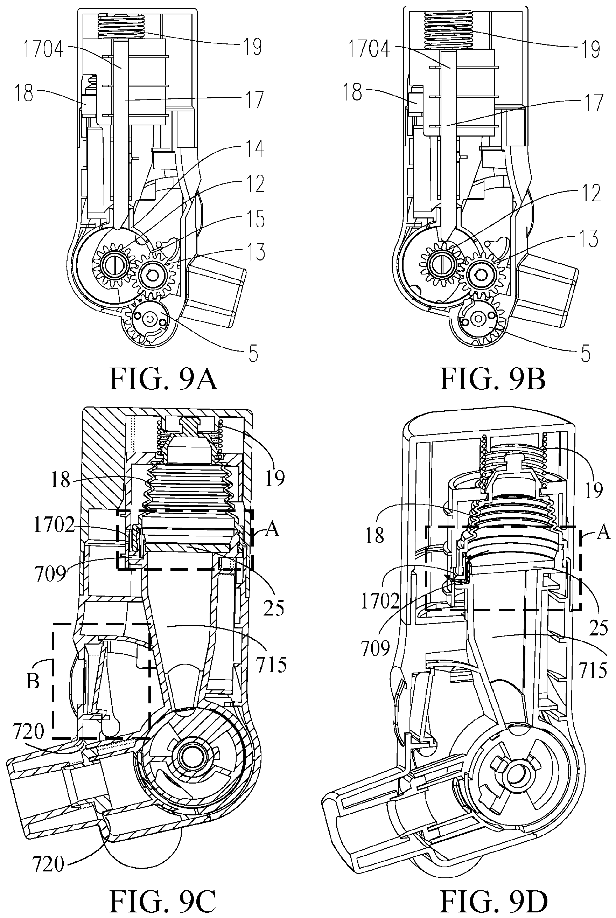

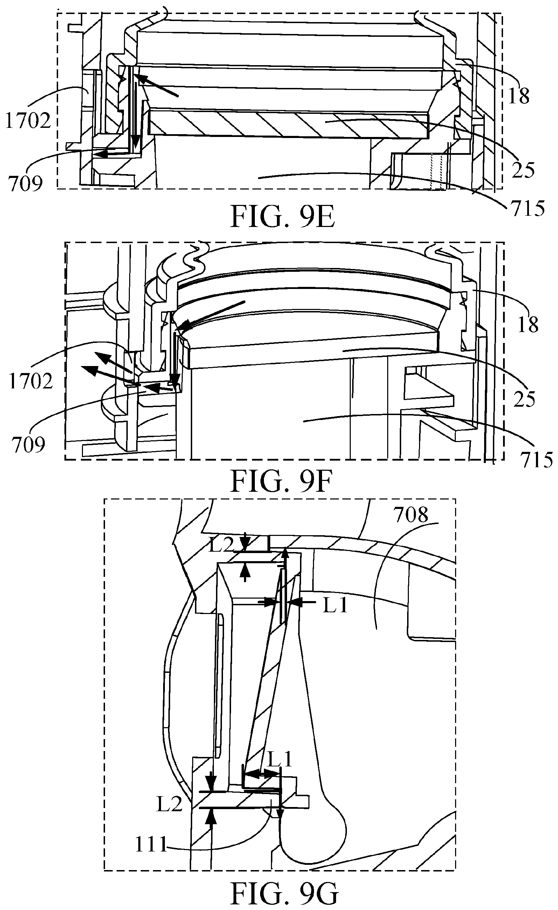

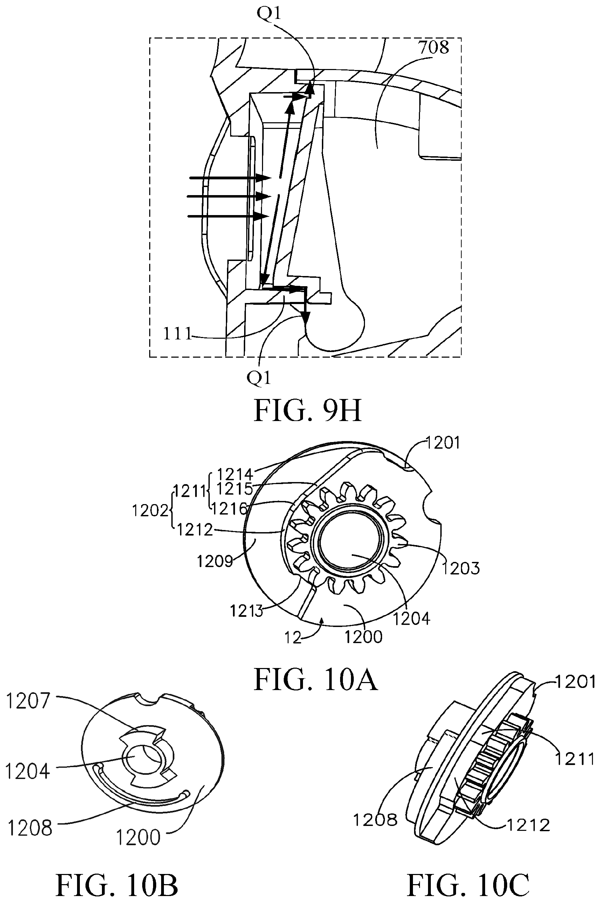

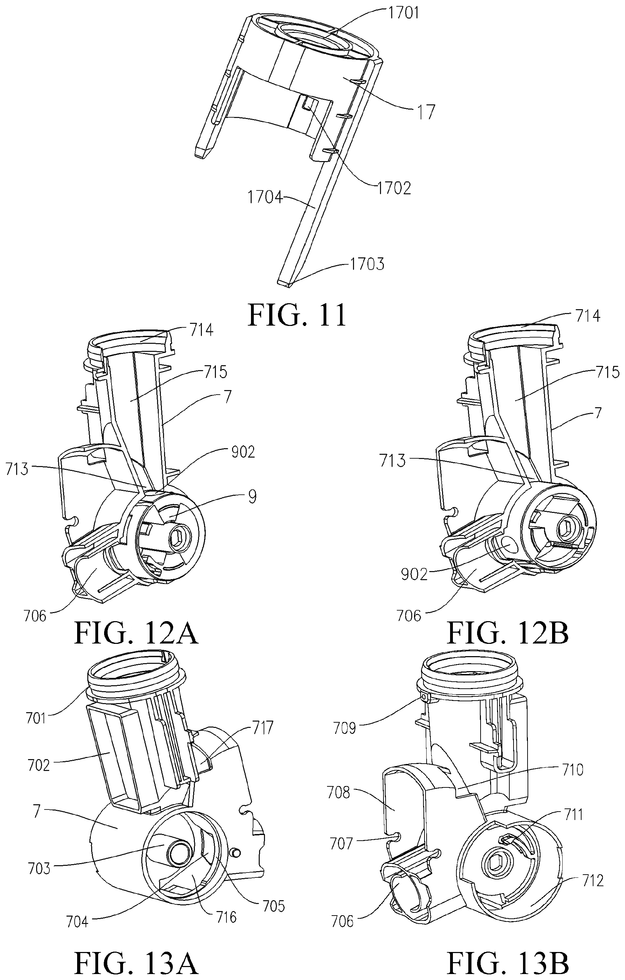

Refer to FIG. 1 to FIG. 11. FIG. 1 is a schematic diagram of a structure of a powder inhaler in a cap-closing state according to an embodiment of this application; FIG. 2 is a schematic diagram of a structure of the powder inhaler provided in FIG. 1 in a cap-opening state; FIG. 3 is a schematic diagram of a structure of the powder inhaler provided in FIG. 1 in another cap-opening state; FIG. 4 is a schematic diagram of a structure of the powder inhaler that is provided in FIG. 3 and that is viewed from another angle; FIG. 5A is a schematic diagram of a structure of an outer cap of the powder inhaler that is provided in FIG. 1 and that is viewed from an angle; FIG. 5B is a schematic diagram of a structure of the outer cap that is provided in FIG. 5A and that is viewed from another angle; FIG. 6A is a schematic diagram of a structure of the powder inhaler that is provided in FIG. 1 with an outer cap removed and that is viewed from an angle; FIG. 6B is a schematic diagram of a structure of the powder inhaler that is provided in FIG. 1 with an outer cap removed and that is viewed from another angle; FIG. 7A is a schematic diagram of a structure of the powder inhaler that is provided in FIG. 2 and that is viewed from another angle; FIG. 7B is a schematic partially enlarged view of the powder inhaler provided in FIG. 7A; FIG. 8A is a schematic diagram of a structure of the powder inhaler provided in FIG. 1 with an outer cap removed; FIG. 8B is a schematic partially enlarged view of the powder inhaler provided in FIG. 8A; FIG. 9A is a schematic cross-sectional view of the powder inhaler that is provided in FIG. 1 with an outer cap removed and that is in a state; FIG. 9B is a schematic cross-sectional view of the powder inhaler that is provided in FIG. 1 with an outer cap removed and that is in another state; FIG. 9C is a schematic cross-sectional view of the powder inhaler that is provided in FIG. 1 with an outer cap removed and that is viewed from another angle in a state; FIG. 9D is a schematic cross-sectional view of the powder inhaler that is provided in FIG. 1 with an outer cap removed and that is viewed from another angle in another state; FIG. 9E is a schematic partially enlarged view of a region A in FIG. 9C; FIG. 9F is a schematic partially enlarged view of a region A in FIG. 9D; FIG. 9G is a schematic partially enlarged view of a region B in FIG. 9C; FIG. 9H is a schematic diagram of an L-shaped air inlet flow channel of the powder inhaler provided in FIG. 1; FIG. 10A is a schematic diagram of a structure of a drive cam that is of the powder inhaler provided in FIG. 1 and that is viewed from an angle; FIG. 10B is a schematic diagram of a structure of the drive cam that is provided in FIG. 10A and that is viewed from another angle; FIG. 10C is a schematic diagram of a structure of the drive cam that is provided in FIG. 10A and that is viewed from still another angle; and FIG. 11 is a schematic diagram of a structure of an airbag pressing member of the powder inhaler provided in FIG. 1.

Referring to FIG. 1 to FIG. 4, this application provides a powder inhaler. The powder inhaler includes a housing assembly (not labeled in the figure), a functional mechanism (not shown in FIG. 1 to FIG. 4), and an outer cap 4. The functional mechanism is disposed in the housing assembly, and the outer cap 4 is connected to the housing assembly and can be limited to rotate back and forth between a first position and a second position. When the outer cap 4 is at the first position, the outer cap 4 is in a closed state; and when the outer cap 4 is at the second position, the outer cap 4 is in a fully opened state. The outer cap 4 is linked to and cooperates with the functional mechanism, and a linked action of each functional mechanism is implemented by rotating back and forth between the first position and the second position by the outer cap 4, so that the powder inhaler implements a function of dispensing powder such as drug powder. Rotating back and forth in this application refers to rotating back and forth based on a repeated path, and directions of the two times of rotation are opposite, for example, rotating clockwise from the first position to the second position, and then rotating counterclockwise from the second position back to the first position.

Specifically, the housing assembly includes a front housing 1, a rear housing 2, and an upper housing 3. The front housing 1, the rear housing 2, and the upper housing 3 are connected to each other and cooperate with each other to form accommodating space. The functional mechanism is disposed in the accommodating space. The outer cap 4 is rotatably connected to a bottom end of the housing assembly, and therefore can rotate back and forth between the first position and the second position, to implement a cap-opening process and a cap-closing process. The outer cap 4 may be rotatably connected to the bottom end of the housing assembly through a rotating shaft or an arc-shaped slide rail. The front housing 1, the rear housing 2, the upper housing 3, and the outer cap 4 are not limited in shape and structure, and may be made of a material such as metal or plastic.

Referring to FIG. 1 and FIG. 5A to FIG. 9B, the outer cap 4 includes two connecting portions 406 oppositely disposed along a first direction, and the two connecting portions 406 are respectively rotatably connected to two opposite sides of the bottom end of the housing assembly and protrude from the bottom end of the housing assembly. Specifically, the two connecting portions 406 of the outer cap 4 are assembled and connected to the housing assembly through rotating shaft-to-hole engagement. Referring to FIG. 5A, FIG. 5B, FIG. 6A, FIG. 8A, FIG. 8B, FIG. 9A, and FIG. 9B, the powder inhaler further includes a drive gear 5, the drive gear 5 is disposed in the housing assembly, and an end surface of the drive gear 5 is provided with a drive shaft 501. One connecting portion 406 of the outer cap 4 is provided with a first shaft hole 401 and a drive hole 402, and the other connecting portion 406 of the outer cap 4 is provided with a second shaft hole 404. The front housing 1 corresponding to the first shaft hole 401 and the second shaft hole 404 respectively is provided with a cylinder 105, and the cylinder 105 is defined as a first cylinder. The first shaft hole 401 and the second shaft hole 404 of the outer cap 4 fit with the corresponding cylinder 105 of the front housing 1, and the drive hole 402 of the outer cap 4 fits with the drive shaft 501 of the drive gear 5, so that the outer cap 4 is rotatably connected to the bottom end of the housing assembly.

As shown in FIG. 6B, the powder inhaler further includes a sealing ring 6, and the sealing ring 6 is disposed between the connecting portion 406 of the outer cap 4 and the drive gear 5 for sealing, to ensure consistency of an airflow channel and consistency of inhalation resistance of the powder inhaler, and prevent air from entering the housing assembly from a position between the drive gear 5 and the connecting portion 406 of the outer cap 4.

Referring to FIG. 5A, FIG. 6A, FIG. 7A, and FIG. 7B, one connecting portion 406 of the outer cap 4 is further provided with an arc-shaped rib 405, and the front housing 1 is provided with a sound elastic arm 108. The arc-shaped rib 405 on the outer cap 4 is configured to cooperate with the sound elastic arm 108 on the front housing 1, to provide an audible prompt indicating that the cap is fully opened when the outer cap 4 rotates from the first position to the second position. Specifically, in a cap-opening process of the outer cap 4, that is, in a process in which the outer cap 4 rotates from the first position to the second position, the sound elastic arm 108 is located on an outer side of the arc-shaped rib 405, and the sound elastic arm 108 moves along an outer periphery of the arc-shaped rib 405. After the outer cap 4 is fully opened, that is, when the outer cap 4 rotates to the second position, the sound elastic arm 108 engages with the arc-shaped rib 405 to provide the audible prompt indicating that the cap is fully opened. In a cap-closing process of the outer cap 4, that is, in a process in which the outer cap 4 rotates and resets from the second position to the first position, the outer cap 4 is located on an inner side of the arc-shaped rib 405, and the outer cap 4 moves along an inner periphery of the arc-shaped rib 405. In addition, after the outer cap 4 is fully closed, that is, when the outer cap 4 rotates to the first position, the sound elastic arm 108 engages with the arc-shaped rib 405 to provide an audible prompt indicating that the cap is fully closed. For example, in a preferred implementation, an angle of the outer cap 4 at the first position is defined as 0 degrees, and an angle of the outer cap 4 at the second position relative to the first position is 150 degrees. In the cap-opening process, when the outer cap 4 rotates from 0 degrees to 150 degrees, that is, when a rotation angle of the outer cap 4 is 150 degrees, the arc-shaped rib 405 of the outer cap 4 contacts the sound elastic arm 108 of the front housing 1, to implement an audible prompt function for indicating that the cap is fully opened. The angle of the outer cap 4 at the second position is not limited to 150 degrees and may be designed as needed. For example, the angle may be greater than or equal to 120 degrees and less than or equal to 180 degrees, provided that a suction nozzle 101 can be exposed when the outer cap 4 is at the second position.

Referring to FIG. 6A and FIG. 12A to FIG. 13B, the powder inhaler has an inhalation channel 706 and a suction nozzle 101. The suction nozzle 101 is in communication with the inhalation channel 706, to facilitate a user in inhaling drug powder from a position of an outlet 102 of the suction nozzle 101. Specifically, the housing assembly includes a suction nozzle 101, and the suction nozzle 101 is disposed on the front housing 1 and is sleeved on the inhalation channel 706. When the outer cap 4 is configured to be at the first position, the outer cap 4 blocks the outlet 102 of the suction nozzle 101; and when the outer cap 4 is configured to be at the second position, the outlet 102 of the suction nozzle 101 is exposed.

Specifically, as shown in FIG. 5A, the connecting portion 406 of the outer cap 4 are provided with two drive holes 402, the two drive holes 402 are respectively provided on two sides of the first shaft hole 401, an end surface of the drive gear 5 is provided with two drive shafts 501, the two drive shafts 501 are respectively disposed on two sides of the cylinder 105 of the front housing 1, and the two drive holes 402 fit with and are connected to the two drive shafts 501 in one-to-one correspondence. In another implementation, the drive holes 402 and the drive shafts 501 may also be correspondingly set in other quantities such as one, three, or the like. It can be understood that, a connection manner between the outer cap 4 and the drive gear 5 is not limited to the foregoing manner, and the outer cap 4 and the drive gear 5 may also be integrally formed, adhesively bonded, or welded, provided that rotation of the outer cap 4 can drive the drive gear 5 to rotate.

As shown in FIG. 9A, the powder inhaler further includes an intermediate gear 13, the intermediate gear 13 is disposed in the housing assembly, and the intermediate gear 13 meshes with the drive gear 5. Through engagement and connection between the outer cap 4 and the drive gear 5, linkage that the outer cap 4 drives the drive gear 5 and the functional mechanism is implemented. Specifically, the outer cap 4 rotates around the cylinder 105 of the front housing 1, and the drive hole 402 of the outer cap 4 drives the drive gear 5 to synchronously rotate, thereby implementing linkage of functional mechanisms through the intermediate gear 13.

The functional mechanism includes an air compressing mechanism, a powder delivery mechanism, an inhalation trigger mechanism, a metering mechanism, and the like. Rotation of the outer cap 4 in the opening process drives the air compressing mechanism to implement an air compressing function, and after air compressing is completed, drives the powder delivery mechanism to implement a powder delivery function, to deliver powder, such as drug powder, to a position of the inhalation channel 706 of the powder inhaler, and implement an inhalation trigger function via an inhalation trigger mechanism, thereby facilitating a user in inhaling the drug powder. Further, when the outer cap 4 is closed, the air compressing mechanism, the powder delivery mechanism, and the inhalation trigger mechanism are driven to reset, and the metering mechanism is driven to count.

The following describes the functional mechanisms.

### (1) Air compressing mechanism

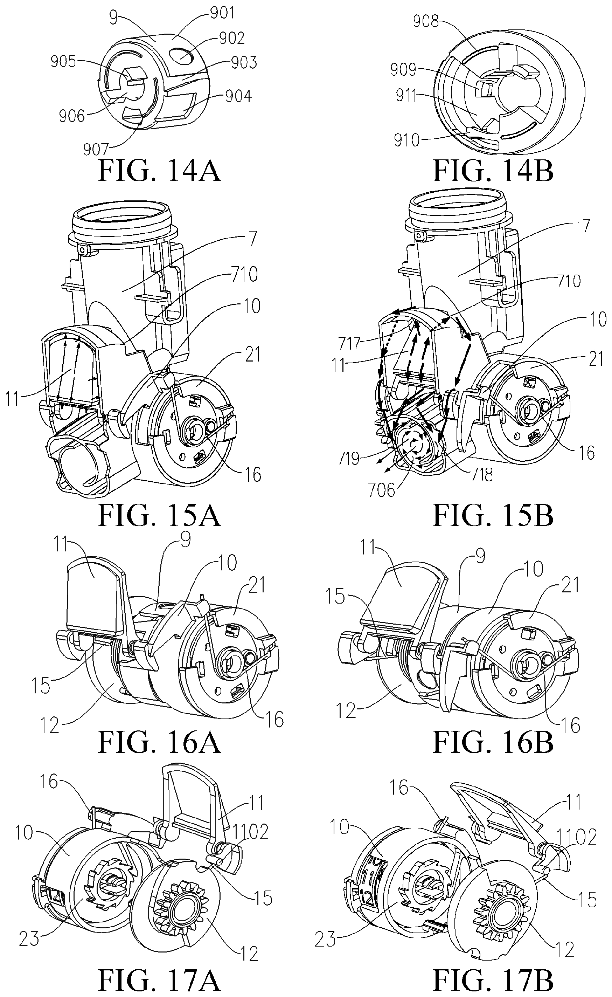

Refer to FIG. 12A to FIG. 14B. FIG. 12A is a schematic diagram of a structure of a powder metering wheel of a powder delivery mechanism of the powder inhaler provided in FIG. 1 being at a third position in a powder container; FIG. 12B is a schematic diagram of a structure of a powder metering wheel of a powder delivery mechanism of the powder inhaler provided in FIG. 1 being at a fourth position in a powder container; FIG. 13A is a schematic diagram of a structure of a powder container that is of the powder inhaler provided in FIG. 1 and that is viewed from an angle; FIG. 13B is a schematic diagram of a structure of the powder container that is provided in FIG. 13A and that is viewed from another angle; FIG. 14A is a schematic diagram of a structure of a powder metering wheel that is of the powder inhaler provided in FIG. 1 and that is viewed from an angle; and FIG. 14B is a schematic diagram of a structure of the powder metering wheel that is provided in FIG. 14A and that is viewed from another angle.

Referring to FIG. 5A to FIG. 14B, specifically, the air compressing mechanism includes an airbag pressing member 17, an air compressing airbag 18, an elastic member 19, and a drive cam 12. To facilitate understanding of a function of the air compressing mechanism, the powder delivery mechanism is introduced herein. The powder delivery mechanism includes a powder container 7 and a powder metering wheel 9. The powder metering wheel 9 has a dose cup 902. The powder container 7 has a storage cavity 715. The storage cavity 715 is configured to store powder. One end of the elastic member 19 acts on the airbag pressing member 17. The airbag pressing member 17 is configured to press the air compressing airbag 18 under driving of the elastic member 19, to enable the air compressing airbag 18 to be compressed to implement an air compressing function. The airbag pressing member 17 is further configured to press the elastic member 19 under an action of the drive cam 12, and drive the air compressing airbag 18 to extend.

Referring to FIG. 12A to FIG. 13B, specifically, a first end of the storage cavity 715 has a powder outlet 713, and a second end of the storage cavity 715 has an air compressing port 714; and the air compressing airbag 18 is disposed at the second end of the storage cavity 715 and is in communication with the air compressing port 714. Specifically, an outer side surface at the second end of the storage cavity 715 has an annular rib 701. One end of the air compressing airbag 18 that is close to the powder container 7 engages with the annular rib 701 of the powder container 7, so that the air compressing airbag 18 is connected to the powder container 7. The airbag pressing member 17 is movably sleeved on an outer side of the air compressing airbag 18 and an outer side of the storage cavity 715. The elastic member 19 may be an elastic structural member such as a spring. The airbag pressing member 17 is configured to press the air compressing airbag 18 under driving of the elastic member 19. The airbag pressing member 17 can move back and forth between a fifth position and a sixth position, so that the air compressing airbag 18 compresses air into the storage cavity 715, to implement an air compressing function, so as to press the powder in the storage cavity 715 from the powder outlet 713 into the dose cup 902 of the powder metering wheel 9.

As shown in FIG. 9A, FIG. 9B, FIG. 11, FIG. 13A, and FIG. 13B, a top wall of the airbag pressing member 17 has a fixing hole 1701. The fixing hole 1701 is configured to connect to a top portion of the air compressing airbag 18. For example, the top portion of the air compressing airbag 18 may pass through the fixing hole 1701, one part of the air compressing airbag 18 is limited outside the top wall of the airbag pressing member 17, and the other part of the air compressing airbag 18 is limited on a side of the top wall of the airbag pressing member 17 that is close to the air compressing port 714, to drive the air compressing airbag 18 to extend and contract through movement of the airbag pressing member 17 and cooperation of the elastic member 19. Driving the air compressing airbag 18 to extend and contract via the airbag pressing member 17 can improve air compressing efficiency of the air compressing airbag 18, and prevent the air compressing airbag 18 from failing to reset due to trapped air, causing abnormal operation of the air compressing airbag 18.

The powder inhaler further includes a drive cam 12, and the drive cam 12 is disposed in the housing assembly. Specifically, referring to FIG. 9A, the powder inhaler further includes a gear bracket 14. The gear bracket 14 is disposed in the housing assembly. The drive cam 12 is assembled on the gear bracket 14. One end of the gear bracket 14 is assembled and connected to the powder metering wheel 9, and the other end of the gear bracket 14 is assembled and connected to the drive cam 12. The outer cap 4 drives the drive gear 5 to rotate, the drive gear 5 meshes with the intermediate gear 13, and the intermediate gear 13 meshes with the drive cam 12, so that the drive cam 12 and the drive gear 5 rotate in a same direction. It can be understood that, the drive gear 5 and/or the intermediate gear 13 may also be omitted in this application, provided that rotation of the outer cap 4 can drive the cam 12 to rotate. The drive cam 12 and the elastic member 19 are configured to drive the airbag pressing member 17 to move back and forth between the fifth position and the sixth position, thereby driving the air compressing airbag 18 to extend and contract, so as to implement an air compressing function and a resetting function. Specifically, the elastic member 19 is disposed on a side of the top wall of the airbag pressing member 17, one end of the elastic member 19 abuts against the top wall of the airbag pressing member 17, and the other end of the elastic member 19 abuts against a top wall of the upper housing 3, to drive the airbag pressing member 17 to move; the drive cam 12 rotates to provide clearance for the airbag pressing member 17, and the elastic member 19 drives the airbag pressing member 17 to move from the fifth position to the sixth position; and the drive cam 12 presses the airbag pressing member 17, and the airbag pressing member 17 resets and moves from the sixth position to the fifth position and presses the elastic member 19.

Referring to FIG. 10A to FIG. 11, one end of a side wall of the airbag pressing member 17 that is away from the top wall of the airbag pressing member 17 has a protruding rod 1704, one end of the protruding rod 1704 that is away from the top wall of the airbag pressing member 17 has an arc surface 1703, the drive cam 12 has a cam curved surface 1202, and the arc surface 1703 is used to cooperate with the cam curved surface 1202 of the drive cam 12, so that the airbag pressing member 17 moves back and forth between the fifth position and the sixth position. Specifically, when the airbag pressing member 17 is at the fifth position, the arc surface 1703 of the protruding rod 1704 of the airbag pressing member 17 abuts against a side surface of the drive cam 12, and the arc surface 1703 of the airbag pressing member 17 is limited in an arc-shaped groove 1201 of the drive cam 12. In this case, the top wall of the airbag pressing member 17 compresses the elastic member 19 into a compressed state, and the top wall of the airbag pressing member 17 stretches the air compressing airbag 18 into an extended state. In the cap-opening process of the outer cap 4, the drive cam 12 is driven to rotate, the elastic member 19 continuously extends, and an elastic force of the elastic member 19 drives the airbag pressing member 17 to move vertically downward, that is, the elastic member 19 drives the airbag pressing member 17 to move from the fifth position to the sixth position, the arc surface 1703 of the airbag pressing member 17 moves along the cam curved surface 1202 of the drive cam 12, and the airbag pressing member 17 moves downward to enable the air compressing airbag 18 to be compressed and press air into the storage cavity 715.

In an implementation, a ratio of a teeth quantity between the drive gear 5 and the drive cam 12 is 18:15, that is, when the outer cap 4 is opened by 150°, the drive gear 5 synchronously rotates by 150°. In this case, a rotation angle of the drive cam 12 is 180°. It can be understood that, setting the ratio of the teeth quantity between the drive gear 5 and the drive cam 12 to 18:15 can reduce a volume of the drive gear 5, thereby saving space. In another implementation, the powder inhaler may not be provided with the drive gear 5 and the intermediate gear 13; instead, the outer cap 4 and the drive cam 12 are directly linked, so that rotation of the outer cap 4 drives the drive cam 12 to rotate, thereby implementing linkage of the functional mechanisms.

Specifically, as shown in FIG. 9A to FIG. 11, the drive cam 12 includes a body portion 1200 and a gear 1203, the gear 1203 is coaxially connected to the body portion 1200, and is configured to drive the body portion 1200 to rotate, and the gear 1203 meshes with the intermediate gear 13. A surface of the body portion 1200 has a guide groove 1209, a side surface of the guide groove 1209 is a cam curved surface 1202, and the cam curved surface 1202 cooperates with the arc surface 1703 of the airbag pressing member 17, so that the airbag pressing member 17 moves back and forth between the fifth position and the sixth position. Specifically, the drive cam 12 has a central hole 1204, and the central hole 1204 is defined as a first central hole. The gear 1203 is disposed on a surface of the body portion 1200 and is disposed surrounding the central hole 1204. The central hole 1204 runs through the body portion 1200 and the gear 1203. The central hole 1204 of the drive cam 12 is sleeved on the gear bracket 14 of the powder inhaler. The guide groove 1209 is provided on a surface of the body portion 1200 that faces the gear 1203, and the guide groove 1209 is spaced apart from the gear 1203. The airbag pressing member 17 moves up and down along a longitudinal direction between the fifth position and the sixth position. In this application, the cam curved surface 1202 of the drive cam 12 directly abuts against the protruding rod 1704 of the airbag pressing member 17, to simplify a structure of the air compressing mechanism and improve operational stability of the air compressing mechanism. In an implementation, the air compressing mechanism includes only four independent components, namely, an airbag pressing member 17, an air compressing airbag 18, an elastic member 19, and a drive cam 12, resulting in a simple structure of the air compressing mechanism.

In some implementations, one end of the protruding rod 1704 of the airbag pressing member 17 that is away from the top wall has a tip, an end surface of the tip is an arc surface 1703, and an outer peripheral side surface of the body portion 1200 of the drive cam 12 has an arc-shaped groove 1201. When the airbag pressing member 17 is configured to be at the fifth position, the tip of the protruding rod 1704 is embedded into the arc-shaped groove 1201 of the body portion 1200, to implement initial positioning and preliminary limitation on the airbag pressing member 17.

In a specific implementation, the arc-shaped groove 1201 is a circular-arc-shaped groove, a bottom surface of the arc-shaped groove 1201 is circular-arc-shaped, and the arc surface 1703 of the protruding rod 1704 is a circular arc surface. It can be understood that, the arc-shaped groove 1201 is provided on the cam curved surface 1202 of the drive cam 12, and the arc surface 1703 of the protruding rod 1704 is embedded in the arc-shaped groove 1201 to perform initial positioning and preliminary limitation on the airbag pressing member 17, so that movement of the air compressing mechanism needs to overcome resistance to upward movement of the arc surface 1703 of the airbag pressing member 17 disengaging from the arc-shaped groove 1201, to effectively prevent false trigger of the air compressing mechanism.

In a specific implementation, a bottom surface of the arc-shaped groove 1201 is circular-arc-shaped, and the arc surface 1703 of the protruding rod 1704 is a circular arc surface. In a process in which the arc surface 1703 of the protruding rod 1704 moves from one side of the bottom surface of the arc-shaped groove 1201 to the other side of the bottom surface of the arc-shaped groove 1201, the protruding rod 1704 remains stationary, and rotation of the outer cap 4 constitutes an idle stroke.

Further, referring to FIG. 9A to FIG. 13B, a side wall of the storage cavity 715 of the powder container 7 has a vent hole 709, and the side wall of the airbag pressing member 17 has a pressure relief hole 1702. As shown in FIG. 9C and FIG. 9E, when the airbag pressing member 17 is configured to be at the fifth position, the side wall of the airbag pressing member 17 seals the vent hole 709, and the vent hole 709 is not in communication with the pressure relief hole 1702. As shown in FIG. 9D and FIG. 9F, when the airbag pressing member 17 is configured to be at the sixth position, the pressure relief hole 1702 is in communication with the vent hole 709. In a process in which the airbag pressing member 17 moves from the fifth position to the sixth position, air is first pressed into the storage cavity 715 via the air compressing airbag 18, so as to fill and compact the powder in the storage cavity 715 into the dose cup 902 of the powder metering wheel 9 via the powder outlet 713, thereby improving consistency of powder filling. After the pressure relief hole 1702 is in communication with the vent hole 709, pressure in the storage cavity 715 is relieved, to release the pressure in the storage cavity 715 of the powder container 7 to atmospheric pressure, so as to avoid excessive pressure in the storage cavity 715 due to unrelieved pressure in the storage cavity 715 in a subsequent powder delivery process, causing the powder to leak through a gap during rotation of the powder metering wheel 9 of the powder delivery mechanism, so as to reduce powder waste. That is, in a process in which the airbag pressing member 17 moves from the fifth position to the sixth position, an air compressing process is performed first, followed by a pressure relief process. The pressure relief process is performed in a later stage of a downward pressing stroke of the airbag pressing member 17. Because the pressure relief process of the storage cavity 715 in this application is completed before the powder metering wheel 9 rotates from the third position to the fourth position, less powder leaks from the powder outlet 713 under air pressure after the powder metering wheel 9 starts to rotate from the third position to the fourth position.

Specifically, referring to FIG. 9C and FIG. 9D, in some implementations, a second end of the storage cavity 715 is provided with a filter membrane 25, the filter membrane 25 is located at the air compressing port 714 and is spaced apart from a port of the air compressing port 714, to isolate space of the air compressing airbag 18 from internal space of the storage cavity 715. The filter membrane 25 may be a waterproof breathable membrane, and can be used to filter impurities from powder such as drug powder and filter moisture and the like, to prevent the powder in the storage cavity 715 from absorbing moisture. The vent hole 709 is provided on the side wall of the storage cavity 715. One end of the vent hole 709 is in communication with space between the filter membrane 25 and the air compressing airbag 18. That is, the vent hole 709 is not in direct communication with the inside of the storage cavity 715, and is relatively independent of the inside of the storage cavity 715. After the vent hole 709 is in communication with the pressure relief hole 1702, air in the space between the filter membrane 25 and the air compressing airbag 18 can be discharged via the vent hole 709 and the pressure relief hole 1702 in sequence, to relieve pressure in the space between the filter membrane 25 and the air compressing airbag 18. In addition, because the filter membrane 25 is a breathable membrane, air in the storage cavity 715 can also pass through the filter membrane 25, enter the space between the filter membrane 25 and the air compressing airbag 18, and then be discharged via the vent hole 709, to relieve the pressure in the storage cavity 715. This prevents the vent hole 709 from being in direct communication with the inside of the storage cavity 715, which may cause the air in the storage cavity 715 to directly leak via the vent hole 709 in the pressure relief process, resulting in the drug powder in the storage cavity 715 flying or leaking. In addition, during extension of the air compressing airbag 18, that is, during inhalation of the air compressing airbag 18, external air enters the space between the filter membrane 25 and the air compressing airbag 18 via the vent hole 709, and then enters the storage cavity 715 via the filter membrane 25, to avoid a case in which the vent hole 709 is in direct communication with the inside of the storage cavity 715, and the external air directly enters the storage cavity 715 from the vent hole 709 during inhalation of the air compressing airbag 18, bringing external water molecules into the storage cavity 715, and consequently causing waste due to flying or dampening drug powder in the storage cavity 715. In addition, the filter membrane 25 can also prevent the drug powder from entering the air compressing airbag 18 from the storage cavity 715, to avoid a problem of drug powder waste.

Referring to FIG. 13A, the side wall of the storage cavity 715 of the powder container 7 is further provided with an accommodating cavity 702. The accommodating cavity 702 is configured to store a desiccant. The storage cavity 715 and the accommodating cavity 702 have a common side wall. The common side wall may be made of a water-permeable material, so that the desiccant in the accommodating cavity 702 can absorb moisture in the storage cavity 715, to prevent the powder in the storage cavity 715 from being dampened.

In a preferred implementation, in a process in which the airbag pressing member 17 moves from the fifth position to the sixth position, a downward pressing stroke of the airbag pressing member 17 ranges from 2 mm to 6 mm. For example, in a specific implementation, the downward pressing stroke of the airbag pressing member 17 is 3.5 mm. The first 3 mm of the downward pressing stroke is an air compressing stroke, and the last 0.5 mm of the downward pressing stroke is a pressure relief stroke. When the downward pressing stroke of the airbag pressing member 17 reaches 3 mm, the pressure relief hole 1702 and the vent hole 709 are at a critical point of communication. When the downward pressing stroke of the airbag pressing member 17 ranges from 3 mm to 3.5 mm, the pressure relief hole 1702 is in communication with the vent hole 709 to implement pressure relief. An angle of the outer cap 4 at the first position is defined as 0 degrees, and an angle of the outer cap 4 at the second position is 150 degrees. When the downward pressing stroke of the airbag pressing member 17 of the air compressing mechanism is 3.5 mm, that is, when the airbag pressing member 17 is at the sixth position, an angle of the outer cap 4 is 62.5 degrees, an angle at which the outer cap 4 drives the drive gear 5 to rotate is 62.5 degrees, and a corresponding rotation angle of the drive cam 12 is 75 degrees.

Further, preferably, in a cap-opening process, when a rotation angle of the outer cap 4 is 55 degrees, the downward pressing stroke of the airbag pressing member 17 reaches 3 mm, and the air compressing mechanism completes a process of compressing and discharging the powder in the powder container 7, namely the air compressing process. When a rotation angle of the outer cap 4 is 62.5 degrees, the pressure relief process is completed, that is, the compressed air in the powder container 7 is released to atmospheric pressure, to avoid powder leakage when the powder metering wheel 9 rotates. It can be understood that, selection of the foregoing angles is merely by way of example, and another angle range may also be selected.

Referring to FIG. 10A to FIG. 10C, in some implementations, the cam curved surface 1202 includes a first curved surface segment 1211 and a second curved surface segment 1212 that are connected to each other, and the second curved surface segment 1212 is located at one end of the first curved surface segment 1211 that is away from the arc-shaped groove 1201. The first curved surface segment 1211 is a non-circular arc surface, and the second curved surface segment 1212 is a circular arc surface and is disposed concentric to the peripheral side surface of the body portion 1200.

It can be understood that, the first curved surface segment 1211 is set as a non-circular arc surface, so that in a cap-closing process of the outer cap 4 of the powder inhaler, the drive cam 12 rotates in a reverse direction, and rotation of the first curved surface segment 1211 drives the airbag pressing member 17 to reset from the sixth position to the fifth position. Specifically, in a process in which the airbag pressing member 17 resets from the sixth position to the fifth position, the arc surface 1703 of the protruding rod 1704 of the airbag pressing member 17 abuts against the first curved surface segment 1211, the first curved surface segment 1211 is a non-circular arc surface, and the protruding rod 1704 of the airbag pressing member 17 is continuously pushed to move upward due to reverse rotation of the first curved surface segment 1211, to implement resetting of the airbag pressing member 17.

Preferably, the first curved surface segment 1211 includes a first arc surface segment 1214, a plane segment 1215, and a second arc surface segment 1216 that are connected to each other. The plane segment 1215 is located between the first arc surface segment 1214 and the second arc surface segment 1216, and the plane segment 1215 is located at one end of the first arc surface segment 1214 that is away from the arc-shaped groove 1201. In the cap-opening process of the outer cap 4, in a process in which the airbag pressing member 17 moves from the fifth position to the sixth position, resistance exerted by the side wall of the arc-shaped groove 1201 that is close to the first arc surface segment 1214 on the arc surface 1703 of the protruding rod 1704 first needs to be overcome, so that the tip of the protruding rod 1704 disengages from the arc-shaped groove 1201. This process is a cap-opening idle stroke, which requires a relatively large torque, to prevent accidental cap opening.

In an implementation, an angle of the outer cap 4 at the first position is defined as 0 degrees, an angle of the outer cap 4 at the second position is 150 degrees, and a process in which the outer cap 4 rotates from 0 degrees to 12 degrees is a cap-opening idle stroke. A process in which the outer cap 4 rotates from 0 degrees to 8 degrees is a first cap-opening sub-idle stroke, and a process in which the outer cap 4 rotates from 8 degrees to 12 degrees is a second cap-opening sub-idle stroke. In the first cap-opening sub-idle stroke, rotation of the outer cap 4 enables the arc surface 1703 at the tip of the protruding rod 1704 to move from abutting against one end of the arc-shaped groove 1201 that is away from the first arc surface segment 1214 to abutting against one end of the arc-shaped groove 1201 that is close to the first arc surface segment 1214, that is, the arc surface 1703 at the tip of the protruding rod 1704 slides across the bottom surface of the arc-shaped groove 1201. Preferably, a torque in this process is 0.05 N•m, which can effectively prevent accidental cap opening due to non-human factors. In the second cap-opening sub-idle stroke, rotation of the outer cap 4 enables the arc surface 1703 at the tip of the protruding rod 1704 to move from abutting against one end of the arc-shaped groove 1201 that is close to the first arc surface segment 1214 to abutting against the first arc surface segment 1214, that is, the arc surface 1703 at the tip of the protruding rod 1704 needs to disengage from the arc-shaped groove 1201, which requires a larger torque than that in the first cap-opening sub-idle stroke. Preferably, a torque in the second cap-opening sub-idle stroke is 0.15 N•m. Setting greater cap-opening resistance can more effectively prevent accidental cap opening.

In the cap-opening process of the outer cap 4, when the outer cap 4 rotates to 12 degrees, the arc surface 1703 at the tip of the protruding rod 1704 of the airbag pressing member 17 disengages from the arc-shaped groove 1201 and abuts against the first arc surface segment 1214, so that a cap-opening idle stroke of the outer cap 4 is completed. Next, in a process in which the outer cap 4 rotates from 12 degrees to 62.5 degrees, the air compressing mechanism performs an air compressing process and a pressure relief process, and in this case, the outer cap 4 performs a first cap-opening sub-load stroke.

When the outer cap 4 rotates to 12 degrees, the arc surface 1703 at the tip of the protruding rod 1704 has disengaged from the arc-shaped groove 1201, and the outer cap 4 is instantaneously opened and rotates from 12 degrees to 55 degrees, to drive the drive cam 12 to instantaneously rotate to 66 degrees. In this case, the protruding rod 1704 of the airbag pressing member 17 also instantaneously moves downward, and the arc surface 1703 at the tip of the protruding rod 1704 instantaneously moves to abut against one end of the plane segment 1215 that is close to the second arc surface segment 1216. A downward pressing stroke of the protruding rod 1704 of the airbag pressing member 17 reaches 3 mm, so that the air compressing mechanism implements an instantaneous air compressing function. When the outer cap 4 rotates to 55 degrees, the pressure relief hole 1702 and the vent hole 709 are at a critical point of communication. In a process in which the outer cap 4 rotates from 55 degrees to 62.5 degrees, the drive cam 12 rotates from 66 degrees to 75 degrees. The tip of the protruding rod 1704 of the airbag pressing member 17 moves from abutting against one end of the second arc surface segment 1216 that is close to the plane segment 1215 to abutting against one end of the second arc surface segment 1216 that is away from the plane segment 1215, that is, the tip of the protruding rod 1704 slides across the second arc surface segment 1216. In this process, a downward pressing stroke of the protruding rod 1704 of the airbag pressing member 17 ranges from 3 mm and 3.5 mm, and the pressure relief hole 1702 is in communication with the vent hole 709 to implement pressure relief. A torque of the outer cap 4 in the first cap-opening sub-load stroke is constant. Preferably, a torque of the outer cap 4 in the first cap-opening sub-load stroke is 0 N•m, that is, the torque is 0 N•m in a process in which the outer cap 4 rotates from 12 degrees to 62.5 degrees, which is conducive to instantaneous opening of the outer cap 4, so the air compressing mechanism rapidly and instantaneously compresses air, thereby improving an air compressing effect.

When the outer cap 4 rotates to 62.5 degrees, the arc surface 1703 at the tip of the protruding rod 1704 of the airbag pressing member 17 is located at a critical point between the first curved surface segment 1211 and the second curved surface segment 1212. In a process in which the outer cap 4 continues to rotate from 62.5 degrees to 150 degrees, the drive cam 12 rotates from 75 degrees to 180 degrees, and the arc surface 1703 of the protruding rod 1704 of the airbag pressing member 17 moves from abutting against one end of the second curved surface segment 1212 that is close to the first curved surface segment 1211 to abutting against one end of the second curved surface segment 1212 that is away from the first curved surface segment 1211. Because the second curved surface segment 1212 is a circular arc surface, and the second curved surface segment 1212 is disposed concentric to the outer peripheral side surface of the body portion 1200 and a pitch circle of the gear 1203, in a process in which the outer cap 4 rotates from 62.5 degrees to 150 degrees, the protruding rod 1704 of the airbag pressing member 17 remains at the sixth position, that is, at a position of 3.5 mm, and the protruding rod 1704 of the airbag pressing member 17 does not move.

Referring to FIG. 1 to FIG. 11, the second curved surface segment 1212 of the drive cam 12 is provided with a stop groove 1213 at one end away from the first curved surface segment 1211. After the outer cap 4 is fully opened, the tip of the protruding rod 1704 of the airbag pressing member 17 is located in the stop groove 1213, and through action of gravity of the airbag pressing member 17 on the drive cam 12, the tip of the protruding rod 1704 of the airbag pressing member 17 limits the drive cam 12, to prevent, when the outer cap is fully opened, the drive cam 12 from rotating in a reverse direction under the action of a resetting torsion spring 15 (as shown in FIG. 17A) after a user releases the outer cap, which may cause a problem that the outer cap automatically is closed after the outer cap is fully opened. In a specific implementation, the stop groove 1213 is a circular-arc-shaped groove, and a bottom surface of the stop groove 1213 is circular-arc-shaped, to facilitate better cooperation between the tip of the protruding rod 1704 and the stop groove 1213.

### (2) Powder delivery mechanism

Referring to FIG. 12A to FIG. 14B, the powder delivery mechanism includes a powder container 7 and a powder metering wheel 9, and the powder metering wheel 9 of the powder delivery mechanism is rotatably connected to the powder container 7. Specifically, the powder container 7 has a storage cavity 715, an inhalation channel 706, and a first cylindrical groove 716. The powder metering wheel 9 is mounted in the first cylindrical groove 716, and the powder metering wheel 9 can rotate back and forth between a third position and a fourth position. At the third position, a dose cup 902 of the powder metering wheel 9 is at a powder filling position; and at the fourth position, the dose cup 902 of the powder metering wheel 9 is at a powder inhalation position, namely, a position corresponding to the inhalation channel 706. It should be noted that, the powder metering wheel 9 of this application rotates back and forth between the third position and the fourth position along a minor arc, that is, a direction in which the powder metering wheel 9 rotates from the third position to the fourth position is opposite to a direction in which the powder metering wheel 9 rotates from the fourth position to the third position, and the powder metering wheel 9 does not perform full-circle rotation movement along an inner periphery of the first cylindrical groove 716 to rotate back and forth between the third position and the fourth position. The powder metering wheel 9 rotates back and forth along the minor arc between the third position and the fourth position, so that a movement path of the powder metering wheel 9 can be minimized, and the powder metering wheel 9 moves along an optimal path, to more effectively prevent waste and loss of the drug powder in the dose cup 902 during movement of the powder metering wheel 9.

The powder metering wheel 9 includes the dose cup 902. When the powder metering wheel 9 is configured to be at the third position, the dose cup 902 is disposed corresponding to a powder outlet 713 of the storage cavity 715, to receive powder in the powder container 7. When the powder metering wheel 9 is configured to be at the fourth position, the dose cup 902 is disposed corresponding to an inlet 704 of the inhalation channel 706. Specifically, in a process in which an airbag pressing member 17 moves from a fifth position to a sixth position, the powder metering wheel 9 is at the third position, and the airbag pressing member 17, driven by an elastic member 19, acts on an air compressing airbag 18, and fills and presses the powder in the storage cavity 715 into the dose cup 902 of the powder metering wheel 9.

Referring to FIG. 13A, FIG. 14A, and FIG. 14B, specifically, the powder metering wheel 9 is mounted in the first cylindrical groove 716. The powder metering wheel 9 has an outer arc surface 901, and the outer arc surface 901 of the powder metering wheel 9 is closely attached to an inner arc surface 705 of the first cylindrical groove 716 of the powder container 7. The outer arc surface 901 of the powder metering wheel 9 is a circular arc annular surface covering a back-and-forth stroke, and a radian corresponding to a circular arc of the outer arc surface 901 is greater than or equal to 140 degrees and less than or equal to 170 degrees. Preferably, the radian corresponding to the circular arc of the outer arc surface 901 is approximately 150°.

Referring to FIG. 13A and FIG. 14B, a cylinder 703 is disposed in the first cylindrical groove 716 of the powder container 7, and the cylinder 703 is defined as a second cylinder. The powder metering wheel 9 has a central hole 906, and the central hole 906 is defined as a second central hole. A first end of the powder metering wheel 9 has a tightening elastic arm 909, the tightening elastic arm 909 is defined as a first tightening elastic arm, and the tightening elastic arm 909 is disposed on an outer periphery of the central hole 906. The tightening elastic arm 909 of the powder metering wheel 9 is disposed in cooperation with the cylinder 703 in the first cylindrical groove 716 of the powder container 7. Specifically, the cylinder 703 in the first cylindrical groove 716 is assembled into the central hole 906, and the tightening elastic arm 909 abuts against the cylinder 703 in the first cylindrical groove 716. An inner diameter formed by the tightening elastic arm 909 is less than an outer diameter of the cylinder 703, so that during assembly, the tightening elastic arm 909 elastically deforms. The elastic deformation of the tightening elastic arm 909 of the powder metering wheel 9 provides a pressing force for attachment between the inner arc surface 705 of the first cylindrical groove 716 and the outer arc surface 901 of the powder metering wheel 9, thereby improving sealing reliability, enabling the powder metering wheel 9 to be assembled more tightly in the first cylindrical groove 716 of the powder container 7, and facilitating a secure assembly connection between the powder metering wheel 9 and the powder container 7.

The dose cup 902 is disposed on an outer side surface of the powder metering wheel 9. Specifically, the dose cup 902 is disposed on the outer arc surface 901 of the powder metering wheel 9, and the dose cup 902 is configured to accommodate powder. In a preferred implementation, the outer arc surface 901 of the powder metering wheel 9 is provided with only one dose cup 902, so that the powder metering wheel 9 rotates back and forth between the third position and the fourth position, to drive the dose cup 902 to rotate back and forth between a position of the inlet 704 of the inhalation channel 706 and a position of the powder outlet 713, so as to implement filling and delivery of the powder, thereby facilitating inhalation by a user.

It can be understood that, an amount of powder accommodated in the dose cup 902 is fixed, and an amount of powder entering the inhalation channel 706 during user inhalation is fixed, so that a case in which a plurality of dose cups 902 are circumferentially spaced apart on an outer side surface of a cylindrical metering member, and when the metering member rotates, a plurality of doses are allowed to be continuously dispensed into the inhalation channel 706, leading to a possibility of an overdose of powder, such as drug powder is avoided. Alternatively, when a series of dose grooves or one dose groove is provided on a surface of a plate-shaped metering member, and the powder is delivered in a translational manner, a position state used for inhalation is inconsistent with a cap-opening position state of the outer cap 4, causing the user to inhale a plurality of doses of powder during single inhalation, and further causing a problem of an overdose of powder inhaled by the user. That is, only one dose cup 902 is disposed on the outer arc surface 901 of the powder metering wheel 9, so that an amount of powder inhaled by the user during single inhalation at a position of an outlet 102 of a suction nozzle 101 is fixed, eliminating a possibility of inhaling a plurality of doses of powder, thereby enabling precise control over an amount of inhaled powder and simplifying a structure.

In another implementation, the dose cup 902 disposed on the outer arc surface 901 of the powder metering wheel 9 may also include a plurality of sub-dose cups, that is, at one position, the dose cup 902 may be separated to form a plurality of mutually spaced sub-dose cups, for example, one or more separating places may be disposed in the dose cup 902, to separate the dose cup 902 into a plurality of sub-dose cups. Alternatively, in another implementation, a plurality of dose cups 902 may also be spaced apart on the outer arc surface 901 of the powder metering wheel 9, provided that a powder accommodating amount in the dose cup 902 at a fixed position is fixed, to facilitate precise control over an amount of powder inhaled by the user.

Referring to FIG. 14A, in an implementation, a first powder scraping groove 903 is further provided on the outer arc surface 901 of the powder metering wheel 9, and the first powder scraping groove 903 is a notch provided on the outer arc surface 901. Specifically, the first powder scraping groove 903 is circumferentially and obliquely provided relative to the powder metering wheel 9. The first powder scraping groove 903 is configured to scrape off and discharge fine drug powder adhering to the inner arc surface 705 of the powder container 7, so as to prevent the fine drug powder on the inner arc surface 705 of the powder container 7 from obstructing movement of the powder metering wheel 9, thereby improving movement smoothness of the powder metering wheel 9 in the first cylindrical groove 716 of the powder container 7. Preferably, a depth of the first powder scraping groove 903 ranges from 0.2 mm to 0.4 mm.

In an implementation, a second powder scraping groove 904 is further provided on the outer arc surface 901 of the powder metering wheel 9, and a depth of the second powder scraping groove 904 is greater than the depth of the first powder scraping groove 903. Preferably, the depth of the second powder scraping groove 904 ranges from 0.7 mm to 0.9 mm. The second powder scraping groove 904 is configured to scrape off and discharge large-particle drug powder adhering to the inner arc surface 705 of the powder container 7, so as to further improve the movement smoothness of the powder metering wheel 9 in the first cylindrical groove 716 of the powder container 7. In some implementations, a width of the second powder scraping groove 904 may also be greater than a width of the first powder scraping groove 903. Along a direction in which the powder metering wheel 9 rotates from the third position to the fourth position, the second powder scraping groove 904 is provided on a side of the first powder scraping groove 903 that is away from the dose cup 902, so that in a process in which the powder metering wheel 9 rotates from the third position to the fourth position, that is, in a drug delivery stroke of the powder metering wheel 9, the second powder scraping groove 904 first scrapes off and discharges the large-particle drug powder adhered to the inner arc surface 705 of the powder container 7, to reduce movement resistance caused by the large-particle drug powder to the powder metering wheel 9, and then the first powder scraping groove 903 scrapes off and discharges remaining fine drug powder adhered to the inner arc surface 705 of the powder container 7, to better ensure the movement smoothness of the powder metering wheel 9 in the first cylindrical groove 716 of the powder container 7. The first powder scraping groove 903 and the second powder scraping groove 904 have different sizes. In the drug delivery stroke and a resetting stroke of the powder metering wheel 9, that is, in a process in which the powder metering wheel 9 reversely rotates from the fourth position to the third position, the first powder scraping groove 903 and the second powder scraping groove 904 can clean the drug powder on the inner arc surface 705 of the powder container 7 a plurality of times and in a plurality of gradients, thereby further improving accuracy of a dose of drug powder delivery.

In an implementation, an end surface at a first end of the powder metering wheel 9 has a first rib 908. The first rib 908 is disposed to avoid friction between a plane and adjacent components, and smoothness of rotating back and forth by the powder metering wheel 9 is improved through contact friction of the rib.

In an implementation, an end surface at a second end of the powder metering wheel 9 has a second rib 907. The second rib 907 is disposed to avoid friction between a plane and adjacent components, and smoothness of rotating back and forth by the powder metering wheel 9 is improved through contact friction of the rib.

In an implementation, an end surface at a first end of the powder metering wheel 9 has a first rib 908, and an end surface at a second end of the powder metering wheel 9 has a second rib 907.

In an implementation, referring to FIG. 14B, the first end of the powder metering wheel 9 further has a groove 911 and a drive elastic arm 910. The groove 911 is provided around an outer periphery of the central hole 906, one end of the drive elastic arm 910 is connected to a side wall of the groove 911, the other end of the drive elastic arm 910 is a free end, and the drive elastic arm 910 is spaced apart from the tightening elastic arm 909. One end of a wedge-shaped post 1005 of a dose protection plate 10 extends into the groove 911, and the drive elastic arm 910 is configured to abut against the wedge-shaped post 1005 of the dose protection plate 10 to drive the dose protection plate 10 to rotate and reset. Providing the groove 911 can also reduce a weight of the powder metering wheel 9, making it easier to drive the powder metering wheel 9 to rotate. A specific manner and process in which the drive elastic arm 910 drives the dose protection plate 10 to rotate and reset are described in detail subsequently in a process in which the outer cap 4 is closed to trigger reset of a functional mechanism. Details are not described in further detail herein.

Referring to FIG. 10B and FIG. 14A, a surface of the body portion 1200 of the drive cam 12 that faces away from the gear 1203 is further provided with an annular boss 1207. The annular boss 1207 is coaxially disposed with the gear 1203, and is configured to drive the powder metering wheel 9 to rotate. Specifically, the second end of the powder metering wheel 9 has a boss 905, the boss 905 is defined as a first boss, and the boss 905 of the powder metering wheel 9 is configured to cooperate with the annular boss 1207 of the drive cam 12 to implement rotation through driving. Specifically, two annular bosses 1207 are disposed centrally symmetrically about a center of the central hole 1204, and two bosses 905 are disposed centrally symmetrically about a center of the central hole 906.

In a preferred implementation, in a cap-opening process, an angle of the outer cap 4 at the second position is 150 degrees. In a process in which the outer cap 4 rotates from the first position (namely, 0 degrees) to the second position, where in a process in which the outer cap 4 rotates from 0 degrees to 62.5 degrees (that is, the outer cap 4 rotates by 62.5 degrees), the outer cap 4 drives the drive gear 5 to rotate, thereby driving the drive cam 12 to rotate from 0 degrees to 75 degrees (that is, the drive cam 12 rotates by 75 degrees). In this process, the powder metering wheel 9 is in a static state, the boss 905 of the powder metering wheel 9 does not contact the annular boss 1207 of the drive cam 12, and the powder metering wheel 9 does not rotate at the third position. In a process in which the outer cap 4 rotates from 62.5 degrees to 150 degrees (that is, the outer cap 4 rotates by 87.5 degrees), the outer cap 4 drives the drive gear 5 to rotate, thereby driving the drive cam 12 to rotate from 75 degrees to 180 degrees (that is, the drive cam 12 rotates by 105 degrees). In this process, the boss 905 of the powder metering wheel 9 is disposed in contact with the annular boss 1207 of the drive cam 12. The annular boss 1207 of the drive cam 12 cooperates with the boss 905 of the powder metering wheel 9, to drive the powder metering wheel 9 to rotate from the third position to the fourth position. The powder metering wheel 9 rotates by 105 degrees in this process, that is, when the dose cup 902 of the powder metering wheel 9 rotates from the position of the powder outlet 713 to the position of the inlet 704 of the inhalation channel 706, a rotation angle of the powder metering wheel 9 is 105 degrees.

In a process in which the outer cap 4 rotates from 62.5 degrees to 150 degrees, the drive cam 12 rotates from 75 degrees to 180 degrees, and drives the powder metering wheel 9 to rotate from the third position to the fourth position, that is, the dose cup 902 of the powder metering wheel 9 rotates from the position of the powder outlet 713 of the powder container 7 to the position of the inlet 704 of the inhalation channel 706. In this process, the powder delivery mechanism implements a powder delivery process. In the cap-opening process of the outer cap 4, a second cap-opening sub-load stroke is performed in this rotation range, requiring a relatively large torque. A torque of the outer cap 4 is constant in the second cap-opening sub-load stroke. Preferably, the torque of the outer cap 4 in the second cap-opening sub-load stroke is 0.1 N•m, to ensure that the outer cap 4 is opened smoothly at a constant speed without any abrupt torque change until cap opening is completed, thereby ensuring powder delivery performance and preventing powder leakage, flying, or waste.

The outer cap 4 is linked to and cooperates with the powder metering wheel 9 and the air compressing mechanism respectively. When the outer cap 4 is configured to be at the first position, the outer cap 4 limits the airbag pressing member 17 at the fifth position. In a process in which the outer cap 4 rotates from the first position to the second position, limitation on the airbag pressing member 17 is first released, so that the elastic member 19 drives the airbag pressing member 17 to move from the fifth position to the sixth position, and then drives the powder metering wheel 9 to rotate from the third position to the fourth position. In a process in which the outer cap 4 reversely rotates and resets from the second position to the first position, the outer cap 4 drives the powder metering wheel 9 to reversely rotate and reset from the fourth position to the third position, and drives the airbag pressing member 17 to move in a reverse direction and reset from the sixth position to the fifth position.

After the powder delivery mechanism completes the powder delivery process, the outer cap 4 rotates to the second position, the dose cup 902 of the powder metering wheel 9 of the powder delivery mechanism is located at a position corresponding to the inlet 704 of the inhalation channel 706, and the outer cap 4 is fully opened to expose an outlet 102 of the suction nozzle 101, so that the user can inhale at a position of the outlet 102 of the suction nozzle 101, thereby facilitating triggering an action of an inhalation trigger mechanism to implement an inhalation trigger function.

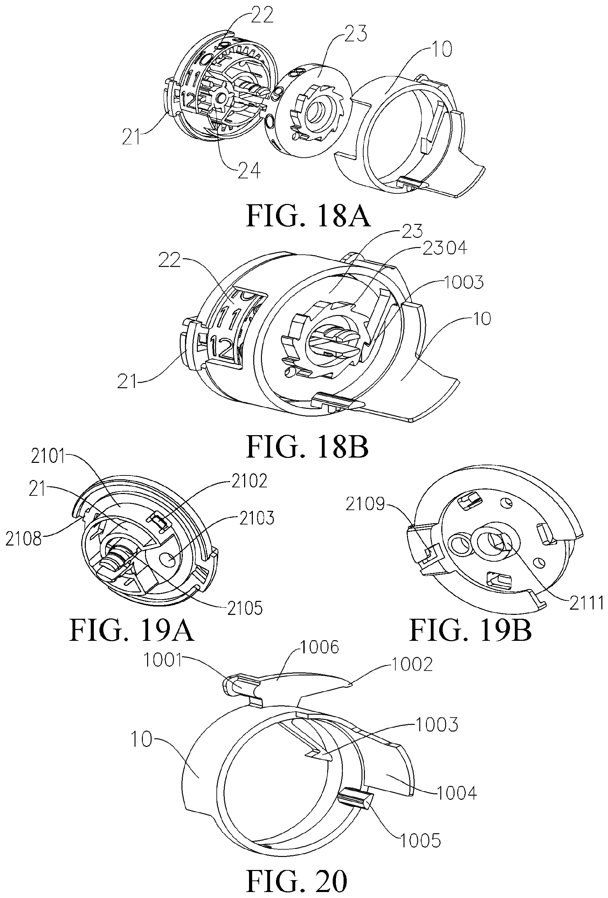

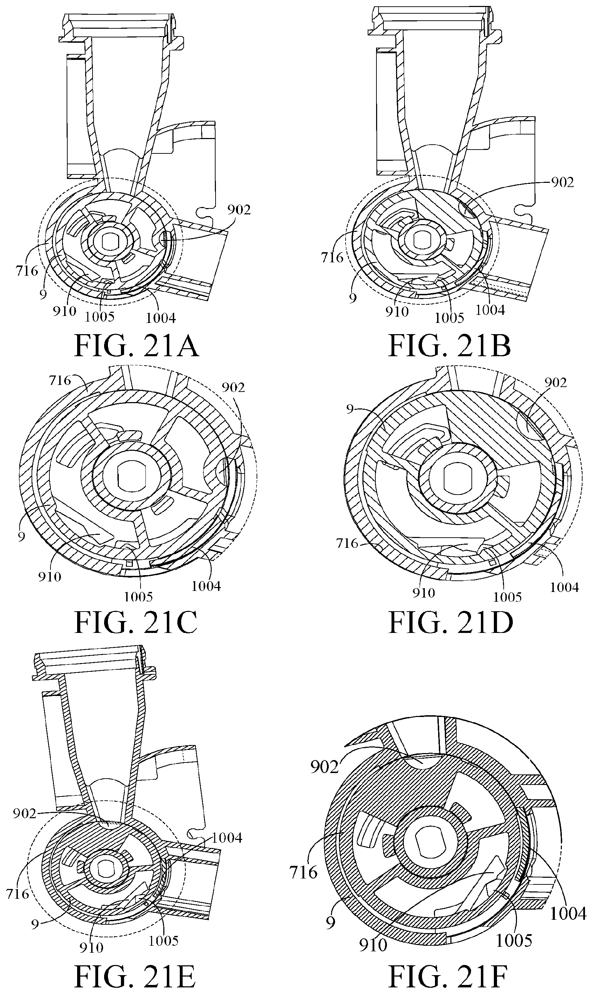

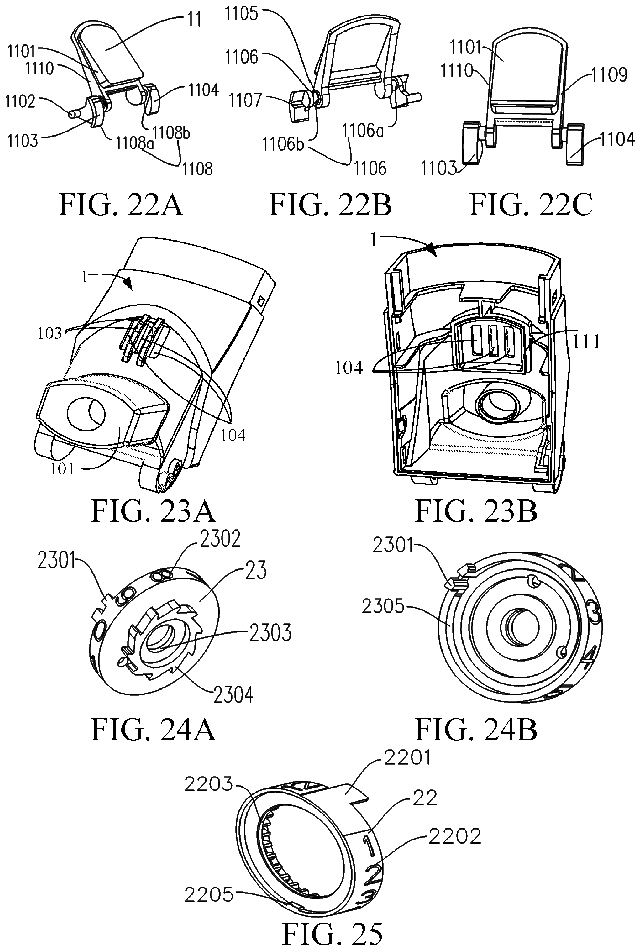

Refer to FIG. 15A to FIG. 23B. FIG. 15A is a schematic diagram of a structure of an inhalation trigger device that is of the powder inhaler provided in FIG. 1 and that is in a state in a powder container; FIG. 15B is a schematic diagram of a structure of the inhalation trigger device that is provided in FIG. 15A and that is in another state in a powder container; FIG. 16A is a schematic diagram of a structure of the inhalation trigger device provided in FIG. 15A with a powder container removed; FIG. 16B is a schematic diagram of a structure of the inhalation trigger device provided in FIG. 15B with a powder container removed; FIG. 17A is a schematic diagram of a structure of the inhalation trigger device that is provided in FIG. 16A and that is viewed from another angle; FIG. 17B is a schematic diagram of a structure of the inhalation trigger device provided in FIG. 16B viewed from another angle; FIG. 18A is a schematic diagram of an exploded structure of a counting mechanism of the powder inhaler provided in FIG. 1; FIG. 18B is a schematic diagram of an assembly structure of the counting mechanism provided in FIG. 18A; FIG. 20 is a schematic diagram of a structure of a dose protection plate of an inhalation trigger device of the powder inhaler provided in FIG. 1; FIG. 21A is a schematic cross-sectional view of assembly of a powder metering wheel and a dose protection plate that are of the powder inhaler provided in FIG. 1 and that are in a state; FIG. 21B is a schematic cross-sectional view of assembly of a powder metering wheel and a dose protection plate that are of the powder inhaler provided in FIG. 1 and that are in another state; FIG. 21C is a partially enlarged schematic diagram of FIG. 21A; FIG. 21D is a partially enlarged schematic diagram of FIG. 21B; FIG. 21E is a schematic cross-sectional view of assembly of a powder metering wheel and a dose protection plate that are of the powder inhaler provided in FIG. 1 and that are in still another state; FIG. 21F is a partially enlarged schematic diagram of FIG. 21E; FIG. 22A is a schematic diagram of a structure of an air inlet baffle that is of an inhalation trigger device of the powder inhaler provided in FIG. 1 and that is viewed from an angle; FIG. 22B is a schematic diagram of a structure of the air inlet baffle that is provided in FIG. 22A and that is viewed from another angle; FIG. 22C is a schematic diagram of a structure of the air inlet baffle that is provided in FIG. 22A and that is viewed from still another angle; FIG. 23A is a schematic diagram of a structure of a front housing that is of the powder inhaler provided in FIG. 1 and that is viewed from an angle; and FIG. 23B is a schematic diagram of a structure of a front housing that is of the powder inhaler provided in FIG. 1 and that is viewed from another angle.

### (3) Inhalation trigger mechanism

Referring to FIG. 15A to FIG. 23B, the inhalation trigger mechanism includes an air inlet baffle 11 and a dose protection plate 10, the dose protection plate 10 includes a blocking portion 1004, and the air inlet baffle 11 is linked to and cooperates with the dose protection plate 10. A powder inhaler further includes a resetting torsion spring 15 and a drive torsion spring 16. The air inlet baffle 11 and the dose protection plate 10 of the inhalation trigger mechanism, in combination with the resetting torsion spring 15, the drive torsion spring 16, a powder container 7, a powder metering wheel 9, and a drive cam 12, jointly implement the inhalation trigger function.

Referring to FIG. 13A and FIG. 13B, the powder container 7 further has an airflow channel 708 in communication with an inhalation channel 706, and the inhalation channel 706 needs to be in communication with external air via the airflow channel 708. Referring to FIG. 13A to FIG. 17B, when the inhalation trigger mechanism is in an initial state, the air inlet baffle 11 seals the airflow channel 708, and the airflow channel 708 is not in communication with the external air. The blocking portion 1004 of the dose protection plate 10 blocks a powder outlet of a dose cup 902 located at an inlet 704 of the inhalation channel 706. In this case, the inhalation channel 706 cannot be in communication with the external air via the airflow channel 708. When negative pressure in the inhalation channel 706 exceeds a threshold, that is, when a flow rate of user inhalation exceeds an operating threshold, the inhalation trigger mechanism is triggered to act. Specifically, the air inlet baffle 11 rotates and enables the airflow channel 708 to be opened, the airflow channel 708 is in communication with the external air, and the inhalation channel 706 is in communication with the external air via the airflow channel 708. The air inlet baffle 11 triggers the dose protection plate 10 to rotate, causing the blocking portion 1004 of the dose protection plate 10 to deviate and unblock the powder outlet of the dose cup 902. In this way, the powder outlet of the dose cup 902 is exposed at the position of the inlet 704 of the inhalation channel 706. The operating threshold may range from 15 L/min to 35 L/min. Preferably, the operating threshold for triggering the inhalation trigger mechanism to act ranges from 20 L/min to 25 L/min.

It can be understood that, the inhalation trigger mechanism is disposed in the powder inhaler. Only when the flow rate of the user inhalation exceeds the operating threshold, the air inlet baffle 11 rotates to enable the airflow channel 708 to be opened, so that the inhalation channel 706 is in communication with the external air via the airflow channel 708. In addition, rotation of the air inlet baffle 11 triggers the dose protection plate 10 to rotate so as to unblock the powder outlet of the dose cup 902, so that the powder outlet of the dose cup 902 is in communication with the inhalation channel 706. Consequently, powder in the dose cup 902 is exposed, and is brought out by an air flow in the inhalation channel 706 and is deagglomerated. In this case, an airflow rate is relatively high, resulting in a better deagglomeration effect. This implements flow rate threshold control for powder release, improves a deagglomeration effect during powder release, and avoids powder waste.

Referring to FIG. 13B and FIG. 15A to FIG. 22C, a side wall of an air inlet port of the airflow channel 708 has a mounting hole 707, the air inlet baffle 11 is rotatably mounted in the mounting hole 707, and a side wall of the airflow channel 708 surrounds the air inlet baffle 11.

Specifically, the air inlet baffle 11 includes a baffle body 1110, and the baffle body 1110 is rotatably connected to the side wall of the airflow channel 708. In an implementation, the air inlet baffle 11 further includes a rotating shaft 1106, the rotating shaft 1106 is disposed at a first end of the baffle body 1110, the rotating shaft 1106 runs through the mounting hole 707 on the side wall of the port of the airflow channel 708, and is rotatably connected to the mounting hole 707.

Referring to FIG. 9G, FIG. 9H, and FIG. 13A to FIG. 22C, a first surface of the baffle body 1110 has a boss 1101. The first surface is a surface of the baffle body 1110 that faces the outer side of the port of the airflow channel 708. The boss 1101 is defined as a second boss, and the boss 1101 is configured to increase complexity of a flow channel on a side surface. Specifically, the boss 1101 protruding from an outer surface of the baffle body 1110 is disposed on the baffle body 1110, so that the flow channel on the side surface changes from a straight-through flow channel to an "L"-shaped flow channel, thereby increasing flow resistance of an air flow, implementing a lower trigger flow rate for a same air-exposed area, and facilitating implementation of an inhalation trigger function, so as to make the powder inhalation device easier to use for a frail user. The "L"-shaped flow channel refers to a flow channel including a first flow channel segment and a second flow channel segment that are bent. An included angle between the first flow channel segment and the second flow channel segment may range from 80 degrees to 100 degrees, for example, 90 degrees, which will be described in detail below.

In this application, the outer surface of the baffle body 1110 is defined relative to the port of the airflow channel 708, where a surface facing the outer side of the port of the airflow channel 708 is defined as the outer surface. The boss 1101 may be directly formed by the outer surface of the baffle body 1110 protruding outward, for example, the baffle body 1110 has a solid structure. The boss 1101 may also be formed by the baffle body 1110 being recessed, that is, the boss 1101 is formed by bending a part of the baffle body 1110 toward the outer side of the port of the airflow channel 708, so that the baffle body 1110 forms only a frame, thereby reducing a weight of the air inlet baffle 11. The boss 1101 and the baffle body 1110 may be integrally formed, or the boss 1101 may be directly connected and fixed to the outer surface of the baffle body 1110.

In an implementation, the boss 1101 covers a central region of a surface of the baffle body 1110 that faces the outer side of the port the airflow channel 708, a projection of the boss 1101 on the baffle body 1110 is similar in shape to the baffle body 1110, and the projection of the boss 1101 on the baffle body 1110 covers more than 60% of an area of the baffle body 1110, to further increase flow resistance of the flow channel on the side surface, ensure consistency of inhalation resistance of the powder inhaler at various stages of a drug delivery process, and improve a drug delivery effect of the powder inhaler, thereby enhancing user compliance and providing a better user experience. An area of the baffle body 1110 covered by the projection of the boss 1101 on the baffle body 1110 is related to a width of the annular surface 1109 and may be designed as needed.

In another implementation, the boss 1101 covers another region of the surface of the baffle body 1110 that faces the outer side of the port of the airflow channel 708, the boss 1101 may also be disposed in another shape, and a ratio of a projection area of the boss 1101 on the baffle body 1110 to the area of the baffle body 1110 may also be set to another value, provided that the boss 1101 can increase complexity of the flow channel on the side surface and enhance the flow resistance of the air flow.

In an implementation, along a direction from the first end of the baffle body 1110 to an opposite second end (a direction away from the rotating shaft 1106) of the baffle body 1110, a height of the boss 1101 gradually decreases, so that a top surface of the boss 1101 forms an inclined surface, and the inclined surface provides a specific flow guiding effect, to facilitate guiding a direction of the air flow, thereby better facilitating implementation of an inhalation trigger function.

Specifically, referring to FIG. 9G, FIG. 9H, FIG. 15A, FIG. 15B, and FIG. 22A to FIG. 23B, a front housing 1 has a suction nozzle 101. The suction nozzle 101 is disposed corresponding to the inhalation channel 706 and is in communication with the inhalation channel 706. A side wall of the front housing 1 is provided with an air inlet 104 and a grille 103. The grille is disposed to protrude from an outer wall surface of the front housing 1. The air inlet 104 is in communication with the external air and internal space of a housing assembly. In a specific implementation, the grille 103 is disposed corresponding to a position above the suction nozzle 101 and is adjacent to the air inlet 104. The grille 103 is disposed to protrude from the outer wall surface of the front housing 1, to prevent the user's lips from sealing the air inlet 104 due to contact with the air inlet 104 when the user inhales drug powder at a position of the suction nozzle 101, causing a problem of unsmooth air inlet or the external air failing to enter the inside of the housing assembly via the air inlet 109. As shown in FIG. 23B, in a preferred implementation, the side wall of the front housing 1 is provided with three air inlets 104 and two grilles 103, and the grilles 103 and the air inlets 104 are alternately disposed. In another implementation, the air inlets 104 and the grilles 103 may be disposed at other positions, and may also be disposed in any other quantity.

As shown in FIG. 22C, along a circumferential direction of the boss 1101, an outer peripheral side surface of the boss 1101 and an outer peripheral side surface of the baffle body 1110 are evenly spaced apart, and a part of the surface of the baffle body 1110 that faces the outer side of the port of the airflow channel 708 and that is not covered by the boss 1101 forms an annular surface 1109. Both the annular surface 1109 and the outer peripheral side surface of the boss 1101 are configured to cooperate with the front housing 1 of the powder inhaler to form an L-shaped air inlet flow channel. Specifically, as shown in FIG. 23A and FIG. 23B, an inner wall surface of the front housing 1 is provided with an annular flange 111, and the annular flange 111 is disposed surrounding the air inlet 104. Specifically, the annular flange 111 is disposed surrounding the three air inlets 104 and the two grilles 103. Referring to FIG. 9C to FIG. 9H, one end of the annular flange 111 is disposed in the airflow channel 708, and the side wall of the front housing 1 seals the port of the airflow channel 708. When the inhalation trigger mechanism is in an initial state, an inner peripheral side surface of the annular flange 111 of the front housing 1 is spaced apart from and cooperates with an outer peripheral side surface of the boss 1101 of the air inlet baffle 11 to form a first flow channel segment. An end surface at an end of the annular flange 111 that is away from the front housing 1 abuts against and cooperates with the annular surface 1109 of the air inlet baffle 11 to form a second flow channel segment, and the first flow channel segment is in communication with the second flow channel segment to form an L-shaped air inlet flow channel Q1. That is, before the inhalation trigger mechanism is triggered, the external air enters internal space of the housing assembly through the air inlet 104 on the front housing 1, and flows through the L-shaped air inlet flow channel Q1 formed by cooperation of the baffle body 1110 and the boss 1101 of the air inlet baffle 11 with the annular flange 111 of the front housing 1, allowing the external air to enter the inside of the powder inhaler, to ensure consistency of inhalation resistance of the powder inhaler at various stages of the drug delivery process, prevent excessive inhalation resistance inside the powder inhaler prior to inhalation trigger from affecting the air compressing process or the powder delivery process, and improve a drug delivery effect of the powder inhaler.

When a user inhales at the suction nozzle 101 and negative pressure in the inhalation channel 706 exceeds a threshold, that is, when an airflow rate during user inhalation exceeds an operating threshold, the internal negative pressure drives the air inlet baffle 11 to rotate and enables the airflow channel 708 to be opened, and the airflow channel 708 is in communication with the external air via the air inlet 104.

As shown in FIG. 9G and FIG. 9H, a distance between the top surface of the boss 1101 of the air inlet baffle 11 and the surface of the baffle body 1110 that faces the outer side of the port of the airflow channel 708 is a first distance L1, that is, the height of the boss 1101 is L1, a thickness of the annular flange 111 on an inner wall surface of the front housing 1 is a second distance L2, and a ratio of the first distance L1 to the second distance L2 is 1:2 to 7:1. Preferably, the first distance L1 is greater than the second distance L2, and a ratio of the first distance L1 to the second distance L2 is 1:1 to 5:1. Setting the ratio between the first distance L1 and the second distance L2 within the foregoing range can increase a pressure difference between an inner side and an outer side of the air inlet baffle 11, that is, increase a pressure difference between a side of the air inlet baffle 11 that is close to the airflow channel 708 and a side of the air inlet baffle 11 that is close to the air inlet 104 of the front housing 1, and increase other resistance. The increased pressure difference on two sides of the air inlet baffle 11 is more conducive to driving the air inlet baffle 11 to rotate to enable the airflow channel 708 to be opened, thereby facilitating implementation of the inhalation trigger function.

In an implementation, the air inlet baffle 11 further includes a rotating member 1108, the rotating shaft 1106 is disposed at one end of the baffle body 1110, and the rotating member 1108 is connected to a free end of the rotating shaft 1106 and is spaced apart from the baffle body 1110. A first end of the rotating member 1108 has a curved surface, and the curved surface faces the outer side of the port of the airflow channel 708 (specifically, a line connecting two ends of the curved surface is substantially parallel to the baffle body 1110). Specifically, the curved surface may be an arc surface, and is used to guide an air flow, so that lengths of airflow paths on two sides of the baffle body 1110 are equal.

Specifically, referring to FIG. 9C, FIG. 13A, FIG. 13B, FIG. 15A, and FIG. 15B, the side wall of the airflow channel 708 of the powder container 7 is connected to a side wall of the storage cavity 715. One end of the side wall of the airflow channel 708 that is connected to the side wall of the storage cavity 715 is provided with a first flow guiding hole 710 and a second flow guiding hole 717 that are spaced apart from each other. One end of the inhalation channel 706 that is close to the suction nozzle 101 has a first airflow inlet 718 and a second airflow inlet 719 that are spaced apart from each other. The first flow guiding hole 710 communicates the airflow channel 708 with the first airflow inlet 718, and the second flow guiding hole 717 communicates the airflow channel 708 with the second airflow inlet 719. The inner wall surface of the front housing 1 and an outer wall surface of the inhalation channel 706 are spaced apart to form a flow guiding channel 720 (as shown in FIG. 9C), and the flow guiding channel 720 communicates the airflow channel 708 with the first airflow inlet 718 and the second airflow inlet 719. Referring to FIG. 15B, when an airflow rate during user inhalation exceeds an operating threshold and negative pressure in the inhalation channel 706 exceeds a threshold, the air inlet baffle 11 rotates and enables the airflow channel 708 to be opened, so that the air inlet 104 on the front housing 1 is in communication with the first flow guiding hole 710 and the second flow guiding hole 717 via the airflow channel 708. After the external air enters the inside of the housing assembly via the air inlet 104 of the front housing 1, a part of air flows to the first flow guiding hole 710 via the airflow channel 708, another part of air flows to the second flow guiding hole 717 via the airflow channel 708, and remaining air flows to the flow guiding channel 720 via the airflow channel 708. The air flowing out of the first flow guiding hole 710 flows along the outer side surface of the side wall of the airflow channel 708 to the second curved surface 1104 and finally to the first airflow inlet 718, the air flowing out of the second flow guiding hole 717 flows along the outer side surface of the side wall of the airflow channel 708 to the first curved surface 1103 and finally to the second airflow inlet 719, and the air flowing out of the flow guiding channel 720 can enter both the first airflow inlet 718 and the second airflow inlet 719, thereby facilitating inhalation of powder via the inhalation channel 706. It can be understood that, providing three air inlets 104 on the front housing 1 and providing the first flow guiding hole 710, the second flow guiding hole 717, and the flow guiding channel 720 respectively enable airflow cross-sectional areas of air flows in the three paths to match airflow cross-sectional areas of the three air inlets 104 of the front housing 1, to avoid air loss during air inlet. In addition, both the first curved surface 1103 and the second curved surface 1104 are arc surfaces, and can guide a direction of the air flow, so that lengths of airflow paths on two sides of the baffle body 1110 are equal.

In an implementation, as shown in FIG. 13B and FIG. 15B, two oppositely disposed arc-shaped ribs are disposed in the inhalation channel 706, and the two arc-shaped ribs and the side wall of the inhalation channel 706 surround and form a vortex-shaped airflow channel. Air enters the inhalation channel 706 through the first airflow inlet 718 and the second airflow inlet 719 respectively to form a vortex, and the vortex entrains the drug powder in the dose cup 902 at the inlet 704 of the inhalation channel 706 into the inhalation channel 706. After being deagglomerated in the inhalation channel 706, the drug powder finally flows to the suction nozzle 101 and is inhaled by the user. The foregoing setting is more conducive to deagglomeration of powder such as the drug powder in the inhalation channel 706, to avoid drug powder waste and improve utilization of the drug powder.

Referring to FIG. 22A to FIG. 22C, a surface of a second end of the rotating member 1108 that is away from the baffle body 1110 has a protruding cylinder 1102. The cylinder 1102 is defined as a third cylinder, and is configured to abut against a drive arm of the resetting torsion spring 15 of the powder inhaler, so that the baffle body 1110 is attached to the front housing 1. Specifically, in an implementation, a quantity of rotating shafts 1106 is two, the two rotating shafts 1106 are respectively disposed on two opposite sides of the baffle body 1110, and the two rotating shafts 1106 are respectively defined as a first rotating shaft 1106a and a second rotating shaft 1106b. A quantity of rotating members 1108 is two, and the two rotating members 1108 are respectively defined as a first rotating member 1108a and a second rotating member 1108b. The first rotating member 1108a is connected to a free end of the first rotating shaft 1106a. A first end of the first rotating member 1108a has a first curved surface 1103, and a surface of a second end of the first rotating member 1108a that is away from the baffle body 1110 has the protruding cylinder 1102. The second rotating member 1108b is connected to a free end of the second rotating shaft 1106b, a first end of the second rotating member 1108b has a second curved surface 1104, and a second end of the second rotating member 1108b has a circular-arc-shaped groove surface 1107.

In an implementation, a side surface of the baffle body 1110 further includes a shoulder 1105 surrounding the rotating shaft 1106, the shoulder 1105 is spaced apart from the rotating member 1108, and the shoulder 1105 is configured to maintain an even gap between two sides of the baffle body 1110 and the side wall of the airflow channel 708, thereby facilitating more stable implementation of the inhalation trigger function.

Referring to FIG. 13A, FIG. 13B, and FIG. 15A to FIG. 22C, the powder container 7 further includes a second cylindrical groove 712. The second cylindrical groove 712 and the first cylindrical groove 716 are provided coaxially with and opposite to each other and have a common bottom wall (not labeled in the figure). A counting mechanism and a dose protection plate 10 are mounted in the second cylindrical groove 712. Referring to FIG. 15A to FIG. 19B, the counting mechanism includes a counter base 21. The counter base 21 has a cylindrical shaft 2111. Both the dose protection plate 10 and the counter base 21 of the counting mechanism are mounted in the second cylindrical groove 712 on the powder container 7, and the counter base 21 axially limits the dose protection plate 10. The powder inhaler further includes a drive torsion spring 16 and a resetting torsion spring 15. The drive torsion spring 16 is sleeved on a cylindrical shaft 2111 of the counter base 21. The counter base 21 is provided with a limiting groove 2109, and a fixed arm of the drive torsion spring 16 is fixed on the limiting groove 2109 on the counter base 21. The resetting torsion spring 15 is mounted on a gear bracket 14.

Referring to FIG. 15A to FIG. 20, the dose protection plate 10 includes an annular body 1000, a blocking portion 1004, and a pressing member 1006. The pressing member 1006 of the dose protection plate 10 is disposed on an outer side surface of the annular body 1000. The blocking portion 1004 is connected to one end of the annular body 1000, and is spaced apart from the pressing member 1006. The blocking portion 1004 is configured to block or unblock the dose cup 902. The annular body 1000 is disposed in the second cylindrical groove 712 of the powder container 7. As shown in FIG. 18B, the annular body 1000 is configured to accommodate the counting mechanism. A side wall of the second cylindrical groove 712 of the powder container 7 has a notch, and the pressing member 1006 extends out of the second cylindrical groove 712 through the notch on the side wall of the second cylindrical groove 712, and can rotate back and forth in the notch. As shown in FIG. 20, a surface of the pressing member 1006 that is away from the annular body 1000 includes a pressing arc surface 1002. When the inhalation trigger mechanism is in an initial state, that is, before the inhalation trigger mechanism is triggered, or after the inhalation trigger mechanism has reset, the circular-arc-shaped groove surface 1107 of the rotating member 1108 of the air inlet baffle 11 cooperates with the pressing arc surface 1002 of the dose protection plate 10 to implement concentric arc surface pressing.

The blocking portion 1004 is connected to one end of the annular body 1000, and is configured to block or unblock the dose cup 902. As shown in FIG. 13B, the common bottom wall of the powder container 7 has an arc-shaped notch (not labeled in the figure), so that the blocking portion 1004 of the dose protection plate 10 passes through the arc-shaped notch to enter the first cylindrical groove 716, and can rotate back and forth in the arc-shaped notch, to be located at the position of the inlet 704 of the inhalation channel 706 and block the dose cup 902 at the position before the inhalation trigger mechanism is triggered, and rotate in the arc-shaped notch to deviate from the position of the inlet 704 of the inhalation channel 706 to unblock the dose cup 902 after the inhalation trigger mechanism is triggered, so as to expose the drug powder in the dose cup 902 to facilitate inhalation.

Specifically, referring to FIG. 15A to FIG. 17B and FIG. 20, the pressing member 1006 includes a cylindrical convex surface 1001, and the cylindrical convex surface 1001 is disposed on one side of the pressing arc surface 1002. As shown in FIG. 16A and FIG. 16B, a drive arm of a drive torsion spring 16 of the powder inhaler acts on the cylindrical convex surface 1001, so that when an airflow rate during user inhalation exceeds an operating threshold and the air inlet baffle 11 rotates, after the pressing arc surface 1002 of the dose protection plate 10 disengages from the circular-arc-shaped groove surface 1107 of the air inlet baffle 11, the dose protection plate 10 rotates under the action of the drive arm of the drive torsion spring 16, so that the blocking portion 1004 of the dose protection plate 10 changes from blocking the dose cup 902 of the powder metering wheel 9 to unblocking the dose cup 902 of the powder metering wheel 9, to expose the powder in the dose cup 902 of the powder metering wheel 9 to an air flow to be inhaled by the user.

A movement manner of the inhalation trigger mechanism in the inhalation trigger process is specifically described below.

Referring to FIG. 9D and FIG. 15A to FIG. 22C, before the outer cap 4 is fully opened, as shown in FIG. 15A, FIG. 16A, FIG. 17A, and FIG. 22A, the drive arm of the resetting torsion spring 15 acts on the cylinder 1102 of the air inlet baffle 11, and the drive arm of the resetting torsion spring 15 presses the air inlet baffle 11 to be attached to the front housing 1, so that the boss 1101 is embedded in the annular flange 111, the air inlet baffle 11 does not rotate, and the inhalation channel 706 cannot be in communication with the external air via the airflow channel 708. As shown in FIG. 20 and FIG. 15A, the drive arm of the drive torsion spring 16 acts on the cylindrical convex surface 1001 of the pressing member 1006 of the dose protection plate 10. In this case, the pressing arc surface 1002 of the pressing member 1006 of the dose protection plate 10 acts on the circular-arc-shaped groove surface 1107 of the rotating member 1108 of the air inlet baffle 11 (as shown in FIG. 16A), to implement concentric arc surface pressing.

A guide groove 1209 of the drive cam 12 is located on a surface of the body portion 1200 that faces the gear 1203, and a surface of the body portion 1200 that faces away from the gear 1203 has a rib 1208. When the outer cap 4 is fully opened, that is, after the outer cap 4 rotates to the second position, the rib 1208 of the drive cam 12 presses the drive arm of the resetting torsion spring 15 away from the cylinder 1102 of the air inlet baffle 11 (as shown in FIG. 17B). The rib 1208 releases limitation imposed by the resetting torsion spring 15 on the air inlet baffle 11 of the inhalation trigger mechanism. In this case, the air inlet baffle 11 is not subjected to a pressing force of the resetting torsion spring 15, and only a pressing frictional force exerted by the pressing arc surface 1002 of the dose protection plate 10 on the circular-arc-shaped groove surface 1107 of the air inlet baffle 11 remains. In addition, after the outer cap 4 rotates to the second position, the dose cup 902 of the powder metering wheel 9 is delivered to the position of the inlet 704 of the inhalation channel 706, and the blocking portion 1004 of the dose protection plate 10 is located at the position of the inlet 704 of the inhalation channel 706 and blocks the powder outlet of the dose cup 902.

After the outer cap 4 is fully opened and before the inhalation trigger mechanism is triggered, the air inlet baffle 11 is subjected only to the pressing frictional force exerted by the pressing arc surface 1002 of the dose protection plate 10 on the circular-arc-shaped groove surface 1107 of the air inlet baffle 11, and the air inlet baffle 11 still does not rotate. When a user inhales and an airflow rate during user inhalation exceeds an operating threshold, and negative pressure in the inhalation channel 706 exceeds a threshold, an airflow thrust generated by an inhalation air flow acts on the air inlet baffle 11, to overcome the frictional force exerted by the pressing arc surface 1002 on the circular-arc-shaped groove surface 1107, so that the inhalation trigger mechanism is triggered, and the air inlet baffle 11 generates yaw rotation. After the air inlet baffle 11 rotates, the circular-arc-shaped groove surface 1107 of the air inlet baffle 11 synchronously rotates, causing the pressing arc surface 1002 of the dose protection plate 10 to disengage from the circular-arc-shaped groove surface 1107 of the air inlet baffle 11 (as shown in FIG. 16B). Driven by a driving force of the drive torsion spring 16, the dose protection plate 10 rotates, and the blocking portion 1004 of the dose protection plate 10 synchronously rotates in the circular-arc-shaped notch, so that the blocking portion 1004 of the dose protection plate 10 deviates and unblocks the powder outlet of the dose cup 902. In this case, the powder outlet of the dose cup 902 of the powder metering wheel 9 is exposed to the inhalation channel 706. Under the action of the inhalation air flow of the user, the powder in the dose cup 902 flows through the inhalation channel 706 and the suction nozzle 101 and is inhaled by the user. In a preferred implementation, the dose protection plate 10 rotates downward by 38 degrees under the action of the driving force of the drive torsion spring 16, so that the powder outlet of the dose cup 902 is in communication with the inhalation channel 706, and the powder in the dose cup 902 is exposed to the air flow and is carried away.

Referring to FIG. 14B and FIG. 20, the dose protection plate 10 further includes a wedge-shaped post 1005. One end of the wedge-shaped post 1005 is connected to the annular body 1000, and is spaced apart from both the blocking portion 1004 and the pressing member 1006, and the other end of the wedge-shaped post 1005 is configured to cooperate with the drive elastic arm 910 of the powder metering wheel 9, so that in a cap-closing process of the outer cap 4, the drive elastic arm 910 drives the dose protection plate 10 to rotationally reset via the wedge-shaped post 1005. A specific process and manner in which the drive elastic arm 910 drives, via the wedge-shaped post 1005, the dose protection plate 10 to rotate and reset are described in detail subsequently in a process in which the outer cap 4 is closed to trigger reset of a functional mechanism. Details are not described in further detail herein.

Referring to FIG. 20, the dose protection plate 10 further includes an elastic arm claw 1003, and one end of the elastic arm claw 1003 is connected to the annular body 1000. Specifically, one end of the elastic arm claw 1003 is connected to the inner wall surface of the annular body 1000, and the other end of the elastic arm claw 1003 is configured to drive a counting mechanism to count. The following provides detailed descriptions of the counting mechanism, and a manner and process in which the elastic arm claw 1003 drives the counting mechanism to perform counting.

Refer to FIG. 24A to FIG. 25. FIG. 24A is a schematic diagram of a structure of a units digit wheel that is of the counting mechanism provided in FIG. 18A and that is viewed from an angle; FIG. 24B is a schematic diagram of a structure of the units digit wheel that is provided in FIG. 24A and that is viewed from another angle; and FIG. 25 is a schematic diagram of a structure of a tens digit wheel of the counting mechanism provided in FIG. 18A.

### (4) Counting mechanism

Referring to FIG. 18A, FIG. 18B, and FIG. 22A to FIG. 25, the counting mechanism includes a counter base 21, a tens digit wheel 22, a units digit wheel 23, and a counter intermediate gear 24, and the counting mechanism implements a counting function in cooperation with a dose protection plate 10 and a powder container 7. Specifically, the tens digit wheel 22 is mounted on the counter base 21. The counter base 21 has a cylindrical surface 2101, a snap catch 2102, and an outer circular arc boss 2108. The tens digit wheel 22 has an inner ring 2203 and an inner circular arc boss 2205. The cylindrical surface 2101 of the counter base 21 cooperates with the inner ring 2203 of the tens digit wheel 22 to implement coaxial rotation. The snap catch 2102 on the counter base 21 axially limits the tens digit wheel 22. The outer circular arc boss 2108 of the counter base 21 cooperates with the inner circular arc boss 2205 of the tens digit wheel 22 to rotationally limit the tens digit wheel 22.

Referring to FIG. 4 and FIG. 25, a digital display window 201 is provided on the housing assembly. Specifically, the digit display window 201 is provided on a rear housing 2 of the housing assembly, and the tens digit wheel 22 further has a full red warning feature 2201. When the full red warning feature 2201 of the tens digit wheel 22 is displayed on the digit display window 201 on the rear housing 2, the tens digit wheel 22 is limited and does not rotate.

The counter base 21 is further provided with a first mounting hole 2103, the counter intermediate gear 24 is mounted on the first mounting hole 2103 of the counter base 21, and the counter intermediate gear 24 meshes with the tens digit wheel 22 through gear features to implement transmission.

The units digit wheel 23 has a second mounting hole 2303 and a toothed shift post 2301, the counter base 21 is provided with a snap-fit post 2105, and the second mounting hole 2303 on the units digit wheel 23 cooperates with the snap-fit post 2105 on the counter base 21 to implement coaxial mounting and axial limiting. The units digit wheel 23 has an annular guide structure 2305 for cooperative mounting with the tens digit wheel 22. First digits 2302 are printed on an outer peripheral side surface of the units digit wheel 23, and second digits 2202 are printed on an outer peripheral side surface of the tens digit wheel 22, to facilitate counting. The toothed shift post 2301 on the units digit wheel 23 cooperates with the counter intermediate gear 24. When the units digit wheel 23 completes one full rotation and is transitioning from a digit "0" to a digit "9", the toothed shift post 2301 of the units digit wheel 23 drives the counter intermediate gear 24 to rotate by two teeth. In addition, the tens digit wheel 22 meshes with the counter intermediate gear 24, and the tens digit wheel 22 synchronously rotates by two teeth to implement transition of one digit.

Referring to FIG. 16A to FIG. 20, the units digit wheel 23 is driven by back-and-forth movement of the dose protection plate 10. Specifically, as shown in FIG. 24A, ratchet teeth 2304 are disposed on the units digit wheel 23. Referring to FIG. 20, the dose protection plate 10 has an elastic arm claw 1003, one end of the elastic arm claw 1003 is connected to the annular body 1000, the ratchet teeth 2304 on the units digit wheel 23 cooperate with the elastic arm claw 1003 of the dose protection plate 10, and before the inhalation trigger mechanism is triggered, the elastic arm claw 1003 engages one of the ratchet teeth 2304 on the units digit wheel 23. When the inhalation trigger mechanism is triggered to move, the air inlet baffle 11 rotates, thereby triggering the dose protection plate 10 to rotate under the action of the drive arm of the drive torsion spring 16. Rotation of the dose protection plate 10 causes the elastic arm claw 1003 to synchronously rotate, and after the elastic arm claw 1003 rotates, the elastic arm claw 1003 engages a next ratchet tooth 2304 on the units digit wheel 23. In a cap-closing process of the outer cap 4, the inhalation trigger mechanism resets, and the dose protection plate 10 reversely rotates and resets to an original state before the inhalation trigger mechanism is triggered. Because the elastic arm claw 1003 of the dose protection plate 10 engages the next ratchet tooth 2304 on the units digit wheel 23, in this process, as the dose protection plate 10 reversely rotates, the engaged ratchet tooth 2304 also rotates, and the units digit wheel 23 rotates under the action of the elastic arm claw 1003 to implement transition of one digit.

In a preferred implementation, the units digit wheel 23 is provided with ten ratchet teeth 2304. When the inhalation trigger mechanism is triggered to move, the dose protection plate 10 is triggered to rotate by 38 degrees, and the elastic arm claw 1003 engages a next ratchet tooth 2304. When the inhalation trigger mechanism resets, the dose protection plate 10 reversely rotates by 38 degrees, and the units digit wheel 23 rotates by 36 degrees under the action of the elastic arm claw 1003 to implement transition of one digit.

Further, the powder container 7 is further provided with a limiting elastic arm 711. Specifically, as shown in FIG. 13B, a side surface of a second cylindrical groove 712 of the powder container 7 has the limiting elastic arm 711, and the limiting elastic arm 711 is configured to limit the counting mechanism to unidirectional rotation. The limiting elastic arm 711 disposed on the powder container 7 cooperates with the ratchet teeth 2304 on the units digit wheel 23 to implement unidirectional rotation of the units digit wheel 23. That is, when the dose protection plate 10 rotates downward, the elastic arm claw 1003 on the dose protection plate 10 scrapes against the units digit wheel 23, and due to the action of the limiting elastic arm 711 on the ratchet teeth 2304 of the units digit wheel 23, the units digit wheel 23 does not rotate along with the dose protection plate 10. When the dose protection plate 10 resets and reversely rotates, the elastic arm claw 1003 on the dose protection plate 10 engages and pulls the units digit wheel 23 to rotate by 36 degrees, thereby implementing unidirectional decremental counting. The foregoing setting can effectively avoid a problem that when the dose protection plate 10 rotates downward, the elastic arm claw 1003 drives the units counting wheel 23 and consequently a counter is abnormal.

For ease of understanding, a cooperative relationship between various functional mechanisms is described below with reference to the cap-closing process, along with how the counting mechanism is triggered to count and how the various functional mechanisms are reset in the cap-closing process.

In the cap-closing process of the outer cap 4, namely, a process in which the outer cap 4 reversely rotates and resets from the second position to the first position, the outer cap 4 drives a powder delivery mechanism and an air compressing mechanism respectively to reset, and triggers the air inlet baffle 11 to reversely rotate and reset, so that the powder inhaler restores to an original state. Reset of the dose protection plate 10 drives the counter to advance counting by one digit.

An angle of the outer cap 4 at the first position is defined as 0 degrees, and an angle of the outer cap 4 at the second position is greater than or equal to 120 degrees and less than or equal to 180 degrees. In an implementation, an angle of the outer cap 4 at the second position is 150 degrees. In a cap-closing process of the outer cap 4 (a process in which the outer cap 4 resets from the second position to the first position), that is, in a process in which the outer cap 4 reversely rotates from 150 degrees to 0 degrees, the outer cap 4 first reversely rotates for an idle stroke, and drives a drive cam 12 to reversely rotate for an idle stroke. Preferably, the outer cap 4 first reversely rotates for an idle stroke of 62.5 degrees, and a corresponding drive cam 12 reversely rotates for an idle stroke of 75 degrees, that is, when the cap-closing idle stroke of the outer cap 4 ends, an angle of the outer cap 4 is 87.5 degrees, and an angle of the drive cam 12 is 105 degrees. In a process in which the outer cap 4 reversely rotates from 87.5 degrees to 0 degrees, the drive cam 12 drives the powder metering wheel 9 to rotate and reset, the powder metering wheel 9 rotates and resets from the fourth position to the third position, and the dose cup 902 of the powder metering wheel 9 rotates from a position corresponding to an inlet 704 of an inhalation channel 706 to a position corresponding to a powder outlet 713 of the powder container 7, thereby resetting the powder delivery mechanism. In this process, the drive cam 12 reversely rotates from 105 degrees to 0 degrees. When the outer cap 4 resets to the first position, that is, after cap closing is completed, an arc-shaped rib 405 on the outer cap 4 cooperates with a sound elastic arm 108 on a front housing 1, to provide an audible prompt indicating that the cap is fully closed, so as to prompt that the outer cap 4 is cap is fully closed.

Because gear meshing transmission is used between the outer cap 4 and the drive gear 5, a gear gap exists, which prevents the outer cap 4 from tightly attaching to the front housing 1 after the outer cap 4 is closed. To eliminate a problem that the outer cap 4 cannot be tightly attached to the front housing 1 due to influence of the gear gap after the outer cap 4 is closed, as shown in FIG. 8A and FIG. 8B, a tightening elastic arm 502 is disposed on the drive gear 5, and the tightening elastic arm 502 is defined as a second tightening elastic arm. A limiting boss 106 is disposed on the front housing 1. Tightening attachment is implemented through cooperation between the tightening elastic arm 502 and the limiting boss 106 of the front housing 1, so that the outer cap 4 is closely attached to the front housing 1, to ensure that the cap is fully closed. In addition, in a cap-opening process of the outer cap 4, that is, in a process in which the outer cap 4 rotates from the first position to the second position, an initial stage is a cap-opening idle stroke. Rotation of the outer cap 4 needs to overcome resistance exerted by the limiting boss 106 on the tightening elastic arm 502, so that the tightening elastic arm 502 can pass over the limiting boss 106 to facilitate rotation of the outer cap 4, so as to prevent accidental cap opening r due to non-human factors.

Further, referring to FIG. 5A, FIG. 6A, FIG. 7A, and FIG. 7B, a limiting groove 403 is further provided on a connecting portion 406 of the outer cap 4 that is provided with the arc-shaped rib 405, and a limiting protruding post 107 is further disposed on the front housing 1. When the outer cap 4 is fully closed, that is, when the outer cap 4 is at the first position, the limiting protruding post 107 is engaged in the limiting groove 403, to limit the outer cap 4 at the first position. Through cooperation between the limiting groove 403 and the limiting protruding post 107 of the front housing 1, tightening attachment between the outer cap 4 and the front housing 1 is implemented, to ensure good attachment between the outer cap 4 and the front housing 1, and prevent any gap caused by incomplete closure. In addition, at the initial stage of the cap-opening process of the outer cap 4, that is, in the cap-opening idle stroke of the outer cap 4, a force exerted by the limiting groove 403 on the limiting protruding post 107 also needs to be overcome, so that the limiting protruding post 107 can disengage from the limiting groove 403 to facilitate rotation of the outer cap 4, so as to further prevent accidental cap opening due to non-human factors.

It can be understood that, the limiting boss 106 and the tightening elastic arm 502 are disposed at a position corresponding to one connecting portion 406 of the outer cap 4, and the limiting groove 403 and the limiting protruding post 107 are disposed at a position corresponding to the other connecting portion 406, to perform cap-closing limitation on two opposite sides of the outer cap 4, so that when the outer cap 4 is at the first position, the two opposite sides of the outer cap 4 can be well attached to the front housing 1, to effectively avoid a problem that the outer cap 4 is incompletely closed and one side of the outer cap 4 is fully closed against the front housing 1 while a gap exists on the other side of the outer cap 4, thereby ensuring consistency of a cap-closing state.

In a cap-closing process of the outer cap 4, because a tip of a protruding rod 1704 of an airbag pressing member 17 is limited in a stop groove 1213, the tip of the protruding rod 1704 of the airbag pressing member 17 first needs to be driven to disengage from the stop groove 1213, that is, resistance exerted by the stop groove 1213 on the tip of the protruding rod 1704 of the airbag pressing member 17 needs to be overcome. In this process, the outer cap 4 undergoes a first cap-closing sub-idle stroke, and the outer cap 4 reversely rotates from 150 degrees to 140 degrees, requiring a relatively large torque. Preferably, in the first cap-closing sub-idle stroke, a torque of the outer cap 4 is 0.05 N•m. Rotation of a drive cam 12 enables the tip of the protruding rod 1704 of the airbag pressing member 17 to disengage from the stop groove 1213 and abut against a second curved surface segment 1212, to effectively prevent accidental cap closing due to non-human factors.

In a process in which the outer cap 4 reversely rotates from 140 degrees to 87.5 degrees, the outer cap 4 drives the drive cam 12 to reversely rotate to 105 degrees. In this process, an annular boss 1207 of the drive cam 12 does not contact a boss 905 of the powder metering wheel 9, and the powder metering wheel 9 does not rotate. The outer cap 4 undergoes a second cap-closing sub-idle stroke, with only the second drive cam 12 reversely rotating and resetting. The tip of the protruding rod 1704 of the airbag pressing member 17 abuts against the second curved surface segment 1212. Because the second curved surface is a circular arc surface, the airbag pressing member 17 does not move in this process and remains at a sixth position. In this process, the powder metering wheel 9 does not need to be driven to rotate, and the airbag pressing member 17 does not need to be driven to reset. A torque required for the outer cap 4 in the second cap-closing sub-idle stroke is relatively small and is a constant torque. Preferably, the torque of the outer cap 4 in the second cap-closing sub-idle stroke is 0 N•m, to accelerate the cap-closing process and enhance smoothness of cap closing.

When the outer cap 4 reversely rotates to 87.5 degrees, the annular boss 1207 of the drive cam 12 starts to contact the boss 905 of the powder metering wheel 9. In a process in which the outer cap 4 reversely rotates from 87.5 degrees to 62.5 degrees, the outer cap 4 drives the drive cam 12 to reversely rotate from 105 degrees to 75 degrees. In this process, the outer cap 4 undergoes a first cap-closing sub-load stroke, the annular boss 1207 of the drive cam 12 engages with the boss 905 of the powder metering wheel 9 and drives the powder metering wheel 9 to reversely rotate, and the powder metering wheel 9 starts to rotate from a fourth position toward a third position. In this process, only the powder metering wheel 9 is resetting, the tip of the protruding rod 1704 of the airbag pressing member 17 still abuts against the second curved surface segment 1212, and the airbag pressing member 17 remains at the sixth position. Because the outer cap 4 needs to drive the powder metering wheel 9 to reversely rotate and reset in the first cap-closing sub-load stroke, a relatively large constant torque is required. Preferably, the torque of the outer cap 4 in the first cap-closing sub-load stroke is 0.1 N•m, to ensure that the powder metering wheel 9 can be driven to reversely rotate.

Further, in the cap-closing process of the outer cap 4, in a preferred implementation, in a process in which the outer cap 4 reversely rotates from 62.5 degrees to 0 degrees, the drive cam 12 reversely rotates from 75 degrees to 0 degrees. Through cooperation between a first curved surface segment 1211 of a cam curved surface 1202 on the drive cam 12 and the arc surface 1703 of the airbag pressing member 17, when the drive cam 12 reversely rotates, the first curved surface segment 1211 of the cam curved surface 1202 continuously pushes up the airbag pressing member 17 until the arc surface 1703 of the airbag pressing member 17 falls into an arc-shaped groove 1201 of the drive cam 12, to complete reset of the air compressing mechanism.

Referring to FIG. 21A to FIG. 21F, specifically, in the cap-closing process of the outer cap 4, in a preferred implementation, in a process in which the outer cap 4 reversely rotates from 56 degrees to 25 degrees, the drive cam 12 reversely rotates from 68 degrees to 30 degrees. In this process, a drive elastic arm 910 on the powder metering wheel 9 cooperates with a wedge-shaped post 1005 of the dose protection plate 10, to drive the dose protection plate 10 to rotate and reset.

Specifically, in a process in which the outer cap 4 reversely rotates from 87.5 degrees to 56 degrees, referring to FIG. 21A and FIG. 21C, the drive elastic arm 910 on the powder metering wheel 9 does not contact the wedge-shaped post 1005 of the dose protection plate 10, and in this process, the dose protection plate 10 does not reversely rotate. Referring to FIG. 21B and FIG. 21D, when the outer cap 4 reversely rotates to 56 degrees, the drive elastic arm 910 on the powder metering wheel 9 starts to contact the wedge-shaped post 1005 of the dose protection plate 10. In a process in which the outer cap 4 reversely rotates from 56 degrees to 25 degrees, the annular boss 1207 of the drive cam 12 contacts the boss 905 of the powder metering wheel 9. Reverse rotation of the drive cam 12 drives the powder metering wheel 9 to reversely rotate, which in turn causes a force exerted by the drive elastic arm 910 of the powder metering wheel 9 on the wedge-shaped post 1005 of the dose protection plate 10 to drive the dose protection plate 10 to reversely rotate by 38 degrees, thereby implementing reset of the dose protection plate 10. In a process in which the outer cap 4 reversely rotates from 8 degrees to 0 degrees, the dose protection plate 10 has already completed resetting and does not rotate. In this process, only the powder metering wheel 9 is still reversely rotating. Referring to FIG. 21E and FIG. 21F, the drive elastic arm 910 on the powder metering wheel 9 passes over the wedge-shaped post 1005 of the dose protection plate 10. When the outer cap 4 reversely rotates to 0 degrees, the powder metering wheel 9 fully resets, the limiting protruding post 107 is engaged in the limiting groove 403, and the tightening elastic arm 502 acts on the limiting boss 106, to ensure that the outer cap 4 is fully closed.

Before the outer cap 4 is fully closed, when the outer cap 4 reversely rotates to 12.5 degrees, the drive cam 12 reversely rotates to 15 degrees, a rib 1208 on the drive cam 12 disengages from a drive arm of a resetting torsion spring 15, and the drive arm of the resetting torsion spring 15 acts again on a cylinder 1102 on an air inlet baffle 11, to provide a resetting force for the air inlet baffle 11. Under the action of a resetting pressing force exerted by the resetting torsion spring 15 on the air inlet baffle 11, the air inlet baffle 11 reversely rotates to complete resetting, the pressing arc surface 1002 of the dose protection plate 10 acts again on a circular-arc-shaped groove surface 1107 of the air inlet baffle 11 to implement concentric arc surface pressing, and the dose protection plate 10 of the inhalation trigger mechanism completes resetting. In a process in which the outer cap 4 reversely rotates from 8 degrees to 0 degrees, the drive elastic arm 910 on the drive powder metering wheel 9 passes over the wedge-shaped post 1005 on the dose protection plate 10. In this process, the outer cap 4 undergoes a third cap-closing sub-load stroke, the drive powder metering wheel 9 continues to reversely rotate, while the dose protection plate 10 has completed resetting and does not reversely rotate. After the drive elastic arm 910 on the powder metering wheel 9 passes over the wedge-shaped post 1005 on the dose protection plate 10, the drive elastic arm 910 does not exert a force on the wedge-shaped post 1005, and under the action of the drive torsion spring 16, the pressing arc surface 1002 of the dose protection plate 10 acts again on the circular-arc-shaped groove surface 1107 of the air inlet baffle 11 to implement concentric arc surface pressing. This ensures that the powder metering wheel 9 completes resetting after the inhalation trigger mechanism completes resetting, to avoid a case of incomplete resetting of the inhalation trigger mechanism.

In a process in which the outer cap 4 reversely rotates from 62.5 degrees to 8 degrees, reverse resetting of the powder metering wheel 9 and upward pushing resetting of the airbag pressing member 17 need to be simultaneously performed. In this process, the outer cap 4 undergoes a second cap-closing sub-load stroke, which requires a relatively large torque, and the torque of the second cap-closing sub-load stroke gradually increases. Preferably, in the second cap-closing sub-load stroke, the torque of the outer cap 4 gradually increases from 0.10 N•m to 0.20 N•m, to ensure that the powder metering wheel 9 and the airbag pressing member 17 can continuously reset. When the outer cap 4 rotates to 8 degrees, the airbag pressing member 17 completes resetting, that is, the airbag pressing member 17 resets to the fifth position, and the tip of the protruding rod 1704 of the airbag pressing member 17 re-enters the arc-shaped groove 1201, to perform limitation on the airbag pressing member 17. In a process in which the outer cap 4 reversely rotates from 8 degrees to 0 degrees, the outer cap 4 undergoes a third cap-closing sub-load stroke. In this process, resistance encountered by the powder metering wheel 9 in passing over the wedge-shaped post 1005 needs to be overcome. Preferably, a torque of the outer cap 4 in the third cap-closing sub-load stroke is 0.1 N•m until cap closing is completed.

Specifically, in the first cap-closing sub-load stroke and the second cap-closing sub-load stroke, the outer cap 4 drives, via the powder metering wheel 9, the dose protection plate 10 to reset to a position beyond an inlet 704 of an inhalation channel 706, and compresses the drive torsion spring 16. In addition, the resetting torsion spring 15 drives the air inlet baffle 11 to reset and rotate and close an airflow channel 708. In the third cap-closing sub-load stroke of the outer cap 4, the powder metering wheel 9 disengages from the dose protection plate 10. Specifically, the drive elastic arm 910 of the powder metering wheel 9 disengages from the wedge-shaped post 1005 of the dose protection plate 10, and the drive torsion spring 16 drives the dose protection plate 10 to rotate, so that a blocking portion 1004 of the dose protection plate 10 rotates to the inlet 704 of the inhalation channel 706, and is limited at a position of the inlet 704 of the inhalation channel 706 by the air inlet baffle 11.

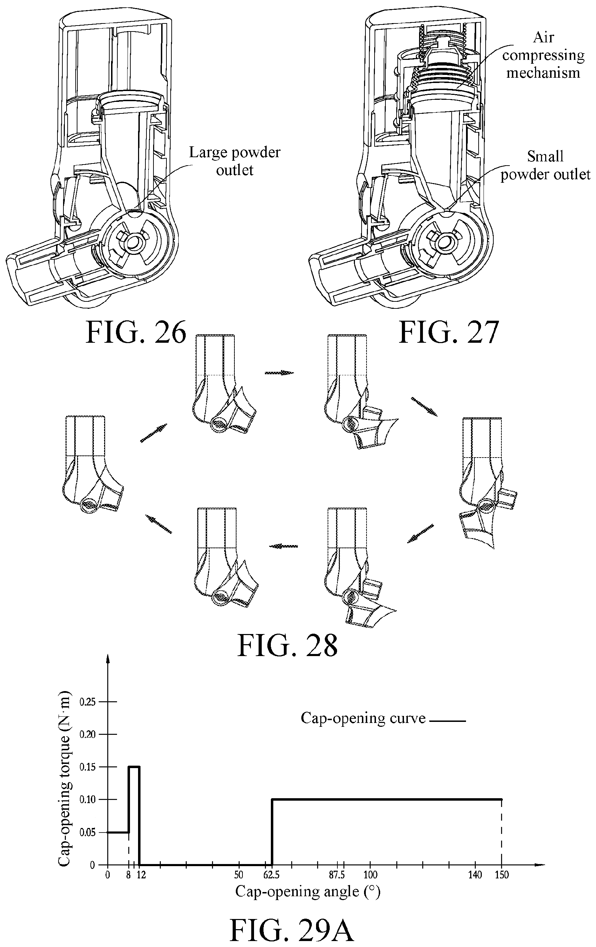

Refer to FIG. 26 and FIG. 27, FIG. 26 is a schematic cross-sectional view of another embodiment of a powder inhaler according to this application; and FIG. 27 is a schematic cross-sectional view of still another embodiment of a powder inhaler according to this application.

In the powder inhaler shown in FIG. 1, powder filling is performed by using a solution of an air compressing mechanism and a large-sized powder outlet 713. In this embodiment, referring to FIG. 9C and FIG. 9D, a cross-sectional area of the powder outlet 713 of the powder container 7 is greater than a cross-sectional area of the dose cup 902 on the powder metering wheel 9. A relatively large size of the powder outlet 713 of the powder container 7 enables more efficient implementation of an air compressing and powder filling processes. In another embodiment, air compressing may not be performed in the manner shown in FIG. 9C.

For example, as shown in FIG. 26, in another embodiment, an air compressing mechanism may not be disposed in the powder inhaler. Instead, a large-sized powder outlet 713 is directly used, so that a cross-sectional area of the powder outlet 713 of the powder container 7 is greater than a cross-sectional area of the dose cup 902 on the powder metering wheel 9. Because in the powder inhaler provided in the embodiments of this application, an axis of the housing assembly is always parallel to a vertical direction in a cap opening and closing process of the outer cap 4, a powder outlet 713 at a bottom portion of the storage cavity 715 is always located directly above the dose cup 902 of the powder metering wheel 9 along the vertical direction in a powder filling process. Consequently, by directly relying on gravity of the powder in the storage cavity 715 of the powder container 7, powder in the powder container 7 can also be filled into the dose cup 902 of the powder metering wheel 9, thereby implementing powder filling.

Alternatively, as shown in FIG. 27, in still another embodiment, an air compressing mechanism may also be disposed in the powder inhaler. However, a powder outlet 713 of the powder container 7 is provided as a small-sized powder outlet 713. Specifically, a cross-sectional area of the powder outlet 713 of the powder container 7 is less than a cross-sectional area of the dose cup 902 on the powder metering wheel 9. A cross-sectional shape of the powder outlet 713 may be a circle or any other shape, and a diameter of the powder outlet 713 ranges from 1 mm to 3 mm, so as to compact and fill the powder in the powder container 7 into the dose cup 902 on the powder metering wheel 9 via the air compressing mechanism, thereby implementing powder filling. A specific setting manner for air compressing and powder filling may be designed or selected as needed. This is not limited in this application.

The following describes a process from cap opening to cap closing, namely, a specific running state of a powder inhaler in an entire process in which the outer cap 4 rotates from a first position (0 degrees) to a second position (150 degrees), a user inhales, and then the outer cap 4 rotates from the second position (150 degrees) to the first position (0 degrees).

### (1) Cap-opening process

In the cap-opening process of the outer cap 4, the process in which the outer cap 4 rotates from the first position (0 degrees) to the second position (150 degrees) includes, in chronological order, a cap-opening idle stroke, an air compressing process, and a powder delivery process.

### (1) Cap-opening idle stroke

In a process of the cap-opening idle stroke, the outer cap 4 rotates from 0 degrees to 12 degrees, where a process in which the outer cap 4 rotates from 0 degrees to 8 degrees is to prevent the outer cap 4 from being opened due to non-human factors, and a process in which the outer cap rotates 4 from 8 degrees to 12 degrees is to prevent accidental cap opening. When the outer cap 4 is at the first position, namely, 0 degrees, the outer cap 4 blocks the suction nozzle 101.

### (2) Air compressing process

In the air compressing process, the outer cap 4 rotates from 12 degrees to 62.5 degrees, where in a process in which the outer cap 4 rotates from 12 degrees to 55 degrees, an air compressing function is implemented, so that the powder in the storage cavity 715 of the powder container 7 is compacted and filled into the dose cup 902 of the powder metering wheel 9; when the outer cap 4 rotates to 55 degrees, the pressure relief hole 1702 and the vent hole 709 are at a critical point of communication; and in a process in which the outer cap 4 rotates from 55 degrees to 62.5 degrees, the pressure relief hole 1702 and the vent hole 709 are in communication, to implement a pressure relief function, releasing compressed air in the storage cavity 715 of the powder container 7 to atmospheric pressure, so as to avoid powder leakage when the powder metering wheel 9 rotates. In this process, the outer cap 4 can be instantaneously opened during cap opening, so that the air compressing mechanism can rapidly compress air, to enhance an air compressing effect.

### (3) Powder delivery process

In the powder delivery process, the outer cap 4 rotates from 62.5 degrees to 150 degrees, and rotation of the outer cap 4 drives the drive cam 12 to rotate. The annular boss 1207 of the drive cam 12 contacts and cooperates with the boss 905 of the powder metering wheel 9. Rotation of the drive cam 12 drives the powder metering wheel 9 to rotate by 105 degrees, so that the powder metering wheel 9 rotates from the third position to the fourth position, and the dose cup 902 of the powder metering wheel 9 rotates from a position corresponding to the powder outlet 713 of the storage cavity 715 of the powder container 7 to a position corresponding to the inlet 704 of the inhalation channel 706. When the outer cap 4 rotates to the second position, an arc-shaped rib 405 on the outer cap 4 cooperates with a sound elastic arm 108 on a front housing 1, to provide an audible prompt indicating that the outer cap 4 is fully opened.

### (2) Inhalation trigger process

After the powder delivery process is completed, the dose cup 902 of the powder metering wheel 9 is delivered to the position corresponding to the inlet 704 of the inhalation channel 706, the blocking portion 1004 of the dose protection plate 10 blocks the dose cup 902 of the powder metering wheel 9, the outer cap 4 is at the second position (150 degrees), and the outlet 102 of the suction nozzle 101 is exposed. When a user inhales at the position of the outlet 102 of the suction nozzle 101, and when an airflow rate during user inhalation exceeds an operating threshold (20 L/min to 25 L/min) and negative pressure in the inhalation channel 706 exceeds a threshold, the inhalation trigger mechanism is triggered to act, the air inlet baffle 11 rotates, the pressing arc surface 1002 of the dose protection plate 10 disengages from the circular-arc-shaped groove surface 1107 of the air inlet baffle 11, the dose protection plate 10 rotates by 38 degrees under driving of the drive torsion spring 16, and the blocking portion 1004 of the dose protection plate 10 deviates and unblocks the dose cup 902 of the powder metering wheel 9. The dose cup 902 is exposed at the inlet 704 of the inhalation channel 706, the dose cup 902 is in communication with the inhalation channel 706, and the powder in the dose cup 902 is exposed to an inhalation airflow of the user and is carried away, to complete the inhalation trigger process.

### (3) Cap-closing process

The cap-closing process is performed after completion of the inhalation trigger process. In the cap-closing process, the outer cap 4 reversely rotates and resets from the second position (150 degrees) to the first position (0 degrees) again, and the outer cap 4 drives the air compressing mechanism, the powder metering wheel 9, the dose protection plate 10, and the air inlet baffle 11 to reset. Specifically, the cap-closing process includes a cap-closing idle stroke and a functional mechanism resetting process.

### (1) Cap-closing idle stroke

In the cap-closing idle stroke, the outer cap 4 reversely rotates from 150 degrees to 87.5 degrees (that is, the outer cap 4 reversely rotates by 62.5 degrees), where a process in which the outer cap 4 rotates from 150 degrees to 140 degrees is to avoid triggering cap closing due to non-human factors, and in a later stage of the cap-closing idle stroke, the outer cap 4 rotates from 140 degrees to 87.5 degrees.

### (2) Resetting process of the functional mechanism

In the resetting process of the functional mechanism, the outer cap 4 reversely rotates from 87.5 degrees to 0 degrees. In this process, the powder metering wheel 9 continuously reversely rotates and resets from the fourth position to the third position. In a process in which the outer cap 4 reversely rotates from 87.5 degrees to 62.5 degrees, only the powder metering wheel 9 reversely rotates; and in a process in which the outer cap 4 reversely rotates from 62.5 degrees to 8 degrees, the powder metering wheel 9 reversely rotates, and the airbag pressing member 17 continuously pushes upward to enable the air compressing mechanism to reset. In a process in which the outer cap 4 reversely rotates from 56 degrees to 25 degrees, the drive elastic arm 910 on the powder metering wheel 9 cooperates with the wedge-shaped post 1005 of the dose protection plate 10, to drive the dose protection plate 10 to rotate and reset, the pressing arc surface 1002 of the dose protection plate 10 acts again on the circular-arc-shaped groove surface 1107 of the air inlet baffle 11 to implement concentric arc surface pressing, and the inhalation trigger mechanism completes resetting; and in a process in which the outer cap 4 reversely rotates from 8 degrees to 0 degrees, the air compressing mechanism has already reset. In this case, rotational resistance of the powder metering wheel 9 is overcome in the cap-closing stroke, and reset of the powder metering wheel 9 is completed when the outer cap 4 is at the first position (0 degrees). When the outer cap 4 resets to the first position, an arc-shaped rib 405 on the outer cap 4 cooperates with a sound elastic arm 108 on a front housing 1, to provide an audible prompt indicating that the cap is fully closed, so as to prompt that the outer cap 4 is cap is fully closed.

It can be learnt from the above that, the outer cap 4 has different functions at different positions during cap opening and closing, such as an idle stroke or driving different functional mechanisms to operate. Based on different functions of the outer cap 4 at different positions during cap opening and closing, in this application, torques of the outer cap 4 in different strokes are designed during cap opening and closing, so that a cap opening and closing process of the powder inhaler better conforms to user habits.

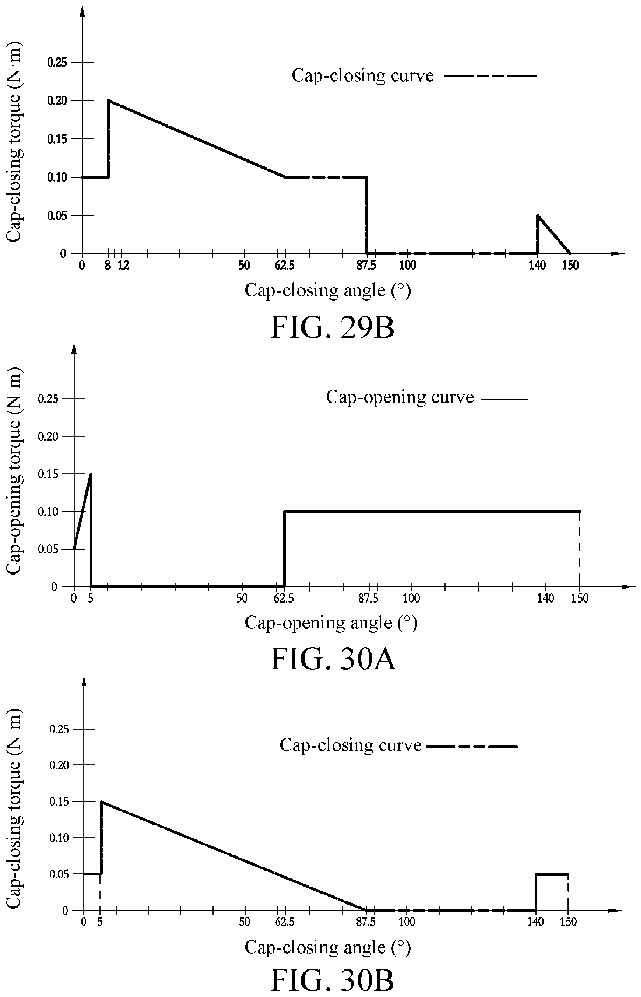

Refer to FIG. 28 to FIG. 30B. FIG. 28 is a schematic diagram of a cycle of a cap opening and closing process of the powder inhaler provided in FIG. 1; FIG. 29A is a schematic diagram of a curve of a cap-opening angle and a torque in an implementation of a cap-opening process of the powder inhaler provided in FIG. 1; FIG. 29B is a schematic diagram of a curve of a cap-closing angle and a torque in an implementation of a cap-closing process of the powder inhaler provided in FIG. 1; FIG. 30A is a schematic diagram of a curve of a cap-opening angle and a torque in another implementation of a cap-opening process of the powder inhaler provided in FIG. 1; and FIG. 30B is a schematic diagram of a curve of a cap-closing angle and a torque in another implementation of a cap-closing process of the powder inhaler provided in FIG. 1.

### (1) Cap-opening process

Referring to FIG. 28, FIG. 29A, and FIG. 29B, in some implementations, a stroke (namely, the cap-opening process) of the outer cap 4 rotating from the first position to the second position includes a cap-opening idle stroke and a cap-opening load stroke following the cap-opening idle stroke. For example, the stroke of the outer cap 4 rotating from the first position to the second position includes only the cap-opening idle stroke and the cap-opening load stroke following the cap-opening idle stroke.

In the cap-opening idle stroke, the outer cap 4 does not trigger an action of the functional mechanism. In the cap-opening load stroke, the outer cap 4 triggers the functional mechanism to deliver powder to the inhalation channel 706, where a maximum torque of the outer cap 4 in the cap-opening idle stroke is greater than a maximum torque in the cap-opening load stroke, which can avoid accidental cap opening due to non-human factors in the cap-opening idle stroke, and also ensures rapid or smooth operation at different stages in the cap-opening load stroke. Preferably, a torque of the outer cap 4 in the cap-opening idle stroke is greater than or equal to 0.05 N•m and less than or equal to 0.3 N•m, and a torque of the outer cap 4 in the cap-opening load stroke is greater than or equal to 0 N•m and less than or equal to 0.15 N•m.

An angle of the outer cap 4 at the first position is defined as 0 degrees, and an angle of the outer cap 4 at the second position is greater than or equal to 120 degrees and less than or equal to 180 degrees. In a preferred implementation, an angle of the outer cap 4 at the second position is 150 degrees. A critical angle between the cap-opening idle stroke and the cap-opening load stroke is greater than or equal to 10 degrees and less than or equal to 15 degrees. In a preferred implementation, the critical angle between the cap-opening idle stroke and the cap-opening load stroke is specifically 12 degrees, that is, that the outer cap 4 rotates from 0 degrees to 12 degrees is the cap-opening idle stroke, and that the outer cap 4 rotates from 12 degrees to 150 degrees is the cap-opening load stroke.

### (1) Cap-opening idle stroke

The cap-opening idle stroke includes a first cap-opening sub-idle stroke and a second cap-opening sub-idle stroke following the first cap-opening sub-idle stroke, where a critical angle between the first cap-opening sub-idle stroke and the second cap-opening sub-idle stroke is greater than or equal to 6 degrees and less than or equal to 10 degrees. As shown in FIG. 25, in a preferred implementation, a critical angle between the first cap-opening sub-idle stroke and the second cap-opening sub-idle stroke is 8 degrees. That is, that the outer cap 4 rotates from 0 degrees to 8 degrees is the first cap-opening sub-idle stroke, and that the outer cap 4 rotates from 8 degrees to 12 degrees is the second cap-opening sub-idle stroke.

A torque of the outer cap 4 in the first cap-opening sub-idle stroke is greater than or equal to 0.02 N•m and less than or equal to 0.08 N•m, and a torque of the outer cap 4 in the second cap-opening sub-idle stroke is greater than or equal to 0.1 N•m and less than or equal to 0.2 N•m. Preferably, the torque of the outer cap 4 in the first cap-opening sub-idle stroke is 0.05 N•m, that is, an initial torque set for the outer cap 4 rotating from 0 degrees to 8 degrees is 0.05 N•m, which can effectively avoid cap opening due to non-human factors. The torque of the outer cap 4 in the second cap-opening sub-idle stroke is 0.15 N•m, that is, the torque set for the outer cap 4 rotating from 8 degrees to 12 degrees is 0.15 N•m. Setting relatively large cap-opening resistance for the outer cap 4 rotating from 8 degrees to 12 degrees can avoid accidental cap opening.

### (2) Cap-opening load stroke

The cap-opening load stroke includes a first cap-opening sub-load stroke and a second cap-opening sub-load stroke following the first cap-opening sub-load stroke, where in the first cap-opening sub-load stroke, the outer cap 4 triggers the air compressing mechanism to press powder in the storage cavity 715 into the dose cup 902, and in the second cap-opening sub-load stroke, the outer cap 4 triggers the dose cup 902 to deliver the powder to the inhalation channel 706.

A critical angle between the first cap-opening sub-load stroke and the second cap-opening sub-load stroke is greater than or equal to 60 degrees and less than or equal to 65 degrees. In a preferred implementation, the critical angle between the first cap-opening sub-load stroke and the second cap-opening sub-load stroke is 62.5 degrees. That is, that the outer cap 4 rotates from 12 degrees to 62.5 degrees is the first cap-opening sub-load stroke, and that the outer cap 4 rotates from 62.5 degrees to 150 degrees is the second cap-opening sub-load stroke.

A torque of the outer cap 4 in the first cap-opening sub-load stroke is constant, and is greater than or equal to 0 N•m and less than or equal to 0.05 N•m. Preferably, a torque of the outer cap 4 in the first cap-opening sub-load stroke is 0 N•m, that is, a torque set for the outer cap 4 rotating from 12 degrees to 62.5 degrees is 0 N•m. An air compressing process is performed in the first cap-opening sub-load stroke, and setting a torque in this angular range to 0 N•m enables instantaneous opening during cap opening, causing the air compressing mechanism to rapidly compress air, thereby enhancing an air compressing effect.

A torque of the outer cap 4 in the second cap-opening sub-load stroke is constant, and is greater than or equal to 0.05 N•m and less than or equal to 0.15 N•m. Preferably, a torque of the outer cap 4 in the second cap-opening sub-load stroke is 0.1 N•m, that is, a torque set for the outer cap 4 rotating from 62.5 degrees to 150 degrees is 0.1 N•m. In the second cap-opening sub-load stroke, the outer cap 4 performs a powder delivery process. At this stage, the outer cap 4 is opened smoothly and at a constant speed, and the torque set in the second cap-opening sub-load stroke is a constant torque of 0.1 N•m without any abrupt torque change until cap opening is completed, thereby ensuring a powder delivery effect.

### (2) Cap-closing process

Referring to FIG. 28, FIG. 29A, and FIG. 29B, in some implementations, a stroke (namely, the cap-closing process) of the outer cap 4 rotating and resetting from the second position to the first position includes a cap-closing idle stroke and a cap-closing load stroke following the cap-closing idle stroke, where in the cap-closing load stroke, the outer cap 4 triggers the functional mechanism to reset.

An angle of the outer cap 4 at the first position is defined as 0 degrees, and an angle of the outer cap 4 at the second position is greater than or equal to 120 degrees and less than or equal to 180 degrees. In a preferred implementation, an angle of the outer cap 4 at the second position is 150 degrees. In some implementations, a critical angle between the cap-closing idle stroke and the cap-closing load stroke is greater than or equal to 80 degrees and less than or equal to 95 degrees. Preferably, a critical angle between the cap-closing idle stroke and the cap-closing load stroke is 87.5 degrees. That is, that the outer cap 4 rotates from 150 degrees to 87.5 degrees is a cap-closing idle stroke, and that the outer cap 4 rotates from 87.5 degrees to 0 degrees is a cap-closing load stroke.

### (1) Cap-closing idle stroke

A maximum torque of the outer cap 4 in the cap-closing idle stroke is greater than or equal to 0.03 N•m and less than or equal to 0.07 N•m. Preferably, the maximum torque of the outer cap 4 in the cap-closing idle stroke can avoid accidental cap closing due to non-human factors.

In some implementations, the cap-closing idle stroke includes a first cap-closing sub-idle stroke and a second cap-closing sub-idle stroke following the first cap-closing sub-idle stroke, and a critical angle between the first cap-closing sub-idle stroke and the second cap-closing sub-idle stroke is greater than or equal to 135 degrees and less than or equal to 145 degrees. Preferably, a critical angle between the first cap-closing sub-idle stroke and the second cap-closing sub-idle stroke is 140 degrees. That is, that the outer cap 4 rotates from 150 degrees to 140 degrees is a first cap-closing sub-idle stroke, and that the outer cap 4 rotates from 140 degrees to 87.5 degrees is a second cap-closing sub-idle stroke.

A torque of the outer cap 4 in the first cap-closing sub-idle stroke is constant, and is greater than or equal to 0.03 N•m and less than or equal to 0.07 N•m. Preferably, the torque of the outer cap 4 in the first cap-closing sub-idle stroke is 0.05 N•m, that is, the torque of the outer cap 4 rotating from 150 degrees to 140 degrees is 0.05 N•m, which can avoid triggering cap closing due to non-human factors.

A torque of the outer cap 4 in the second cap-closing sub-idle stroke is constant, and is less than or equal to 0.02 N•m. Preferably, the torque of the outer cap 4 in the second cap-closing sub-idle stroke is 0 N•m, to accelerate the cap-closing process and enhance smoothness of cap closing.

### (2) Cap-closing load stroke

A maximum torque of the outer cap 4 in the cap-closing load stroke is greater than 0.05 N•m and less than or equal to 0.3 N•m. Specifically, the cap-closing load stroke includes a first cap-closing sub-load stroke, a second cap-closing sub-load stroke, and a third cap-closing sub-load stroke that are set in chronological order. In the first cap-closing sub-load stroke, the outer cap 4 triggers only the powder metering wheel 9 to reset and rotate. In the second cap-closing sub-load stroke, the outer cap 4 continues to trigger the powder metering wheel 9 to reset and rotate and triggers the air compressing mechanism to complete resetting. In the third cap-closing sub-load stroke, the outer cap 4 triggers only the powder metering wheel 9 to reset and rotate, and triggers the powder metering wheel 9 to reset and rotate to the first position. In the cap-closing load stroke, the outer cap 4 further triggers the dose protection plate 10 and the air inlet baffle 11 to complete resetting.

A critical angle between the first cap-closing sub-load stroke and the second cap-closing sub-load stroke is greater than or equal to 60 degrees and less than or equal to 65 degrees. Preferably, a critical angle between the first cap-closing sub-load stroke and the second cap-closing sub-load stroke is 62.5 degrees, that is, that the outer cap 4 rotates from 87.5 degrees to 62.5 degrees is the first cap-closing sub-load stroke. A critical angle between the second cap-closing sub-load stroke and the third cap-closing sub-load stroke is greater than or equal to 6 degrees and less than or equal to 10 degrees. Preferably, a critical angle between the second cap-closing sub-load stroke and the third cap-closing sub-load stroke is 8 degrees, that is, that the outer cap 4 rotates from 62.5 degrees to 8 degrees is the second cap-closing sub-load stroke, and that the outer cap 4 rotates from 8 degrees to 0 degrees is the third cap-closing sub-load stroke.

A torque of the outer cap 4 in the first cap-closing sub-load stroke is constant, and is greater than or equal to 0.05 N•m and less than or equal to 0.15 N•m. Preferably, the torque of the outer cap 4 in the first cap-closing sub-load stroke is 0.1 N•m, that is, a torque of the outer cap 4 rotating from 87.5 degrees to 62.5 degrees is 0.1 N•m, and only the powder metering wheel 9 rotates in the first cap-closing sub-load stroke.

A torque of the outer cap 4 in the second cap-closing sub-load stroke gradually increases, and a maximum value of the torque is greater than or equal to 0.15 N•m and less than or equal to 0.3 N•m. Preferably, the torque of the outer cap 4 in the second cap-closing sub-load stroke gradually increases from 0.10 N•m to 0.20 N•m, that is, a torque of the outer cap 4 rotating from 62.5 degrees to 8 degrees gradually increases from 0.10 N•m to 0.20 N•m. In the second cap-closing sub-load stroke, the powder metering wheel 9 is still rotating, and the airbag pressing member 17 is continuously pushed upward to reset.

A torque of the outer cap 4 in the third cap-closing sub-load stroke is constant, and is greater than or equal to 0.05 N•m and less than or equal to 0.15 N•m. Preferably, the torque of the outer cap 4 in the third cap-closing sub-load stroke is 0.1 N•m, that is, a torque of the outer cap 4 rotating from 8 degrees to 0 degrees is 0.1 N•m. In the third cap-closing sub-load stroke, the airbag pressing member 17 has fully reset. In this case, rotational resistance of the powder metering wheel 9 is overcome in the cap-closing stroke until cap closing is completed.

Referring to FIG. 30A and FIG. 30B, in another implementation, a cap-opening process and a cap-closing process of the outer cap 4 of the powder inhaler may also be set according to a correspondence between a cap opening and closing angle and a torque as shown in FIG. 30A and FIG. 30B. In this implementation, torque variation during cap opening and closing exhibits a ramp curve with fewer abrupt torque changes, which is more user-friendly.

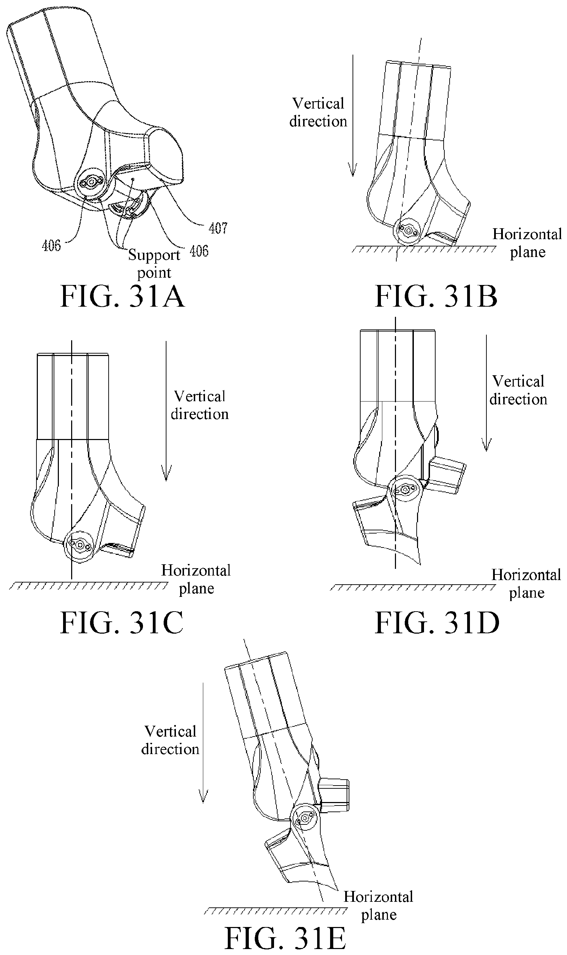

Refer to FIG. 31A to FIG. 31E. FIG. 31A is a schematic diagram of a bottom view structure of the powder inhaler that is provided in FIG. 1 and that is viewed from an angle; FIG. 31B is a schematic diagram of the powder inhaler that is provided in FIG. 31A and that is in a state of being placed on a horizontal surface; FIG. 31C is a schematic diagram of the powder inhaler that is provided in FIG. 31A and that is in a handheld state; FIG. 31D is a schematic diagram of the powder inhaler that is provided in FIG. 31A with a cap opened and that is in a handheld state; and FIG. 31E is a schematic diagram of the powder inhaler that is provided in FIG. 31A and that is in an oral inhalation state.

Referring to FIG. 1, FIG. 5A, FIG. 5B, FIG. 31A, and FIG. 31B, the outer cap 4 of the powder inhaler includes two connecting portions 406 disposed opposite to each other along a first direction and a free end 407 located on one side of each of the two connecting portions 406 along a second direction. The first direction intersects the second direction, the two connecting portions 406 are respectively rotatably connected to two opposite sides of a bottom end of the housing assembly and protrude from the bottom end of the housing assembly, and the free end 407 and the two connecting portions 406 are configured to support the housing assembly, so that the powder inhaler can be stably placed on a horizontal surface. The three support points formed by two connecting portions 406 and one free end 407 may implement stable placement, to prevent the powder inhaler from tipping over due to unstable placement.

The present invention ensures that during use and storage of the device, the powder container 7 is always located above the powder metering wheel 9, to prevent a problem of powder variation caused by frequent movement of the powder in the powder container 7 due to excessive changes in a position state of the device, such as detachment of active ingredients of the powder from carriers, reduction in a powder particle size, and accumulation of small particles at the bottom, thereby ensuring that the powder in the powder container 7 remains in a relatively stable state throughout an entire service life cycle of the powder inhaler.

For example, as shown in FIG. 31B, when the bottom end of the powder inhaler is placed on the horizontal plane, an inclination angle of an axis of the housing assembly relative to a vertical direction is greater than or equal to 0 degrees and less than or equal to 15 degrees. Preferably, when the bottom end of the powder inhaler is placed on the horizontal plane, the inclination angle of the axis of the housing assembly relative to the vertical direction is 6 degrees, which can further ensure minimal changes in the position state of the device, and ensure that when the powder inhaler is placed on the horizontal plane (that is, in a storage state), the powder in the powder container 7 always remains in a relatively stable state.

For example, as shown in FIG. 31C and FIG. 31D, when the powder inhaler is handheld during cap opening and closing, the device is in a vertical state, and the axis of the housing assembly of the powder inhaler is parallel to the vertical direction, which is conducive to cap opening and closing and also minimizes changes in the position state of the device, thereby ensuring that the powder in the powder container 7 remains in a relatively stable state during cap opening and closing.

For example, as shown in FIG. 31E, during oral inhalation by a user, an inclination angle of an axis of the housing assembly of the powder inhaler relative to a vertical direction is greater than or equal to 0 degrees and less than or equal to 15 degrees. Preferably, during oral inhalation by the user, the inclination angle of the axis of the housing assembly of the powder inhaler relative to the vertical direction is 15 degrees, which not only ensures a relatively small change in a position state of the powder inhaler during oral inhalation and enables the powder in the powder container 7 to be in a relatively stable state, but also ensures convenience of inhalation by the user at the outlet 102 of the suction nozzle 101.

The foregoing descriptions are merely embodiments of this application, and the protection scope of this application is not limited thereto. All equivalent structure or process changes made according to the content of this specification and accompanying drawings in this application or by directly or indirectly applying this application in other related technical fields shall fall within the protection scope of this application.

## Claims

1. A powder inhaler, comprising:
a functional mechanism, comprising an inhalation channel;
a suction nozzle, in communication with the inhalation channel; and
an outer cap, being linked to and cooperating with the functional mechanism, and being limited to rotate back and forth between a first position and a second position, wherein the outer cap blocks the suction nozzle when the outer cap is configured to be at the first position; and the outer cap unblocks the suction nozzle when the outer cap is configured to be at the second position, wherein
a stroke of the outer cap rotating from the first position to the second position comprises a cap-opening idle stroke and a cap-opening load stroke following the cap-opening idle stroke; the outer cap does not trigger an action of the functional mechanism in the cap-opening idle stroke; and the outer cap triggers, in the cap-opening load stroke, the functional mechanism to deliver powder to the inhalation channel; and/or
a stroke of the outer cap rotating and resetting from the second position to the first position comprises a cap-closing idle stroke and a cap-closing load stroke following the cap-closing idle stroke; the outer cap does not trigger an action of the functional mechanism in the cap-closing idle stroke; and the outer cap triggers, in the cap-closing load stroke, the functional mechanism to reset.

2. The powder inhaler of claim 1, wherein
the cap-opening idle stroke comprises a first cap-opening sub-idle stroke and a second cap-opening sub-idle stroke following the first cap-opening sub-idle stroke, wherein a torque in the second cap-opening sub-idle stroke is greater than a torque in the first cap-opening sub-idle stroke; and
preferably, a torque gradually increases or abruptly increases in a process of switching from the first cap-opening sub-idle stroke to the second cap-opening sub-idle stroke.

3. The powder inhaler of claim 1, wherein
the cap-opening load stroke comprises a first cap-opening sub-load stroke and a second cap-opening sub-load stroke following the first cap-opening sub-load stroke, wherein
a torque in the first cap-opening sub-load stroke is less than a torque in the second cap-opening sub-load stroke and/or the cap-opening idle stroke.

4. The powder inhaler of claim 1, wherein
the cap-closing idle stroke comprises a first cap-closing sub-idle stroke and a second cap-closing sub-idle stroke following the first cap-closing sub-idle stroke, wherein
a torque in the first cap-closing sub-idle stroke is greater than a torque in the second cap-closing sub-idle stroke; and
preferably, the torque in the first cap-closing sub-idle stroke is constant or gradually increases.

5. The powder inhaler of claim 1, wherein
the cap-closing load stroke comprises a first cap-closing sub-load stroke, a second cap-closing sub-load stroke, and a third cap-closing sub-load stroke that are set in chronological order, wherein
a torque in the second cap-closing sub-load stroke is greater than a torque in the first cap-closing sub-load stroke and/or the third cap-closing sub-load stroke;
preferably, the torque in the third cap-closing sub-load stroke is greater than or equal to the torque in the first cap-closing sub-load stroke; and
preferably, the torque in the second cap-closing sub-load stroke and/or the first cap-closing sub-load stroke gradually increases.

6. The powder inhaler of claim 1, wherein the functional mechanism comprises:
an air compressing mechanism; and
a powder delivery mechanism, comprising a storage cavity and a dose cup, wherein the storage cavity is configured to store the powder, and the storage cavity has a powder outlet; the cap-opening load stroke comprises a first cap-opening sub-load stroke and a second cap-opening sub-load stroke following the first cap-opening sub-load stroke; the outer cap triggers, in the first cap-opening sub-load stroke, the air compressing mechanism to press the powder from the storage cavity into the dose cup; and the outer cap drives, in the second cap-opening sub-load stroke, the dose cup to deliver the powder to the inhalation channel.

7. The powder inhaler of claim 6, wherein
the delivery mechanism comprises a powder container and a powder metering wheel; the powder container has the inhalation channel and the storage cavity; the powder metering wheel is rotatably connected to the powder container; the powder metering wheel comprises the dose cup; and
the outer cap triggers, in the first cap-opening sub-load stroke, the air compressing mechanism to first press air into the powder container, and then relieve pressure in the powder container, wherein
the powder metering wheel is capable of rotating back and forth between a third position and a fourth position; when the powder metering wheel is configured to be at the third position, the dose cup is disposed corresponding to the powder outlet of the storage cavity and is configured to receive the powder in the powder container; when the powder metering wheel is configured to be at the fourth position, the dose cup is disposed corresponding to an inlet of the inhalation channel; and the outer cap drives, in the second cap-opening sub-load stroke, the dose cup to rotate from the third position to the fourth position.

8. The powder inhaler of claim 7, further comprising:
an inhalation trigger mechanism, comprising a dose protection plate and an air inlet baffle that are linked to and cooperate with each other, a resetting torsion spring, and a drive torsion spring, wherein
the resetting torsion spring limits the air inlet baffle to an airflow channel; and the dose protection plate is limited by the air inlet baffle to the inlet of the inhalation channel and blocks the dose cup, wherein
the outer cap presses the resetting torsion spring in the second cap-opening sub-load stroke, and releases limitation of the resetting torsion spring on the air inlet baffle; and
when negative pressure of the airflow channel exceeds a threshold, the air inlet baffle rotates under an action of an air flow to release limitation on the dose protection plate, and the dose protection plate rotates to deviate under an action of the drive torsion spring and unblocks the dose cup of the powder metering wheel.

9. The powder inhaler of claim 1, wherein the functional mechanism comprises:
an air compressing mechanism; and
a powder delivery mechanism, comprising a powder container and a powder metering wheel, wherein the powder container has a storage cavity, and is configured to store the powder; and the powder metering wheel is rotatably connected to the powder container, wherein
the cap-closing load stroke comprises a first cap-closing sub-load stroke, a second cap-closing sub-load stroke, and a third cap-closing sub-load stroke that are set in chronological order; the outer cap triggers, in the first cap-closing sub-load stroke, the powder metering wheel to reset and rotate; the outer cap continues to trigger, in the second cap-closing sub-load stroke, the powder metering wheel to reset and rotate and triggers the air compressing mechanism to complete resetting; and the outer cap triggers, in the third cap-closing sub-load stroke, only the powder metering wheel to reset and rotate, and triggers the powder metering wheel to reset and rotate to the first position.

10. The powder inhaler of claim 9, further comprising:
an inhalation trigger mechanism, comprising a dose protection plate and an air inlet baffle that are linked to and cooperate with each other, a resetting torsion spring, and a drive torsion spring, wherein the resetting torsion spring limits the air inlet baffle to an airflow channel; and the dose protection plate is limited by the air inlet baffle to an inlet of the inhalation channel and blocks a dose cup of the powder metering wheel, wherein
the outer cap presses the resetting torsion spring in the cap-opening load stroke, and releases limitation of the resetting torsion spring on the air inlet baffle;
when negative pressure of the airflow channel exceeds a threshold, the air inlet baffle rotates under an action of an air flow to open the airflow channel and release limitation on the dose protection plate, and the dose protection plate rotates to deviate under an action of the drive torsion spring and unblocks the dose cup of the powder metering wheel; and
further, the outer cap further triggers, in the cap-closing load stroke, the dose protection plate and the air inlet baffle to reset.

11. The powder inhaler of claim 10, wherein
the outer cap drives, in the first cap-closing sub-load stroke and the second cap-closing sub-load stroke via the powder metering wheel, the dose protection plate to reset to a position beyond the inlet of the inhalation channel, and compresses the drive torsion spring; the resetting torsion spring drives the air inlet baffle to reset and rotate and close the airflow channel; and
the outer cap is in the third cap-closing sub-load stroke, the powder metering wheel disengages from the dose protection plate, and the drive torsion spring drives the dose protection plate to rotate to the inlet of the inhalation channel, and is limited by the air inlet baffle at the inlet of the inhalation channel.

12. The powder inhaler according to any one of claims 1 to 11, wherein
an angle of the outer cap at the first position is defined as 0 degrees, and an angle of the outer cap at the second position is greater than or equal to 120 degrees and less than or equal to 180 degrees;
preferably, the angle of the outer cap at the second position is 150 degrees; and
a critical angle between the cap-opening idle stroke and the cap-opening load stroke is greater than or equal to 10 degrees and less than or equal to 15 degrees; and/or a torque of the outer cap in the cap-opening idle stroke is greater than or equal to 0.05 N•m and less than or equal to 0.3 N•m, and a torque of the outer cap in the cap-opening load stroke is greater than or equal to 0 N•m and less than or equal to 0.15 N•m; and/or
the cap-opening idle stroke comprises the first cap-opening sub-idle stroke and the second cap-opening sub-idle stroke following the first cap-opening sub-idle stroke, wherein a critical angle between the first cap-opening sub-idle stroke and the second cap-opening sub-idle stroke is greater than or equal to 6 degrees and less than or equal to 10 degrees; and/or a torque of the outer cap in the first cap-opening sub-idle stroke is greater than or equal to 0.02 N•m and less than or equal to 0.08 N•m, and a torque of the outer cap in the second cap-opening sub-idle stroke is greater than or equal to 0.1 N•m and less than or equal to 0.2 N•m; and/or
the cap-opening load stroke comprises the first cap-opening sub-load stroke and the second cap-opening sub-load stroke following the first cap-opening sub-load stroke, wherein a critical angle between the first cap-opening sub-load stroke and the second cap-opening sub-load stroke is greater than or equal to 60 degrees and less than or equal to 65 degrees; and/or a torque of the outer cap in the first cap-opening sub-load stroke is constant, and is greater than or equal to 0 N•m and less than or equal to 0.05 N•m, and a torque of the outer cap in the second cap-opening sub-load stroke is constant, and is greater than or equal to 0.05 N•m and less than or equal to 0.15 N•m; and/or
a critical angle between the cap-closing idle stroke and the cap-closing load stroke is greater than or equal to 80 degrees and less than or equal to 95 degrees; and/or a maximum torque of the outer cap in the cap-closing idle stroke is greater than or equal to 0.03 N•m and less than or equal to 0.07 N•m, and a maximum torque of the outer cap in the cap-closing load stroke is greater than 0.05 N•m and less than or equal to 0.3 N•m; and/or
the cap-closing idle stroke comprises the first cap-closing sub-idle stroke and the second cap-closing sub-idle stroke following the first cap-closing sub-idle stroke, wherein a critical angle between the first cap-closing sub-idle stroke and the second cap-closing sub-idle stroke is greater than or equal to 135 degrees and less than or equal to 145 degrees; and/or a torque of the outer cap in the first cap-closing sub-idle stroke is constant, and is greater than or equal to 0.03 N•m and less than or equal to 0.07 N•m, and a torque of the outer cap in the second cap-closing sub-idle stroke is constant, and is less than or equal to 0.02 N•m; and/or
the cap-closing load stroke comprises the first cap-closing sub-load stroke, the second cap-closing sub-load stroke, and the third cap-closing sub-load stroke that are set in the chronological order, wherein a critical angle between the first cap-closing sub-load stroke and the second cap-closing sub-load stroke is greater than or equal to 60 degrees and less than or equal to 65 degrees, and a critical angle between the second cap-closing sub-load stroke and the third cap-closing sub-load stroke is greater than or equal to 6 degrees and less than or equal to 10 degrees; and/or a torque of the outer cap in the first cap-closing sub-load stroke is constant, and is greater than or equal to 0.05 N•m and less than or equal to 0.15 N•m, a torque of the outer cap in the second cap-closing sub-load stroke gradually increases, and a maximum value of the torque is greater than or equal to 0.15 N•m and less than or equal to 0.3 N•m, and a torque of the outer cap in the third cap-closing sub-load stroke is constant, and is greater than or equal to 0.05 N•m and less than or equal to 0.15 N•m.

13. An air inlet baffle, configured for a powder inhaler, wherein the air inlet baffle comprises:
a baffle body, wherein
a first surface of the baffle body has a boss, and a peripheral side surface of the boss is spaced apart from a peripheral side surface of the baffle body.

14. The air inlet baffle of claim 13, wherein
the boss covers a central region of the first surface of the baffle body.

15. The air inlet baffle of claim 14, wherein the outer peripheral side surface of the boss is evenly spaced apart from the outer peripheral side surface of the baffle body along a circumferential direction of the boss; and a part of the first surface of the baffle body that is not covered by the boss forms an annular surface.

16. The air inlet baffle of claim 13, wherein
the air inlet baffle further comprises a rotating shaft, disposed at a first end of the baffle body; and
a height of the boss gradually decreases along a direction from the first end of the baffle body to an opposite second end of the baffle body, to enable a top surface of the boss to form an inclined surface.

17. The air inlet baffle of claim 13, wherein
the baffle body is recessed to form the boss.

18. The air inlet baffle of claim 13, wherein
the air inlet baffle further comprises a rotating shaft and a rotating member; the rotating shaft is disposed at one end of the baffle body; and the rotating member is connected to a free end of the rotating shaft and is spaced apart from the baffle body, wherein
a first end of the rotating member has a curved surface.

19. The air inlet baffle of claim 18, wherein
a surface of a second end of the rotating member that is away from the baffle body has a protruding cylinder.

20. The air inlet baffle of claim 19, wherein
a quantity of rotating shafts is two, and the rotating shafts are respectively disposed on two opposite sides of the baffle body and are defined as a first rotating shaft and a second rotating shaft; a quantity of rotating members is two, and the rotating members are defined as a first rotating member and a second rotating member;
the first rotating member is connected to a free end of the first rotating shaft, a first end of the first rotating member has a first curved surface, and a surface of a second end of the first rotating member that is away from the baffle body has the protruding cylinder; and
the second rotating member is connected to a free end of the second rotating shaft, a first end of the second rotating member has a second curved surface, and a second end of the second rotating member has a circular-arc-shaped groove surface.

21. The air inlet baffle of claim 18, wherein
a quantity of rotating shafts is two, and the rotating shafts are respectively disposed on two opposite sides of the baffle body; one end of each rotating shaft is connected to a side surface of the baffle body; and
the side surface of the baffle body further has a shoulder surrounding the rotating shaft, and the shoulder is spaced apart from the rotating member.

22. A dose protection plate, configured for a powder inhaler, wherein the dose protection plate comprises:
an annular body; and
a blocking portion, connected to one end of the annular body and configured to block or unblock a dose cup of the powder inhaler.

23. The dose protection plate of claim 22, wherein
the dose protection plate further comprises a pressing member, and the pressing member is disposed on an outer side surface of the annular body; and a surface of the pressing member that is away from the annular body comprises a pressing arc surface.

24. The dose protection plate of claim 23, wherein
the pressing member comprises a cylindrical convex surface, and the cylindrical convex surface is disposed on one side of the pressing arc surface.

25. The dose protection plate of claim 22, wherein
the dose protection plate further comprises an elastic arm claw, and one end of the elastic arm claw is connected to the annular body.

26. The dose protection plate of claim 22, wherein
the dose protection plate further comprises a wedge-shaped post, and one end of the wedge-shaped post is connected to the annular body.

27. An inhalation trigger mechanism, comprising:
the air inlet baffle according to any one of claims 13 to 21; and/or
the dose protection plate according to any one of claims 22 to 26.

28. A powder inhaler, comprising:
a powder delivery mechanism, comprising a powder container and a powder metering wheel, wherein the powder container has a storage cavity, an inhalation channel, and an airflow channel; the inhalation channel is in communication with the airflow channel; the storage cavity is configured to store powder, and the storage cavity has a powder outlet; the powder metering wheel is rotatably connected to the powder container; the powder metering wheel comprises a dose cup; the dose cup is configured to receive the powder from the powder outlet and deliver the powder to an inlet of the inhalation channel; and
the inhalation trigger mechanism of claim 27, wherein the air inlet baffle is rotatably connected to a side wall of the airflow channel, and the first surface of the baffle body faces an outer side of a port of the airflow channel; the dose protection plate is rotatably connected to the powder container; and the air inlet baffle is linked to and cooperates with the dose protection plate, wherein
when the inhalation trigger mechanism is in an initial state, the air inlet baffle seals the airflow channel, and the inhalation channel is not in communication with external air; the blocking portion blocks the powder outlet of the dose cup that is located at the inlet of the inhalation channel; and
when negative pressure in the inhalation channel exceeds a threshold, the air inlet baffle rotates to enable the airflow channel to be opened and triggers the dose protection plate to rotate, to enable the blocking portion to deviate and unblock the powder outlet of the dose cup, and the airflow channel is in communication with the external air and the inhalation channel.

29. The powder inhaler of claim 28, further comprising:
a housing assembly, comprising a front housing, wherein the front housing has a suction nozzle, and the suction nozzle is disposed corresponding to the inhalation channel and is in communication with the inhalation channel; a side wall of the front housing is provided with an air inlet and a grille, and the grille protrudes out of an outer wall surface of the front housing; an inner wall surface of the front housing is provided with an annular flange surrounding the air inlet, one end of the annular flange is disposed in the airflow channel, and the side wall of the front housing seals the port of the airflow channel;
the air inlet baffle has the annular surface; when the inhalation trigger mechanism is in an initial state, the boss is embedded in the annular flange, an inner peripheral surface of the annular flange is spaced apart from and cooperates with an outer peripheral surface of the boss to form a first flow channel segment, an end surface at one end of the annular flange that is away from the front housing abuts against and cooperates with the annular surface to form a second flow channel segment, and the first flow channel segment and the second flow channel segment form an L-shaped air inlet flow channel; the pressing arc surface cooperates with a circular-arc-shaped groove surface of the air inlet baffle to implement concentric arc surface pressing; and
when the negative pressure in the inhalation channel exceeds the threshold, the air inlet baffle rotates to enable the airflow channel to be opened, and the airflow channel is in communication with the external air through the air inlet.

30. The powder inhaler of claim 29, wherein a ratio of a distance between the top surface of the boss and the first surface of the baffle body to a thickness of the annular flange is 1:2 to 7:1.

31. The powder inhaler of claim 29, wherein the side wall of the front housing is provided with at least two air inlets and at least one grille, and the grille and the air inlets are alternately provided; the annular flange is disposed around the air inlets and the grille;
the side wall of the airflow channel is connected to a side wall of the storage cavity, and one end of the side wall of the airflow channel that is connected to the side wall of the storage cavity is provided with a first flow guiding hole and a second flow guiding hole that are spaced apart from each other; one end of the inhalation channel that is close to the suction nozzle has a first airflow inlet and a second airflow inlet that are spaced apart from each other, the first flow guiding hole is in communication with the airflow channel and the first airflow inlet, and the second flow guiding hole is in communication with the airflow channel and the second airflow inlet; and
the inner wall surface of the front housing is spaced apart from an outer wall surface of the inhalation channel to form a flow guiding channel, and the flow guiding channel is in communication with the airflow channel, the first airflow inlet, and the second airflow inlet.

32. The powder inhaler of claim 29, further comprising:
an outer cap, rotatably connected to the housing assembly and capable of rotating back and forth between a first position and a second position, wherein the outer cap blocks the suction nozzle and the air inlet when the outer cap is configured to be at the first position; and the suction nozzle and the air inlet are exposed when the outer cap is configured to be at the second position; and
an air compressing mechanism, disposed on the powder container, and configured to press the powder in the powder container into the dose cup, wherein
the powder metering wheel is capable of rotating back and forth between a third position and a fourth position; when the powder metering wheel is configured to be at the third position, the dose cup is disposed corresponding to the powder outlet of the storage cavity; when the powder metering wheel is configured to be at the fourth position, the dose cup is disposed corresponding to an inlet of the inhalation channel;
the outer cap is linked to and cooperates with the powder delivery mechanism and the air compressing mechanism respectively; in a process in which the outer cap rotates from the first position to the second position, the outer cap first drives the air compressing mechanism to press the powder in the storage cavity into the dose cup, and then drives the powder metering wheel to rotate from the third position to the fourth position; and
in a process in which the outer cap reversely rotates and resets from the second position to the first position, the outer cap drives the powder metering wheel, the dose protection plate, and the air compressing mechanism respectively to reset, and triggers the air inlet baffle to reversely rotate and reset.

33. An airbag pressing member, configured for a powder inhaler, wherein
a side wall of the airbag pressing member has a pressure relief hole; and the airbag pressing member is configured to press an air compressing airbag.

34. The airbag pressing member of claim 33, wherein
one end of the side wall of the airbag pressing member that is away from a top wall of the airbag pressing member has a protruding rod, and one end of the protruding rod that is away from the top wall has an arc surface.

35. The airbag pressing member of claim 34, wherein one end of the protruding rod that is away from the top wall has a tip, and an end surface of the tip is the arc surface.

36. The airbag pressing member of claim 34, wherein the arc surface is a circular arc surface.

37. The airbag pressing member according to any one of claims 33 to 36, wherein
the top wall of the airbag pressing member has a fixing hole, and the fixing hole is configured to connect to a top portion of the air compressing airbag, so as to drive the air compressing airbag to extend and contract.

38. A drive cam, configured for a powder inhaler, wherein the drive cam comprises:
a body portion; and
a gear, coaxially connected to the body portion, and configured to drive the body portion to rotate, wherein
a surface of the body portion has a guide groove, and a side surface of the guide groove is a cam curved surface.

39. The drive cam of claim 38, wherein
an outer peripheral side surface of the body portion has an arc-shaped groove located at one end of the cam curved surface.

40. The drive cam of claim 39, wherein
the gear is disposed on a surface of the body portion, and the guide groove is provided on a surface of the body portion that faces the gear and is spaced apart from the gear;
the cam curved surface comprises a first curved surface segment and a second curved surface segment that are connected to each other, and the second curved surface segment is located at one end of the first curved surface segment that is away from the arc-shaped groove; and the first curved surface segment is a non-circular arc surface, and the second curved surface segment is a circular arc surface and is disposed concentric to the peripheral side surface of the body portion.

41. The drive cam of claim 40, wherein a stop groove is provided at one end of the second curved surface segment that is away from the first curved surface segment.

42. The drive cam of claim 41, wherein
the arc-shaped groove and/or the stop groove are/is a circular-arc-shaped groove.

43. The drive cam of claim 40, wherein
a surface of the body portion that faces away from the gear has a rib; and one end of the rib is disposed corresponding to an end portion of the second curved surface segment that is close to the first curved surface segment.

44. The drive cam of claim 41, wherein
a surface of the body portion that faces away from the gear further has an annular boss; and the annular boss is disposed coaxially with the gear.

45. An air compressing mechanism, comprising:
an air compressing airbag;
an elastic member;
the airbag pressing member according to any one of claims 33 to 37; and/or
the drive cam according to any one of claims 38 to 44.

46. The air compressing mechanism of claim 45, wherein the arc surface of the protruding rod cooperates with the cam curved surface of the drive cam to enable the airbag pressing member to move back and forth between a fifth position and a sixth position; and
when the airbag pressing member is configured to be at an initial position, the tip of the protruding rod is embedded in the arc-shaped groove of the cam curved surface, to initially position the airbag pressing member.

47. A powder inhaler, comprising:
a powder delivery mechanism, comprising a powder container, wherein the powder container has a storage cavity, a first end of the storage cavity has a powder outlet, and a second end of the storage cavity has an air compressing port; and a side wall of the storage cavity has a vent hole; and
the air compressing mechanism of claim 45 or 46, wherein
the air compressing airbag is disposed at the second end of the storage cavity and is in communication with the air compressing port; the airbag pressing member is movably sleeved on an outer side of the air compressing airbag and an outer side of the storage cavity;
the drive cam and the elastic member are configured to drive the airbag pressing member to move back and forth between the fifth position and the sixth position, so as to drive the air compressing airbag to extend and contract; when the airbag pressing member is configured to be at the fifth position, the side wall of the airbag pressing member seals the vent hole, and the pressure relief hole is not in communication with the vent hole; and when the airbag pressing member is configured to be at the sixth position, the pressure relief hole is in communication with the vent hole, to relieve pressure in the storage cavity.

48. The powder inhaler of claim 47, further comprising:
a filter membrane, disposed at the second end of the storage cavity, wherein the filter membrane is located at the air compressing port and is spaced apart from a port of the air compressing port; and one end of the vent hole is in communication with space between the filter membrane and the air compressing airbag;
a housing assembly, having a suction nozzle; and
an outer cap, rotatably connected to the housing assembly and capable of rotating back and forth between a first position and a second position, wherein the outer cap blocks the suction nozzle when the outer cap is configured to be at the first position; the suction nozzle is exposed when the outer cap is configured to be at the second position;
the powder delivery mechanism further comprises a powder metering wheel; the powder container further has an inhalation channel, and the suction nozzle is in communication with the inhalation channel; the powder metering wheel is rotatably connected to the powder container; the powder metering wheel comprises a dose cup; the powder metering wheel is capable of rotating back and forth between a third position and a fourth position; when the powder metering wheel is configured to be at the third position, the dose cup is disposed corresponding to the powder outlet of the storage cavity and is configured to receive the powder in the powder container; and when the powder metering wheel is configured to be at the fourth position, the dose cup is disposed corresponding to an inlet of the inhalation channel, wherein
the outer cap is linked to and cooperates with the powder metering wheel and the air compressing mechanism respectively; when the outer cap is configured to be at the first position, the airbag pressing member is limited at the fifth position; in a process in which the outer cap rotates from the first position to the second position, limitation on the airbag pressing member is first released, to enable the elastic member to drive the airbag pressing member to move from the fifth position to the sixth position, and then drive the powder metering wheel to rotate from the third position to the fourth position; and
in a process in which the outer cap reversely rotates and resets from the second position to the first position, the outer cap drives the powder metering wheel to reversely rotate and reset, and drives the airbag pressing member to move in a reverse direction and reset to the fifth position.
